# EUROPEAN PATENT APPLICATION

(11) **EP 4 382 523 A1**
(43) Date of publication of application: **12.06.2024**
(21) Application number: 22853372.5
(22) Date of filing: 29.07.2022
(51) Int. Cl.: C07D 405/12, A61K 31/404, C07D 209/08, C07D 209/30

(54) **NOVEL INDOLE DERIVATIVE, PHARMACEUTICAL COMPOSITION COMPRISING SAME, AND USE THEREOF**

(30) Priority: 02.08.2021 KR 20210101378
(71) Applicant: MitoImmune Therapeutics Inc., Seoul 06123 (KR)
(72) Inventor: KIM, Soon Ha, Seoul 06123 (KR); CHANG, Hye Kyung, Seoul 06123 (KR); YOO, Eun Kyung, Seoul 06123 (KR); JANG, Kyung Kuk, Seoul 06123 (KR); SEO, Mooyoung, Seoul 06123 (KR); PARK, Jin Sung, Seoul 06123 (KR); YANG, Jeong-Hee, Seoul 06123 (KR); PARK, Hyunjun, Seoul 06123 (KR); PARK, Jeong Hyang, Seoul 06123 (KR); PARK, Yun Min, Seoul 06123 (KR); KIM, Jae Young, Seoul 06123 (KR); YUN, Yewon, Seoul 06123 (KR)
(74) Representative: Plasseraud IP
(86) International application number: PCT/KR2022/011211
(87) International publication number: WO 2023/013994

(57) **Abstract**

The present invention relates to a compound of Formula 1, a preparation method thereof, a pharmaceutical composition comprising the same as an active ingredient, and a use thereof. The compound of Formula 1 according to the present invention can exhibit cell necrosis and ferroptosis inhibitory efficacy in various cells such as heart, kidney, nerve, retina, liver, or lung cells, and accordingly, can be usefully used for prevention or treatment of cell necrosis or ferroptosis-related diseases.

## Description

### [Technical Field]

The present invention relates to a compound of Formula 1, an isomer thereof, a solvate thereof, a hydrate thereof, or a pharmaceutically acceptable salt thereof, a method for preparing the same, a pharmaceutical composition comprising the same, and a use thereof.

### [Background Art]

Cell death contributes to the maintenance and change of cellular functions for the survival of living organisms, or contributes to maintaining the number of cells in balance with the proliferation of cells for embryonic development, and is also used as a method to effectively and quickly inform the immune system of various danger signals generated from outside, such as invasion of viruses or bacteria in the immune system, and local injury.

However, cell death due to external hazards or local injury can cause local or systemic inflammation due to excessive activation of the immune system, leading to a situation in which living organisms are harmed. Cell death in which this phenomenon occurs is mainly observed in necrotic cell death (or necrosis), and researchers have conducted many studies for disease treatment and improvement by preventing cell necrosis.

The cell necrosis is usually the cell death characterized by oxygen and energy depletion, cell membrane collapse, and the like due to sudden physical and chemical shocks applied to cells, and simultaneously, injury-related substances (DAMPs, damage associated with molecular patterns) such as ATP (adenosine triphosphate), Ca²⁺ (calcium ion), ROS (reactive oxygen species) and HMGB1 (high mobility group box 1) are also released, where these substances are known to induce chained inflammatory reactions and cell death by stimulating or damaging surrounding cells. This cell necrosis is non-regulated cell death (NRCD), and is distinguished from apoptosis, which is regulated cell death (RCD) regulated by caspases.

In the past, a lot of research had been done on apoptosis, which is regulated cell death, in the study of cell death for the treatment of diseases, and pharmaceutical companies had also made great efforts to develop caspase inhibitors, but few drugs have been approved by the FDA. Apoptosis is highly likely to be cell death that occurs to maintain homeostasis in the body, whereas cell necrosis is cell death that occurs mainly in pathological conditions. Accordingly, research on inhibition of cell necrosis is very important in disease prevention and treatment.

Recently, as research on cell death has been actively conducted, regulated necrotic cell death (RNCD), which is distinct from cell necrosis, has been newly identified as ferroptosis.

Ferroptosis is characterized by the accumulation of lipid peroxides and iron involvement. Cells normally receive cystine through system Xc⁻ to maintain the concentration of glutathione, and activate GPX4 (glutathione peroxidase 4) to activate an antioxidation system for removing intracellular lipid peroxidation substances. However, when the glutathione concentration is low or the GPX4 does not function, the cell death occurs by inducing damage to lipids, proteins, nucleic acids, and the like, and cell membrane collapse due to the accumulation of lipid peroxides. It is known that as another factor in the accumulation of lipid peroxides, the ROS generated by the binding of iron ions with intracellular free radicals by the Fenton reaction are involved. The cell death by the lipid peroxide has also an aspect similar to cell necrosis, where a large number of DAMPs are released to the outside of the cells and cause inflammation and death of surrounding cells.

Therefore, it has been frequently reported that ferroptosis is observed in pathological conditions similar to cell necrosis, and inhibition of ferroptosis is very important for disease prevention and treatment.

As representative diseases that cell necrosis, which is non-regulated cell death, and ferroptosis, which is regulated necrotic cell death, appear, ischemic disease (e.g., myocardial infarction, stroke, renal infarction), neurodegenerative disease, and inflammatory disease, aging, macular degeneration and lung disease, and the like have been reported. Accordingly, the discovery and the development of substances that inhibit cell necrosis and ferroptosis in disease research and treatment are also very urgent.

Meanwhile, 5-[(1,1-dioxido-4-thiomorpholinyl)methyl]-2-phenyl-*N-*(tetrahydro-2*H-*pyran-4-yl)-1*H*-indol-7-amine is a compound disclosed through International Patent Publication No. WO2009-025478, which is a material known to exhibit preventive or therapeutic and ameliorating effects on cell necrosis and related diseases. Indole derivatives such as the above compound have pharmaceutically very useful structures, and many studies on these structures are in progress.

The present inventors conducted a study on whether ferroptosis, which is regulated necrotic cell death similar to cell necrosis, shows pharmaceutically similar efficacy to indole derivatives such as the above compound, and confirmed their effects. Accordingly, the present inventors have continuously conducted research on various compounds exhibiting the effects of preventing or treating and ameliorating diseases related to non-regulated cell necrosis, and ferroptosis, which is regulated necrotic cell death, thereby synthesizing novel compounds, and completing the present invention by confirming their effects.

### [Prior Art Documents]

### [Patent Documents]

International Patent Publication No. WO2009-025478

### [Disclosure]

### [Technical Problem]

It is an object of the present invention to provide a novel compound of Formula 1, an isomer thereof, a solvate thereof, a hydrate thereof, or a pharmaceutically acceptable salt thereof.

Also, it is an object of the present invention to provide a pharmaceutical composition for preventing or treating cell necrosis or ferroptosis-related diseases comprising a compound of Formula 1, an isomer thereof, a solvate thereof, a hydrate thereof, or a pharmaceutically acceptable salt thereof as an active ingredient together with a pharmaceutically acceptable carrier.

In addition, it is an object of the present invention to provide a method for preparing a compound of Formula 1, and a method for preventing or treating cell necrosis or ferroptosis-related diseases.

Furthermore, it is an object of the present invention to provide a method for inhibiting ferroptosis, and a method for inhibiting reactive oxygen species.

### [Technical Solution]

The present invention provides a compound of Formula 1 below, an isomer thereof, a solvate thereof, a hydrate thereof, or a pharmaceutically acceptable salt thereof.

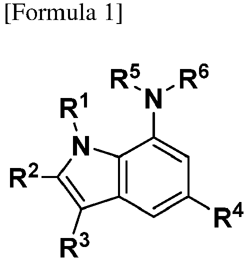

In Formula 1 above,
R¹ is hydrogen or C₁₋₈ alkyl,
R² and R³ are each independently hydrogen, aryl with 5 to 10 atoms, heteroaryl with 5 to 10 atoms, C₁₋₈ alkyl, or C₁₋₈ alkoxy, and when R² and R³ are aryl or heteroaryl, R² and R³ are each independently unsubstituted or substituted with one or more substituents of halogen, C₁₋₈ alkyl, -P(=O)(C₁₋₈ alkyl)₂, and C₁₋₈ alkynyl-OH,
R⁴ is -C₁₋₈ alkylene-O-C₁₋₈ alkylene-O-R⁷, where
R⁷ is C₁₋₈ alkyl or -C₁₋₈ alkylene-O-C₁₋₈ alkyl,
R⁵ and R⁶ are each independently hydrogen, C₁₋₈ alkyl, C₁₋₈ alkoxy, C₃₋₁₀ cycloalkyl, -C₃₋₁₀ heterocycloalkyl, -(CH₂)ₘ-C₃₋₁₀ cycloalkyl, -(CH₂)ₘ-C₃₋₁₀ heterocycloalkyl, aryl with 5 to 10 atoms, benzyl, heteroaryl with 5 to 10 atoms or -(CH₂)ₘ-heteroaryl with 5 to 10 atoms, or are linked together with the N atom, to which they are bonded, to form C₃₋₁₀ heterocycloalkyl, heteroaryl with 5 to 10 atoms or C₁₋₈ alkylimine, where m is an integer of 1 to 4,
R⁵ and R⁶ are unsubstituted or substituted with one or more substituents R⁸ selected from the group consisting of C₁₋₈ alkyl, C₁₋₈ alkoxy, C₁₋₈ haloalkyl, halogen, -OH, oxo (=O), - C(=O)-C₁₋₈ alkyl, -C(=O)O-C₁₋₈ alkyl, -C(=O)NH-aryl with 5 to 10 atoms and -S(=O)₂-C₁₋₈ alkyl, and
R⁸ is unsubstituted or substituted with one or more substituents R⁹ selected from halogen and C₁₋₈ haloalkyl.

In the definition of a substituent according to the present invention, the term 'alkyl' refers to an aliphatic hydrocarbon radical. An alkyl may be a "saturated alkyl" which does not include an alkenyl or alkynyl region, or may be an "unsaturated alkyl" which includes at least one alkenyl or alkynyl region.

"Alkenyl" refers to a group including at least one carbon-carbon double bond, and "alkynyl" refers to a group including at least one carbon-carbon triple bond. The alkyl may be a branched or straight chain type, respectively.

An alkyl group, unless otherwise defined, may have 1 to 20 carbon atoms. An alkyl group may be a medium-sized alkyl having 1 to 10 carbon atoms. An alkyl group may be a lower alkyl having 1 to 6 carbon atoms. Whereas typical alkyl groups include methyl, ethyl, propyl, isopropyl, butyl, isobutyl, t-butyl, pentyl, hexyl, ethenyl, propenyl and butenyl, and the like, an alkyl group is not limited to these. For example, a C₁₋₄alkyl has 1 to 4 carbon atoms in its alkyl chain, and is selected from a group comprised of methyl, ethyl, propyl, isopropyl, n-butyl, iso-butyl, sec-butyl and t-butyl.

The term 'alkylene' refers to a hydrocarbon bivalent group wherein radicals are additionally formed in the alkyl described above, and non-limiting examples include methylene, ethylene, propylene, butylene and isobutylene.

The term 'alkoxy', unless otherwise defined, refers to an alkyloxy, and non-limiting examples include methoxy, ethoxy and propoxy.

The term "haloalkyl" may mean -RX(X is one or more halogen atoms(F, Cl, Br and I)), that is, "haloalkyl" may be an alkyl form in which one or more halogens are substituted. For example, non-limiting examples of "C₁₋₈ haloalkyl" may include trifluoromethyl, or difluoromethyl.

The term 'cycloalkyl', unless otherwise defined, refers to a saturated or unsaturated aliphatic ring. Further, the number of atoms forming the ring may be 3 to 12. Non-limiting examples of typical cycloalkyl groups include cyclopropyl, cyclobutyl, cyclopentyl and cyclohexyl.

The term `heterocycloalkyl', unless otherwise defined, refers to a cycloalkyl as defined above which includes 1 to 3 heteroatoms selected from a group comprised of N, O and S. A heterocycloalkyl may be monocyclic, or may be multicyclic, such as spirocyclic, bridged cyclic or fused cyclic. Non-limiting examples of heterocycloalkyl include pyrrolidine, piperidine, tetrahydropyran, oxetane, thiopyran and groups similar to these.

The term 'aryl' includes at least one ring having a covalent π electron system, for example, monocyclic or fused polycyclic (i.e., cycles that share the adjacent carbon atom pairs) groups. That is, in the present specification, aryl means an aromatic 4-10 membered, preferably 6-10 membered, monocyclic or multicyclic ring including phenyl, naphthyl, and the like, unless otherwise defined.

The term 'heteroaryl', unless otherwise defined, refers to an aromatic 3-10 membered, preferably 4-8 membered, more preferably 5-6 membered cycle that has 1 to 3 hetero atoms selected from N, O and S, and may be fused with benzo or C₃₋₈ cycloalkyl. The monocyclic heteroaryl includes, but not limited to, thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, pyrazine and the like. Non-limiting examples of bicyclic heteroaryl include indole, indoline, benzothiophene, benzofuran, benzimidazole, benzoxazole, benzisoxazole, benzthiazole, benzthiadiazole, benztriazole, quinoline, isoquinoline, purine, puropyridine and groups similar thereto.

The term 'heterocycle', unless otherwise defined, refers to a 3-10 membered, preferably 4-8 membered, more preferably 5-6 membered cycle that has 1 to 3 hetero atoms selected from a group comprised of N, O and S, which may be fused with benzo or C₃₋₈ cycloalkyl, and is saturated or contains 1 or 2 double bonds. Non-limiting examples of heterocycle include pyrroline, pyrrolidine, imidazoline, imidazolidine, pyrazoline, pyrazolidine, pyran, piperidine, morpholine, thiomorpholine, piperazine, and hydrofuran.

Other terms and abbreviations used in the present specification, unless defined otherwise, may be interpreted to have the meanings ordinarily understood by a person having ordinary skill in the art.

In this specification, the "hydrate" may mean a compound of the present invention containing a stoichiometric or non-stoichiometric amount of water bound by non-covalent intermolecular forces, or a salt thereof. The hydrate of the compound of the present invention represented by Formula 1 may include a stoichiometric or non-stoichiometric amount of water that is bound by non-covalent intermolecular forces. The hydrate may contain 1 equivalent or more, preferably, 1 to 5 equivalents of water. Such a hydrate may be prepared by crystallizing the compound of the present invention represented by Formula 1, an isomer thereof, or a pharmaceutically acceptable salt thereof from water or a solvent containing water.

In this specification, the "solvate" may mean a compound of the present invention containing a stoichiometric or non-stoichiometric amount of a solvent bound by non-covalent intermolecular forces, or a salt thereof. Preferred solvents therefor include solvents that are volatile, non-toxic, and/or suitable for administration to humans.

In this specification, the "isomer" may mean a compound of the present invention that has the same chemical formula or molecular formula but is structurally or sterically different, or a salt thereof. Such isomers include all structural isomers such as tautomers, R or S isomers having an asymmetric carbon center, stereoisomers such as geometric isomers (trans, cis), and optical isomers (enantiomers). All these isomers and mixtures thereof are also included within the scope of the present invention.

The compound of Formula 1 may be a compound represented by Formula 2 below.

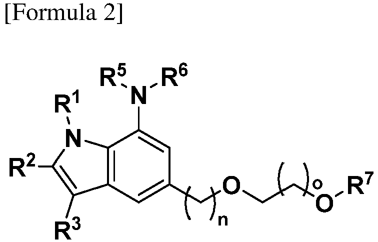

In Formula 2, R¹ to R³, R⁵ to R⁷, n and o are each as defined herein.

According to one aspect of the compound of Formula 1,
R¹ may be hydrogen or C₁₋₆ alkyl,
R² and R³ may be each independently hydrogen, aryl with 5 to 8 atoms, heteroaryl with 5 to 8 atoms containing one or more heteroatoms of N, O, and S, C₁₋₆ alkyl, or C₁₋₆ alkoxy, and when R² and R³ are aryl or heteroaryl, R² and R³ may be each independently unsubstituted or substituted with one or more substituents of halogen, C₁₋₆ alkyl, -P(=O)(C₁₋₆ alkyl)₂, and C₁₋₆ alkynyl-OH,
R⁴ may be C₁₋₆ alkylene-O-C₁₋₆ alkylene-O-R⁷, where
R⁷ may be C₁₋₆ alkyl or C₁₋₆ alkylene-O-C₁₋₆ alkyl,
R⁵ and R⁶ may be each independently hydrogen, C₁₋₆ alkyl, C₁₋₆ alkoxy, C₃₋₇ cycloalkyl, C₃₋₇ heterocycloalkyl, -(CH₂)ₘ-C₃₋₇ cycloalkyl, -(CH₂)ₘ-C₃₋₇ heterocycloalkyl, aryl with 5 to 8 atoms, benzyl, heteroaryl with 5 to 8 atoms containing one or more heteroatoms of N, O, and S, or -(CH₂)ₘ-heteroaryl with 5 to 8 atoms, or may be linked together with the N atom, to which they are bonded, to form C₃₋₇ heterocycloalkyl containing one or more heteroatoms of N, O, and S, heteroaryl with 5 to 10 atoms containing one or more heteroatoms of N, O, and S, or C₁₋₆ alkylimine, where m may be an integer of 1 to 4,
R⁵ and R⁶ may be unsubstituted or substituted with one or more substituents R⁸ selected from the group consisting of C₁₋₆ alkyl, C₁₋₆ alkoxy, C₁₋₆ haloalkyl, halogen, -OH, oxo (=O), -C(=O)-C₁₋₆ alkyl, -C(=O)O-C₁₋₆ alkyl, -C(=O)NH-aryl with 5 to 8 atoms and - S(=O)₂-C₁₋₆ alkyl, and
R⁸ may be unsubstituted or substituted with one or more substituents R⁹ selected from halogen and C₁₋₆ haloalkyl.

In the compound of Formula 1, R¹ may be hydrogen or C₁₋₆ alkyl, and preferably may be hydrogen or C₁₋₃ alkyl.

In one embodiment, when R¹ is C₁₋₈ alkyl, any one of R⁵ and R⁶ may be hydrogen and the other may be C₃₋₁₀ cycloalkyl or C₃₋₁₀ heterocycloalkyl.

In the compound of Formula 1, R² and R³ may be each independently hydrogen, aryl with 5 to 10 atoms, heteroaryl with 5 to 10 atoms, C₁₋₈ alkyl, or C₁₋₈ alkoxy, and preferably may be hydrogen, aryl with 5 to 8 atoms, or C₁₋₆ alkyl.

Here, when R² and R³ are aryl with 5 to 10 atoms or heteroaryl with 5 to 10 atoms, R² and R³ may be each independently unsubstituted or substituted with one or more substituents of halogen, C₁₋₈ alkyl, -P(=O)(C₁₋₈ alkyl)₂, and C₁₋₈ alkynyl-OH.

In one embodiment, R² may be hydrogen or aryl with 5 to 10 atoms, where the aryl may be unsubstituted or substituted with one or more substituents of halogen, C₁₋₈ alkyl, - P(=O)(C₁₋₈ alkyl)₂, and C₁₋₈ alkynyl-OH.

In one embodiment, R³ may be hydrogen, aryl with 5 to 10 atoms, or C₁₋₈ alkyl, and specifically R³ may be hydrogen, phenyl, or methyl.

In one embodiment, when any one of R² and R³ is hydrogen, the other may be aryl with 5 to 10 atoms, heteroaryl with 5 to 10 atoms, C₁₋₈ alkyl, or C₁₋₈ alkoxy.

In one embodiment, R² may be aryl with 5 to 10 atoms, and R³ may be hydrogen.

In one embodiment, R² may be hydrogen, and R³ may be C₁₋₈ alkyl or aryl with 5 to 10 atoms.

In the compound of Formula 1, R⁴ may be C₁₋₈ alkylene-O-C₁₋₈ alkylene-O-R⁷, and R⁷ may be C₁₋₈ alkyl or C₁₋₈ alkylene-O-C₁₋₈ alkyl.

In one embodiment, R⁴ may be -(CH₂)ₙ-O-CH₂-(CH₂)ₒ-O-R⁷, n and o may be integers of 1 to 4, and more specifically, n and o may be each independently an integer of 1 to 3.

In one embodiment, R⁷ may be C₁₋₄ alkyl or C₁₋₃ alkylene-O-C₁₋₃ alkyl.

In the compound of Formula 1, R⁵ and R⁶ may be each independently hydrogen, C₁₋₈ alkyl, C₁₋₈ alkoxy, C₃₋₁₀ cycloalkyl, C₃₋₁₀ heterocycloalkyl, -(CH₂)ₘ-C₃₋₁₀ cycloalkyl, -(CH₂)ₘ-C₃₋₁₀ heterocycloalkyl, aryl with 5 to 10 atoms, benzyl, heteroaryl with 5 to 10 atoms or - (CH₂)ₘ-heteroaryl with 5 to 10 atoms, or may be linked together with the N atom, to which they are bonded, to form C₃₋₁₀ heterocycloalkyl, heteroaryl with 5 to 10 atoms or C₁₋₈ alkylimine. Here, m may be an integer of 1 to 4.

In one embodiment, any one of R⁵ and R⁶ may be hydrogen, and the other may be C₁₋₈ alkyl, C₁₋₈ alkoxy, C₃₋₁₀ cycloalkyl, C₃₋₁₀ heterocycloalkyl, -(CH₂)ₘ-C₃₋₁₀ cycloalkyl, - (CH₂)ₘ-C₃₋₁₀ heterocycloalkyl, aryl with 5 to 10 atoms, benzyl, heteroaryl with 5 to 10 atoms or -(CH₂)ₘ-heteroaryl with 5 to 10 atoms, and m may be an integer of 1 to 4. Here, the heterocycloalkyl and heteroaryl may contain one or more heteroatoms of N, O and S.

In another embodiment of R⁵ and R⁶, R⁵ and R⁶ may simultaneously be C₃₋₇ cycloalkyl, particularly unsubstituted cyclopentyl, or unsubstituted cyclobutyl.

In one embodiment, R⁵ and R⁶ may be linked together with the N atom, to which they are bonded, to form C₃₋₁₀ heterocycloalkyl, or C₁₋₈ alkylimine, specifically, to form C₃₋₇ heterocycloalkyl, or C₂₋₅ alkylimines, and more specifically, to form morpholino, piperidinyl, or propan-2-imine. Here, C₂₋₅ alkylimine may be substituted with C₁₋₈ haloalkyl.

In one embodiment, R⁵ and R⁶ may be each independently hydrogen, tetrahydropyran, cyclopentyl, cyclopropyl, -CH₂-tetrahydropyranyl, tetrahydrofuranyl, oxetanyl, cyclobutyl, C₁₋₈ alkyl, -CH₂-cyclopentyl, -CH₂-cyclopropyl, cyclohexyl, benzyl, piperidinyl, thianyl, -CH₂-thienyl, or -CH₂-pyridinyl.

In the compound of Formula 1, R⁵ and R⁶ may be unsubstituted or substituted with one or more substituents R⁸ selected from the group consisting of C₁₋₈ alkyl, C₁₋₈ alkoxy, C₁₋₈ haloalkyl, halogen, -OH, oxo (=O), -C(=O)-C₁₋₈ alkyl, -C(=O)O-C₁₋₈ alkyl, -C(=O)NH-aryl with 5 to 10 atoms, and -S(=O)₂-C₁₋₈ alkyl.

In one embodiment, the substituent R⁸ may be selected from the group consisting of C₁₋₆ alkyl, C₁₋₆ alkoxy, C₁₋₆ haloalkyl, halogen, -OH, oxo (=O), -C(=O)-C₁₋₆ alkyl, -C(=O)O-C₁₋₆ alkyl, -C(=O)NH-aryl with 5 to 8 atoms and -S(=O)₂-C₁₋₆ alkyl.

In one embodiment, when one or more of R⁵ and R⁶ are C₁₋₈ alkyl, the substituent R⁸ may be C₁₋₈ alkoxy or C₁₋₈ haloalkyl.

In one embodiment, when one or more of R⁵ and R⁶ are C₃₋₁₀ cycloalkyl, or C₃₋₁₀ heterocycloalkyl, the substituent R⁸ may be selected from the group consisting of C₁₋₈ alkyl, C₁₋₈ alkoxy, C₁₋₈ haloalkyl, halogen, -OH, oxo (=O), -C(=O)-C₁₋₈ alkyl, -C(=O)O-C₁₋₈ alkyl, - C(=O)NH-aryl with 5 to 10 atoms and -S(=O)₂-C₁₋₈ alkyl.

In one embodiment, when one or more of R⁵ and R⁶ are C₃₋₁₀ heterocycloalkyl containing a heteroatom of N, the substituent R⁸ may be selected from the group consisting of C₁₋₆ alkyl, C₁₋₆ alkoxy, C₁₋₆ haloalkyl, halogen, -OH, oxo (=O), -C(=O)-C₁₋₆ alkyl, -C(=O)O-C₁₋₆ alkyl, -C(=O)NH-aryl with 5 to 8 atoms and -S(=O)₂-C₁₋₆ alkyl, or
when one or more of R⁵ and R⁶ are C₃₋₁₀ heterocycloalkyl containing a heteroatom of S, the substituent R⁸ may be oxo (=O).

In the compound of Formula 1, R⁸ may be unsubstituted or substituted with one or more substituents R⁹ selected from halogen and C₁₋₈ haloalkyl. Specifically, when R⁸ is - C(=O)NH-aryl with 5 to 10 atoms, R⁸ may be unsubstituted or substituted with one or more substituents R⁹ selected from halogen and C₁₋₈ haloalkyl.

The compound of Formula 1 according to the present invention may be any one selected from the group of compounds:
<1> 5-(2-methoxyethoxymethyl)-2-phenyl-N-tetrahydropyran-4-yl-1H-indol-7-amine; <2> N-cyclopentyl-5-(2-methoxyethoxymethyl)-2-phenyl-1H-indol-7-amine; <3> N,N-dicyclopentyl-5-(2-methoxyethoxymethyl)-2-phenyl-1H-indol-7-amine; <4> 5-(2-methoxyethoxymethyl)-2-phenyl-N-(tetrahydropyran-4-ylmethyl)-1H-indol-7-amine; <5> 4-[5-(2-methoxyethoxymethyl)-2-phenyl-1H-indol-7-yl]morpholine; <6> 5-(2-methoxyethoxymethyl)-2-phenyl-N-tetrahydrofuran-3-yl-1H-indol-7-amine; <7> 5-(2-methoxyethoxymethyl)-N-(oxetane-3-yl)-2-phenyl-1H-indol-7-amine; <8> N-cyclobutyl-5-(2-methoxyethoxymethyl)-2-phenyl-1H-indol-7-amine; <9> N,N-dicyclobutyl-5-(2-methoxyethoxymethyl)-2-phenyl-1H-indol-7-amine; <10> N-cyclobutyl-5-(2-methoxyethoxymethyl)-1-methyl-2-phenyl-indol-7-amine; <11> N-(2,2-dimethylpropyl)-5-(2-methoxyethoxymethyl)-2-phenyl-1H-indol-7-amine; <12> N-(cyclopentylmethyl)-5-(2-methoxyethoxymethyl)-2-phenyl-1H-indol-7-amine; <13> N-cyclohexyl-5-(2-methoxyethoxymethyl)-2-phenyl-1H-indol-7-amine;<14> N-(4,4-difluorocyclohexyl)-5-(2-methoxyethoxymethyl)-2-phenyl-1H-indol-7-amine; <15> N-isobutyl-5-(2-methoxyethoxymethyl)-2-phenyl-1H-indol-7-amine; <16> 5-(2-methoxyethoxymethyl)-2-phenyl-N-propyl-1H-indol-7-amine; <17> N-butyl-5-(2-methoxyethoxymethyl)-2-phenyl-1H-indol-7-amine; <18> N-(2-ethylbutyl)-5-(2-methoxyethoxymethyl)-2-phenyl-1H-indol-7-amine; <19> N-isopentyl-5-(2-methoxyethoxymethyl)-2-phenyl-1H-indol-7-amine; <20> 5-(2-methoxyethoxymethyl)-2-phenyl-N-sec-butyl-1H-indol-7-amine; <21> N-(cyclopropylmethyl)-5-(2-methoxyethoxymethyl)-2-phenyl-1H-indol-7-amine; <22> 5-(2-methoxyethoxymethyl)-N-(1-methylbutyl)-2-phenyl-1H-indol-7-amine; <23> N-(1-ethylpropyl)-5-(2-methoxyethoxymethyl)-2-phenyl-1H-indol-7-amine; <24> (E, Z)-1,1,1-trifluoro-N-[5-(2-methoxyethoxymethyl)-2-phenyl-1H-indol-7-yl]propane-2-imine; <25> 5-(2-methoxyethoxymethyl)-2-phenyl-N-(2,2,2,-trifluoro-1-methyl-ethyl)-1H-indol-7-amine;
<26> N-(1,2-dimethylpropyl)-5-(2-methoxyethoxymethyl)-2-phenyl-1H-indol-7-amine; <27> N-isopropyl-5-(2-methoxyethoxymethyl)-2-phenyl-1H-indol-7-amine; <28> N-benzyl-5-(2-methoxyethoxymethyl)-2-phenyl-1H-indol-7-amine; <29> tert-butyl-4-[[5-(2-methoxyethoxymethyl)-2-phenyl-1H-indol-7-yl]amino]piperidine-1-carboxylate; <30> 1-[4-[[5-(2-methoxyethoxymethyl)-2-phenyl-1H-indol-7-yl]amino]piperidyl]-1-ethanon; <31> 5-(2-methoxyethoxymethyl)-N-(1-methylsulfonyl-4-piperidyl)-2-phenyl-1H-indol-7-amine; <32> N-(1,1-dioxothiane-4-yl)-5-(2-methoxyethoxymethyl)-2-phenyl-1H-indol-7-amine; <33> 5-(2-methoxyethoxymethyl)-N-(1-oxothiane-4-yl)-2-phenyl-1H-indol-7-amine; <34> 5-(2-methoxyethoxymethyl)-N-(1-methyl-4-piperidyl)-2-phenyl-1H-indol-7-amine; <35> 5-(2-ethoxyethoxymethyl)-2-phenyl-N-tetrahydropyran-4-yl-1H-indol-7-amine; <36> N-cyclopentyl-5-(2-ethoxyethoxymethyl)-2-phenyl-1H-indol-7-amine; <37> N-cyclopentyl-5-(2-isopropoxyethoxymethyl)-2-phenyl-1H-indol-7-amine; <38> 5-(2-isopropoxy ethoxymethyl)-2-phenyl-N-tetrahydropyran-4-yl-1H-indol-7-amine; <39> N-cyclopentyl-5-[2-(2-methoxyethoxy)ethoxymethyl]-2-phenyl-1H-indol-7-amine; <40> 5-[2-(2-methoxyethoxy) ethoxymethyl]-2-phenyl-N-tetrahydropyran-4-yl-1H-indol-7-amine; <41> N-[3,5-bis(trifluoromethyl)phenyl]-4-[[5-(2-methoxyethoxymethyl)-2-phenyl-1H-indol-7-yl]amino]piperidine-1-carboxamide; <42> N-(3,5-dichlorophenyl)-4-[[5-(2-methoxyethoxymethyl)-2-phenyl-1H-indol-7-yl]amino]piperidine-1-carboxamide; <43> 5-(2-methoxyethoxymethyl)-2-phenyl-N-(2-thienylmethyl)-1H-indol-7-amine; <44> ethyl 4-[[5-(2-methoxyethoxymethyl)-2-phenyl-1H-indol-7-yl]amino]piperidine-1-carboxylate; <45> 5-(2-methoxyethoxymethyl)-2-phenyl-N-4-pyridylmethyl)-1H-indol-7-amine; <46> 5-(2-methoxyethoxymethyl)-N-(2-methoxyethyl)-2-phenyl-1H-indol-7-amine; <47> 5-(2-methoxyethoxymethyl)-N-(3-methoxypropyl)-2-phenyl-1H-indol-7-amine; <48> 2-(3-fluorophenyl)-5-(2-methoxyethoxymethyl)-N-tetrahydropyran-4-yl-1H-indol-7-amine; <49> N-cyclopentyl-5-(2-methoxyethoxymethyl)-3-methyl-1H-indol-7-amine; <50> 5-(2-methoxyethoxymethyl)-3-methyl-N-tetrahydropyran-4-yl-1H-indol-7-amine; <51> N-cyclopentyl-5-(2-methoxyethoxymethyl)-3-phenyl-1H-indol-7-amine; <52> 5-(2-methoxyethoxymethyl)-3-phenyl-N-tetrahydropyran-4-yl-1H-indol-7-amine; <53>5-((2-methoxyethoxy)methyl)-1-methyl-2-phenyl-N-(tetrahydro-2H-pyran-4-yl)-1H-indol-7-amine; <54>2-(4-bromophenyl)-N-cyclopentyl-5-((2-methoxyethoxy)methyl)-1H-indol-7-amine; <55>2-(4-bromophenyl)-5-((2-methoxyethoxy)methyl)-N-(tetrahydro-2H-pyran-4-yl)-1H-indol-7-amine; <56>(4-(7-(cyclopentylamino)-5-((2-methoxyethoxy)methyl)-1H-indol-2-yl)phenyl)dimethylphosphine oxide; <57>(4-(5-((2-methoxyethoxy)methyl)-7-((tetrahydro-2H-pyran-4-yl)amino-1H-indol-2-yl)phenyl)dimethylphosphine oxide; <58>4-(4-(7-(cyclopentylamino)-5-((2-methoxyethoxy)methyl)-1H-indol-2-yl)phenyl)-2-methylbut-3-yn-2-ol; <59>4-(4-(5-((2-methoxyethoxy)methyl)-7-((tetrahydro-2H-pyran-4-yl)amino)-1H-indol-2-yl)phenyl)-2-methylbut-3-yn-2-ol; <60>5-((2-methoxyethoxy)methyl)-N-(tetrahydro-2H-pyran-4-yl)-2-(p-tolyl)-1H-indol-7-amine; <61>N-cyclopentyl-5-((3-methoxypropoxy)methyl)-2-phenyl-1H-indol-7-amine; <62>5-((3-methoxypropoxy)methyl)-N-(oxetan-3-yl)-2-phenyl-1H-indol-7-amine; <63>5-((3-methoxypropoxy)methyl-2-phenyl-N-(tetrahydro-2H-pyran-4-yl)-1H-indol-7-amine; <64>5-((2-methoxyethoxy)methyl)-2-phenyl-N-(piperidin-4-yl)-1H-indol-7-amine; <65>5-(3-(2-methoxyethoxy)propyl)-2-phenyl-N-(tetrahydro-2H-pyran-4-yl)-1H-indol-7-amine; <66>N-cyclopentyl-5-(2-(2-methoxyethoxy)ethyl)-2-phenyl-1H-indol-7-amine; <67>5-(2-(2-methoxyethoxy)ethyl)-2-phenyl-N-(tetrahydro-2H-pyran-4-yl)-1H-indol-7-amine; <68>5-((2-methoxyethoxy)methyl)-2-phenyl-7-(piperidin-1-yl)-1H-indole; <69>4-((5-((2-methoxyethoxy)methyl)-2-phenyl-1H-indol-7-yl)amino)cyclohexan-1-ol; and <70>4-((5-((2-methoxyethoxy)methyl)-2-phenyl-1H-indol-7-yl)amino)-1-methylcyclohexan-1-ol.

The present invention further provides a method for preparing the compound of Formula 1. In the following, to help understanding of the present invention, a method for preparing the compound of Formula 1 will be described on the basis of an exemplary reaction formula. However, a person having ordinary skill in the art may prepare the compound of Formula 1 by means of various methods based on the structure of Formula 1, and all such methods shall be interpreted as being included in the scope of the present invention. That is, any combination of various synthesis methods stated in the present specification or disclosed in prior art may be used to prepare the compound of Formula 1, such combinations understood as belonging to the scope of the present invention, and the compound of Formula 1 not limited to that which is described in the following.

First, the method for preparing a compound of Formula 1 may comprise steps of: reacting a compound of Formula 3 and HO-C₁₋₈ alkylene-O-R₇ to prepare a compound of Formula 4; reducing the compound of Formula 4; and preparing the compound of Formula 1 from the reduced compound of Formula 4.

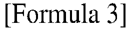

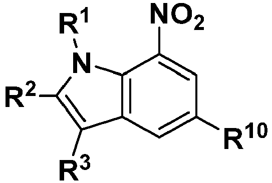

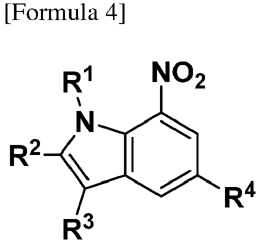

In Formulas above, R¹ to R⁷ are each the same as defined herein, R¹⁰ is -C₁₋₈ alkylene-LG, and LG is a leaving group. Here, the leaving group may be a functional group such as halogen, sulfonic acid ester, or alkoxy, and is not limited as long as the leaving group may be released from the compound of Formula 3 to prepare the compound of Formula 4.

The step of reacting a compound of Formula 3 and HO-C₁₋₈ alkylene-O-R₇ to prepare a compound of Formula 4 may be a step in which the leaving group of the compound of Formula 3 is substituted by HO-C₁₋₈ alkylene-O-R₇ while using a base to prepare a compound of Formula 4. Here, the base may be Et₃N, DIPEA, DBU, NMP, or K₂CO₃, but is not limited thereto.

The step of reducing the compound of Formula 4 is a step of reducing the nitro group of the compound of Formula 4 to an amine group, which may be carried out using an acid catalyst and a metal, or using a metal catalyst in the presence of hydrogen gas. As the metal, iron, zinc, lithium, sodium, and tin (typically, tin chloride) may be used, and as the acid catalyst, hydrochloric acid, sulfuric acid, nitric acid, phosphoric acid, and the like may be used. Also, in a reduction reaction using a metal catalyst in the presence of hydrogen gas, as the usable metal catalyst, palladium, nickel, platinum, ruthenium, rhodium, and the like may be mentioned, where the pressure of hydrogen gas may be usually 1 to 3 atmospheres.

The step of preparing the compound of Formula 1 from the reduced compound of Formula 4 is a step in which the compound of Formula 4 and a ketone compound or an aldehyde compound are subjected to reductive amination reaction to prepare the compound of Formula 1, where in the reductive amination reaction, NaBH(OAc)₃ or NaBH₃CN may be used, without being limited thereto.

Here, the ketone compound or the aldehyde compound is not limited as long as it is a ketone or aldehyde compound capable of reacting with the amine group of the compound of Formula 4 to form R⁵ and R⁶ substituents as in the compound of Formula 1, and having an oxo (=O) group.

For example, when cyclopentanone is used as the ketone compound, the compound of Formula 1 in which one or more of R⁵ and R⁶ are cyclopentyl may be prepared.

In addition, as long as the reaction proceeds smoothly in each manufacturing step, the conditions may not be limited.

Meanwhile, a method for preparing the compound of Formula 3 is described in WO 2009-025478 A1. For example, the compound of Formula 3 in which the leaving group is a halogen may be prepared by reduction of a carboxylic acid or ester to form a hydroxyalkyl, followed by halogenation of the hydroxy group.

The present invention further provides a pharmaceutical composition comprising the compound of Formula 1, an isomer thereof, a solvate thereof, a hydrate thereof, or a pharmaceutically acceptable salt thereof; and a pharmaceutically acceptable carrier.

A pharmaceutical composition for prevention or treatment of cell necrosis or ferroptosis-related diseases selected from the following group, the composition comprising the compound of Formula 1 of Claim 1, an isomer thereof, a hydrate thereof, a solvate thereof, or a pharmaceutically acceptable salt thereof as an active ingredient, and a pharmaceutically acceptable carrier:
The cell necrosis and ferroptosis-related diseases include acute/chronic hepatic disease (e.g. hepatitis, hepatic fibrosis, hepatocirrhosis), neurodegenerative disease (e.g. dementia, Parkinson's disease, Huntington's disease), ischemic cardiac disease, reperfusion injury, ischemic stroke or ischemic injury, pancreatitis, bacterial/viral sepsis, diabetes mellitus or diabetic complications, diabetic vascular disease [in particular, these diabetes are caused by pancreatic cell destroying substances, and mediated by virus, hyperglycemia, fatty acid, diet, toxin, streptozotocin and the like], necrotizing procolitis, cystic fibrosis, rheumatoid arthritis, degenerative arthritis, nephropathy, bacterial infection, viral infection (e.g. HIV), multiple sclerosis, leukemia, lymphoma, neonatal respiratory distress syndrome, asphyxia, tuberculosis, endometriosis, angiasthenia, psoriasis, chilblain, steroid treatment complications, gangrene, pressure sores, hemoglobinuria, burns, hyperthermia, Crohn's disease, celiac disease, compartment syndrome, spinal cord injury, glomerulonephritis, renal failure, muscular dystrophy, metabolic inherited disease, mycoplasma disease, anthrax, Andersen's disease, congenital mitochondrial disease, phenylketonuria, placental infarction, syphilis, osteonecrosis and the like. In addition, necrosis and associated diseases caused by drugs and toxic substances are selected from the group consisting of the necrosis associated with alcoholism, the exposure to, and/or administration and/or self-administration of, cocaine, drugs (e.g., paracetamol), antibiotics, anti-cancer agent, Adriamycin, puromycin, bleomycin, NSAID, cyclosporine, chemical toxins (e.g., carbon tetrachloride, cyanide, methanol, ethylene glycol), poison gas, agrochemicals pesticides, heavy metals (e.g., lead, mercury, cadmium), or injury due to the exposure to radioactivity/UV and associated necrosis thereof.

Further, the compound of Formula 1 is additionally expected to exhibit preventive or therapeutic and improvement effects in cell necrosis and ferroptosis-related diseases of acute/chronic renal disease, traumatic brain damage, the neurogenerative disease of amyotrophic lateral sclerosis, necrotizing colitis, viral infection (e.g. SARS-CoV), skin disease including psoriasis and allergic dermatitis, and organ preservation / organ transplant (see Korean Registered Patents 10-1098583 and 10-1941004).

Further, a pharmaceutical composition comprising the compound of Formula 1 is able to regulate intracellular calcium, and is able to improve ER stress and mitochondrial dysfunction due to abnormal intracellular calcium levels.

In addition, the compound of Formula 1 can inhibit cell death through ferroptosis caused by accumulation of lipid peroxides, which is indicated by the Fenton reaction, and GPX4 pathway interference by Erastin, glutamate, or RSL3, and the like, which are ferroptosis-inducing substances.

Accordingly, a pharmaceutical composition comprising the compound of Formula 1 is expected to exhibit preventive or therapeutic and improvement effects with regard to cell necrosis and ferroptosis-related diseases. Associated diseases are as follow.
Chronic inflammatory pulmonary disease including acute lung injury syndrome / acute pulmonary disease, pneumonia, tuberculosis, asthma, pulmonary hypertension, chronic obstructive pulmonary disease, idiopathic pulmonary fibrosis and cystic fibrosis (See Mitochondrial dysfunction in fibrotic diseases. Cell Death Discov. 2020 Sep 5;6:80*.* Mitochondrial dysfunction in lung aging and diseases. Eur Respir Rev. 2020 Oct 15;29(157):200165*,* see Korean registered patent 10-1636563)
Demyelinating disease including demyelination and amyotrophic lateral sclerosis (ALS), hypertension including pulmonary hypertension, stroke, prion disease, epilepsy, ataxia, migraine, reduced cognitive skill, seizure, tremors, psychiatric disorders (e.g. depression) (See Neuronal and glial calcium signaling in Alzheimer's disease. Cell Calcium. Oct-Nov 2003;34(4-5):385-97*.* Mitochondrial disorders: challenges in diagnosis & treatment. Indian J Med Res. 2015 Jan;141(1):13-26*.)*
Insulin resistance, hyperlipidemia, atherosclerosis, inflammatory bowel disease (IBD) including Crohn's Disease and ulcerative colitis, various cancers and metastasis of cancer (See reticulum stress and oxidative stress in cell fate decision and human disease. Antioxid Redox Signal. 2014 Jul 20;21(3):396-413*.)*

Visual impairment-associated disease (e.g. macular degeneration, retinitis pigmentosa, optic neuropathy, cataract, glaucoma), anemia, cholestasis, hypoparathyroidism, pancytopenia, pancreatic disorder, lactic acidosis, lactacidaemia, hearing loss, short stature, intestinal obstruction, cardiac conduction defect, cardiomyopathy, endometriosis, infertility, early menopause (See Mitochondrial diseases: the contribution of organelle stress responses to pathology. Nat Rev Mol Cell Biol. 2018 Feb;19(2):77-92*.* Seminars in medicine of the Beth Israel Hospital, Boston. Mitochondrial DNA and disease. N Engl J Med. 1995 Sep 7; 333(10): 638-44*.* Mitochondrial injury and dysfunction in hypertension-induced cardiac damage. Eur Heart J. 2014 Dec 7; 35(46): 3258-3266*.)*

Muscular atrophy including limb girdle/Becker muscular dystrophy (GGMD/BMD) and Duchenne muscular dystrophy (DMD) (Duchenne muscular dystrophy is associated with the inhibition of calcium uniport in mitochondria and an increased sensitivity of the organelles to the calcium-induced permeability transition. See Biochim Biophys Acta Mol Basis Dis. 2020 May 1;1866(5):165674*.)*

Aging and aging-related diseases (See Interrelation between ROS and Ca2+ in aging and age-related diseases. Redox Biology. 2020 ; 6:101678*.).*

A pharmaceutical composition comprising the compound of Formula 1 exhibits not only liver protection and liver function improvement effects, but also preventive and therapeutic effects against acute and chronic liver diseases such as fatty liver, hepatic fibrosis, liver cirrhosis, and virus or drug-induced hepatitis. Further, as a result [the pharmaceutical composition] may prevent or treat liver disease complications such as portal hypertension, but is not limited to such. Even further, the pharmaceutical composition according to the present invention is effective in preventing and treating liver disease selected from among liver transplant, alcoholic or non-alcoholic fatty liver disease (See Korean Registered Patent 10-2006247), hepatic fibrosis, liver cirrhosis, and virus or drug-induced hepatitis, and is effective against acute and chronic alcoholic liver disease. Further, the composition according to the present invention is effective in treating or preventing fatty liver caused by fatty acids or acute or chronic liver disease caused by fatty liver.

The compound of Formula 1 may include a stem cell culturing step to improve the differentiation efficiency and maturity of stem cell-derived cardiomyocytes.

Further, the compound of Formula 1 can be used to prevent and treat mucositis.

More specifically, the compound of Formula 1 may be used for preventing or treating heart disease such as ischemic heart disease, cardiac conduction defect, cardiomyopathy, myocardial infarction and heart failure, neurodegenerative disease such as dementia, Parkinson's disease and amyotrophic lateral sclerosis, renal ischemia-reperfusion injury and fibrosis inhibition, acute and chronic respiratory disease and macular degeneration, and the like.

In this specification, the "treatment" means stopping or delaying progression of a disease or reversing or alleviating its symptoms when used in an object showing symptoms of onset, and the "prevention" means stopping or delaying indication of a disease when used in an object that does not show symptoms of onset, but is at high risk.

In the present invention, the "administration" means providing a given compound of the present invention to a subject by any suitable method.

In the present invention, "pharmaceutical composition" may comprise pharmaceutically acceptable carriers, together with the compounds of the present invention.

The compound of Formula 1 according to the present invention may be administered in various oral and non-oral dosage forms for clinical administration, and when formulated, is prepared using diluents or excipients such as commonly used fillers, bulking agents, binding agents, wetting agents, disintegrating agents, surfactants.

Solid formulations for oral administration include tablets, pills, powders, granules, capsules and troches, and such solid formulations are prepared by mixing at least one of the compound of the present invention with at least one excipient, for example, starch, calcium carbonate, sucrose, lactose or gelatin. Further, in addition to simple excipients, lubricating agents such as magnesium stearate talc are used. Liquid formulations for oral administration include suspensions, liquids for internal use, emulsions or syrups, and in addition to the commonly used simple diluents water and liquid paraffin, various excipients, for example wetting agents, sweeteners, aromatics and preservatives may be included.

Formulations for non-oral administration include sterile aqueous solutions, non-aqueous solvents, suspensions, emulsions, lyophilized preparations, suppositories. As the non-aqueous solvent and the suspension solvent, propylene glycol, polyethylene glycol, vegetable oil such as olive oil, injectable ester such as ethyl oleate, and the like can be used. As the base of the suppository, witepsol, macrogol, tween 61, cacao butter, laurin butter, glycerol, gelatin and the like can be used.

Further, the effective human dose for the compound of Formula 1 according to the present invention may vary depending on the age, body weight, sex, mode of administration, health status and severity of illness of the patients, and is normally approximately 0.001-100mg/kg/day, preferably 0.01-35 mg/kg/day. For an adult patient with a body weight of 70kg, it is normally 0.07-7000mg/day, preferably 0.7-2500mg/day, and depending on the judgment of a physician or pharmacist [the compound of Formula 1] may be administered once a day or divisionally across multiple administrations at certain time intervals.

A pharmaceutical composition comprising the compound of Formula 1, an isomer thereof, a hydrate thereof, a solvate thereof, or a pharmaceutically acceptable salt thereof as an active ingredient may be used to be administered as a single therapeutic agent, or to be administered in combination with other therapeutic agents in use.

Another aspect of the present invention provides a method for preventing or treating cell necrosis or ferroptosis-related diseases comprising a step of administering the compound of Formula 1, an isomer thereof, a hydrate thereof, a solvate thereof, or a pharmaceutically acceptable salt thereof to a subject in need thereof in a pharmaceutically effective amount.

In the present invention, the cell necrosis or ferroptosis-related disease may involve lipid peroxidation.

In the present invention, the "lipid peroxidation" means oxidative degradation of fats, oils, waxes, sterols, triglycerides, and the like, and the lipid peroxidation is considered as one of the main causes of the development of various degenerative diseases.

In the present invention, the cell necrosis or ferroptosis-related disease may be, specifically, neurodegenerative disease, liver disease, kidney disease, stroke, myocardial infarction, ocular disease or lung disease.

The neurodegenerative disease may be one or more selected from the group consisting of Alzheimer's disease, Parkinson's disease, epilepsy, Huntington's disease, amyotrophic lateral sclerosis, Friedreich's ataxia, multiple sclerosis, CMT (Charcot-Marie-Tooth) disease, dementia with Lewy bodies, and traumatic brain Injury.

Another aspect of the present invention provides a method for suppressing ferroptosis comprising a step of administering the compound of Formula 1, an isomer thereof, a hydrate thereof, a solvate thereof, or a pharmaceutically acceptable salt thereof, to a subject in need thereof in a pharmaceutically effective amount.

In the present invention, the compound of Formula 1 may interfere or inhibit ferroptosis by acting on Erastin, glutamate, or RSL3, and the like, which are ferroptosis-inducing substances, thereby inhibiting cell death.

Another aspect of the present invention provides a method for reducing reactive oxygen species (ROS) in cells comprising a step of contacting the compound of Formula 1, an isomer thereof, a hydrate thereof, a solvate thereof, or a pharmaceutically acceptable salt thereof with the cells.

In this specification, the "reactive oxygen species (ROS)" mean chemically active molecules, such as free radicals, containing oxygen, and an example of the reactive oxygen species may include oxygen ions and peroxides. The ferroptosis is characterized by rapid accumulation of reactive oxygen species in an iron-dependent manner, and the compound of Formula 1 can inhibit ferroptosis by inhibiting reactive oxygen species.

In the present invention, the "subject" in need of administration may include both mammals and non-mammals. Here, an example of the mammal may include humans, non-human primates such as chimpanzees or monkey species, livestock animals such as cattle, horse, sheep, and the like, but is not limited thereto.

Another aspect of the present invention provides a use of the compound of Formula 1, an isomer, a solvate thereof, or a pharmaceutically acceptable salt thereof, for prevention or treatment of cell necrosis or ferroptosis-related diseases.

The use and the prevention or treatment method of the present invention may be applied mutatis mutandis to the contents of the pharmaceutical composition.

### [Effects of Invention]

The compound of Formula 1 according to the present invention can exhibit cell necrosis inhibitory efficacy in various cells such as heart, kidney, nerve, retina, liver, or lung cells, and in particular can effectively inhibit ferroptosis.

In addition, the compound of Formula 1 according to the present invention can exhibit excellent pharmacokinetic values in plasma and brain.

Accordingly, the compound of Formula 1 according to the present invention can be usefully used for prevention or treatment of cell necrosis or ferroptosis-related diseases in various cells.

### [Brief Description of Drawings]

FIG. 1 is a graph of the calcium concentration regulation effect of the compound of Example 2 under the t-BHP treatment conditions of Experimental Example 2.
FIG. 2 is a time v. average plasma and brain concentration graph following oral administration of the compound of Example 2 according to Experimental Example 9 (ICR mouse, 10 mg/kg).
FIG. 3 is a time v. average plasma and brain concentration graph following oral administration of the compound of the Comparative Example according to Experimental Example 9 (ICR mouse, 10 mg/kg).

### [Best Modes for the Invention]

The advantages and characteristics of the present invention, and method for achieving the same, shall become evident with reference to the embodiments which are described in detail in the following. However, the present invention is not limited to the embodiments disclosed in the following, and may be realized in various different forms. The present embodiments are solely intended to complete the disclosure o the present invention, and to inform persons having ordinary skill in the art of the full scope of the invention; the present invention is defined solely by the appended claims.

### [Examples]

The following preparation examples describe in further detail the preparation of intermediates necessary for synthesis of the compounds according to embodiments of the present invention. The abbreviations used in the following preparation examples and examples are as follow.
Ac: acetyl
ACN: acetonitrile
BOC: t-butoxycarbonyl
Bn: benzyl
Bu: butyl
DBU: 1,8-diazabicyclo(5,4,0)undec-7-en
DCM: dichloromethane
DIBAL-H: diisobutylaluminum hydride
DIPEA: diisopropylethylamine
DMF: N,N-dimethylformamide
DMSO: dimethylsulfoxide
EA: ethyl acetate
EtOH: ethyl alcohol
Et: ethyl
HATU: (1-[bis(dimethylamino)methylene]-1H-1,2,3-triazolo[4,5-b]pyridinium 3-oxide hexafluorophosphate
Hex: hexane
EDC: 1-ehtyl-3-(3-dimethylaminopropyl)carbodiimide
Me: methyl
NMM: N-methylmorpholine
NBS: N-bromosuccinimide
NIS: N-iodosuccimide
PE: petroleum ether
Pd(dppf)Cl₂: [1,1' -bis(diphenylphosphino)ferrocene]dichloropalladium(II)
Ph: phenyl
TBAF: tetrabutylammonium fluoride
TEA: triethylamine
TFA: trifluoroacetic acid
THF: tetrahydrofuran
TMS: trimethylsilyl

### The compounds of the examples of the present invention may be prepared in accordance with Reaction Formula 1 or 2 below. Reaction Formula 1 illustrates a case where R² is a substituent other than hydrogen, and Reaction Formula 2 illustrates a case where R³ is a substituent other than hydrogen.

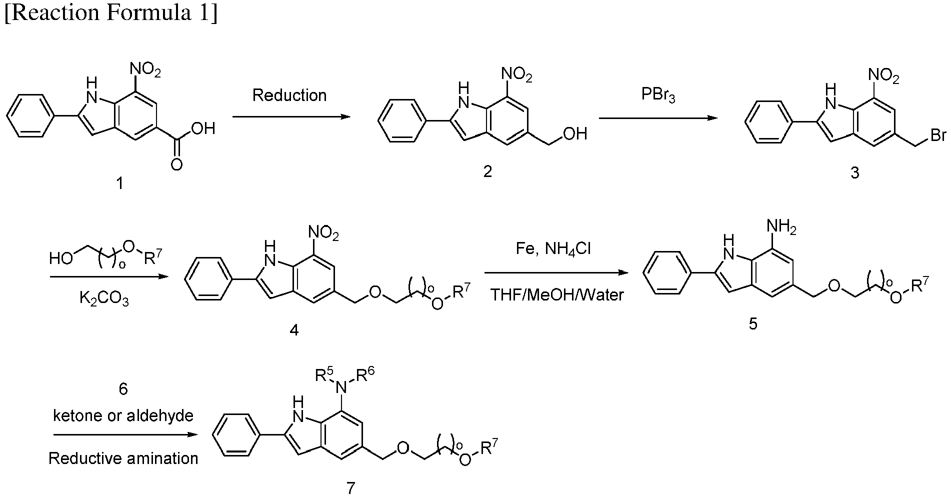

In Reaction Formula 1, Compounds **1** and **2** were synthesized according to the method described in Korean Registered Patent No. 10-1511771.

Intermediate **3** obtained using PBr₃, and an alkoxyalkyl alcohol (HO-CH₂(CH₂)ₒ-O-R⁷) and a base may be used to synthesize Compound **4.** As the base, Et₃N, DIPEA, DBU, NMP, K₂CO₃, and the like may be used.

Compound **4** may be subjected to the reduction reaction by using a metal and an acid catalyst, or by using a metal catalyst in the presence of hydrogen gas to obtain Compound **5.** As the metal, Fe, Zn, Li, Na, or the like may be used, and as the acid catalyst, an inorganic acid such as hydrochloric acid, sulfuric acid, or nitric acid, an organic acid such as AcOH or TFA, or NH₄Cl, and the like may be used. The reduction reaction may be performed using Pd, Ni, Pt, Rt, Rh, or the like as the metal catalyst in the presence of hydrogen gas.

Compound **5,** and Compound **6,** which is a ketone compound or an aldehyde compound, may be subjected to the reductive amination reaction using NaBH(OAc)₃ or NaBH₃CN, and the like with NaBH(OAc)₃ to obtain Compound **7.**

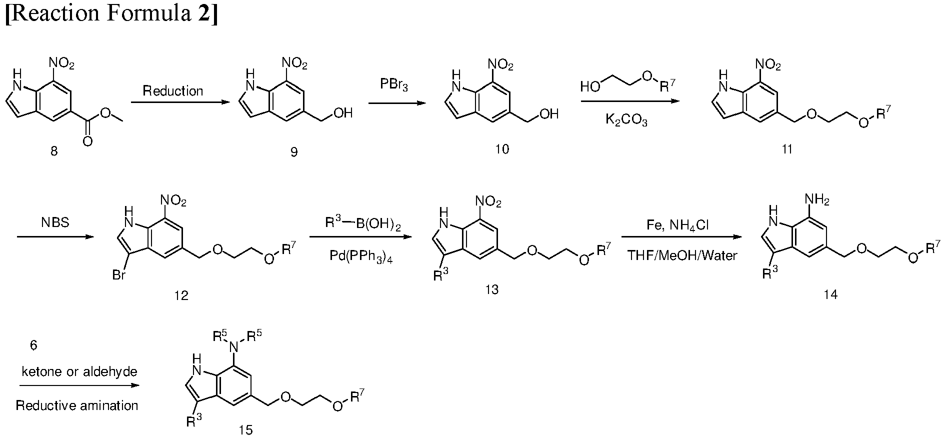

In Reaction Formula 2, Compound **8** is commercially available, and can also be obtained according to CN110818609. The intermediate of Compound **10** obtained by performing reduction and bromination with Compound 8 in a conventional manner may be reacted using 2-alkoxyethyl alcohol (HO-CH₂CH₂-O-R⁷) and a base in the same method as in Scheme 1 to obtain Compound **11.** Bromine is introduced at position 3 of its indole ring using NBS to obtain Compound **13** substituted with R³ by Suzuki reaction, which may proceed in the same manner as for obtaining Compounds 5 and 7 from Compound 4 of Scheme 1 to obtain Compounds **14** and **15.**

Preparation Examples 1 to 42 below show intermediate compounds for preparing example compounds of the present invention, and methods for synthesizing the same.

### Preparation Example 1: (7-nitro-2-phenyl-1H-indol-5-yl)methanol

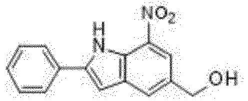

7-nitro-2-phenyl-1H-indol-5-carboxylic acid (1.0 g, 3.45 mmol) was added to THF (10 mL) and cooled to 0°C. 2M BH₃ · SMe₂/THF (5.3 mL), 10.6 mmol) was slowly added dropwise, followed by stirring for 30 minutes at the same temperature. The reaction mixture was warmed to room temperature and stirred for 5 hours. The reaction mixture was cooled to 0°C, and 1N NaOH aqueous solution (10 mL) was slowly added dropwise over 30 minutes. After 1 hour of additional stirring, EA was added, and after 2 extractions the organic layer was collected and washed with brine. The organic layer was dried with MgSO₄ and filtered. The filtrate was concentrated under reduced pressure, then EtOH was added to the concentration residue to crystallize, followed by filtration and vacuum drying to obtain the title compound (770 mg, 71%) as a yellow solid.

¹H NMR (400MHz, CDCl₃); δ 10.08 (br, 1H), 8.17 (s, 1H), 7.97 (s, 1H), 7.76 (d, 2H), 7.51 (t, 2H), 7.49 (t, 1H), 6.94 (s, 1H), 4.87 (s, 2H), 1.81 (t, 1H).

### Preparation Example 2: 5-(bromomethyl)7-nitro-2-phenyl)-1H-indol

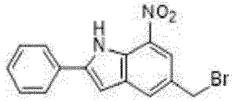

The (7-nitro-2-phenyl-1H-indol-5-yl)methanol (7.6 g, 28.33 mmol) obtained in Preparation Example 1 was added to THF (76 mL) and cooled to 0°C. PBr₃ (1.6 mL, 17.0 mmol) was slowly added dropwise before bringing to room temperature and stirring for 2 hours. The reaction mixture was poured into ice water to stop the reaction, and extracted with EA. The organic layer was dried with MgSO₄ and filtered. The filtrate was concentrated under reduced pressure, then diethyl ether was added to the concentration residue to crystallize, followed by filtration and vacuum drying to obtain the title compound (7.5 g, 80%) as a yellow solid.

¹H NMR (400MHz, CDCl₃); δ 10.04 (br, 1H), 8.13 (s, 1H), 7.94 (s, 1H), 7.72 (d, 2H), 7.50 (t, 2H), 7.42 (t, 1H), 6.90 (s, 1H), 4.84 (s, 2H)

### Preparation Example 3: 5-(2-methoxyethoxymethyl)-7-nitro-2-phenyl-1H-indol

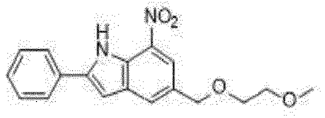

To 2-methoxyethanol (156 mL, excess), K₂CO₃ (6.5 g, 47.11 mmol) was added in an argon atmosphere, and stirred for 20 minutes at room temperature. At room temperature, the 5-(bromomethyl)-7-nitro-2-phenyl-1H-indole (7.8 g, 23.55 mmol) obtained in Preparation Example 2 was added over 5 times. After stirring for 2 hours at room temperature, then confirming the reaction was stopped with TLC, water and EA were added and worked-up. The organic layer was then dried with MgSO₄, then dried and concentrated under reduced pressure. The concentration residue was purified by silica gel column chromatography (Hex: EA = 3:1 ~ 2:1) to obtain the title compound (3.3 g, 43%) as a yellow solid.

¹H NMR (400MHz, CDCl₃); δ 10.06 (br, 1H), 8.13 (s, 1H), 7.94 (s, 1H), 7.71 (d, 2H), 7.49 (t, 2H), 7.47 (t, 1H), 4.70 (s, 2H), 3.68 (m, 2H), 3.60 (m, 2H), 3.40 (s, 3H).

### Preparation Example 4: 5-(2-methoxyethoxymethyl)-2-phenyl-1H-indol-7-amine

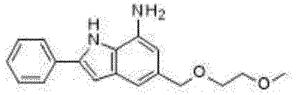

The 5-(2-methoxyethoxymethyl)-7-nitro-2-phenyl-1H-indole (1.91 g, 5.85 mmol) obtained in Preparation Example 3 was dissolved in a mixed solution of MeOH (20 mL) / THF (6 mL) / H₂O (4mL), followed by addition of Fe powder (3.92 g, 70.23 mmol, 12 eq) and NH₄Cl (6.26 g, 117 mmol, 20 eq) and heating to 80°C and stirring for 18 hours. After confirming completion of the reaction with HPLC, the reaction mixture was cooled to room temperature and filtered using a celite pad. H₂O (20 mL) was added to the filtrate, which was extracted with EtOAc (50 mL x 2). The organic layer was washed again with H₂O (30 mL) and brine (20 mL), dried with MgSO₄, filtered and concentrated to obtain the title compound as a brown solid (1.43 g, 75%), which was used in the next reaction without additional purification.

¹H NMR (400MHz, DMSO-d₆); δ 10.91 (br, 1H), 7.81 (d, 2H), 7.46 (t,2H), 7.30 (t, 1H), 6.76 (s, 1H), 6.74 (s, 1H), 6.34 (s, 1H), 5.18 (br, 2H),4.40 (s, 2H), 3.51-3.46 (m, 4H), 3.25 (s, 3H).

Mass [M+H] = 297.4 (M+1)

### Example 1: 5-(2-methoxyethoxymethyl)-2-phenyl-N-tetrahydropyran-4-yl-1H-indol-7-amine

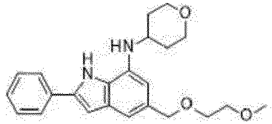

To the 5-(2-methoxyethoymethyl)-2-phenyl-1H-indol-7-amine (500 mg, 1.69 mmol) obtained in Preparation Example 4, DCM (50 mL) and tetrahydro-4H-pyran-4-one (187 µL, 2.02 mmol) were added. After stirring for 1 hour at room temperature, NaBH(OAc)₃ (1.43 g, 6.75 mmol) was added. After stirring for 15 hours at room temperature and confirming completion of the reaction, 1N NaOH aqueous solution (50 mL) was added, followed by stirring for 1 hour. The layers were purified, then dried with MgSO₄ and filtered. The filtrate was concentrated under reduced pressure, and the concentration residue was purified by silica gel column chromatography (Hex/EA, 1/1 + DCM 2%) to obtain the title compound (307 mg, 48%) as a light brown solid.

¹H NMR (400MHz, DMSO-d₆); δ 10.94 (s, 1H), 7.81 (d, 2H), 7.47 (t, 2H), 7.33 (t, 1H), 6.77 (two s, 2H), 6.30 (s, 1H), 5.37 (d, 1H, NH), 4.44 (s, 2H), 3.94 (m, 2H), 3.65 (m, 1H), 3.55-3.45 (m, 6H), 3.25 (s, 3H), 2.06 (m, 2H), 1.48 (m, 2H).

Mass [M+H] = 381.3 (M+1)

### Example 2: N-cyclopentyl-5-(2-methoxyethoxymethyl)-2-phenyl-1H-indol-7-amine

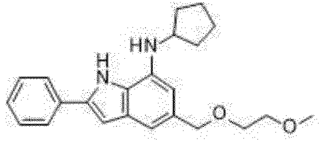

The 5-(2-methoxyethoxymethyl)-2-phenyl-1H-indol-7-amine obtained in Preparation Example 4 and commercially available cyclopentanone (1 equivalent) were reacted using the method of Example 1 to obtain the title compound.

¹H NMR (400MHz, CDCl₃); δ 8.35 (br s, 1H), 7.68 (d, 2H), 7.40 (t,2H), 7.30 (t, 1H), 7.05 (s, 1H), 6.74 (s, 1H), 6.56 (s, 1H), 4.61 (s, 2H), 3.94 (m, 1H), 3.62-3.55 (m, 4H), 3.37 (s, 3H), 2.08 (m, 2H), 1.76 (m, 2H), 1.61 (m, 4H).

Mass [M +H] = 365.5 (M+1)

### Example 3: N,N-dicyclopentyl-5-(2-methoxyethoxymethyl)-2-phenyl-1H-indol-7-amine

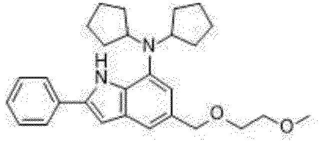

The 5-(2-methoxyethoxymethyl)-2-phenyl-1H-indol-7-amine obtained in Preparation Example 4 and commercially available cyclopentanone (10 equivalents) were reacted using the method of Example 1 to obtain the title compound.

¹H NMR (400MHz, CDCl₃); δ 8.73 (br s, 1H), 7.67 (d, 2H), 7.42 (t, 2H), 7.40 (s, 1H), 7.27 (t, 1H), 7.07 (s, 1H), 6.74 (s, 1H), 4.63 (s, 2H), 3.69 (m, 2H), 3.55 (br s, 4H), 3.37 (s, 3H), 1.80 (m, 4H), 1.32 (m, 8H), 1.32 (m, 4H).

Mass [M+H] = 433.1 (M+1)

### Example 4: 5-2(methoxyethoxymethyl)-2-phenyl-N-(tetrahydropyran-4-ylmethyl)-1H-indol-7-amine

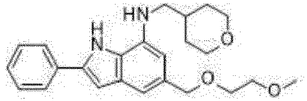

The 5-(2-methoxyethoxymethyl)-2-phenyl-1H-indol-7-amine obtained in Preparation Example 4 and commercially available tetrahydro-2H-pyran-4-carbaldehyde were reacted using the method of Example 1 to obtain the title compound.

¹H NMR (400MHz, CDCl₃); δ 8.41 (br s, 1H), 7.66 (d, 2H), 7.41 (t, 2H), 7.28 (t, 1H), 7.04 (s, 1H), 6.75 (s, 1H), 6.46 (s, 1H), 4.60 (s, 2H), 4.01 (m, 2H), 3.60 (m, 4H), 3.40 (t, 2H), 3.37 (s, 3H), 3.07 (d, 2H), 2.00 (m, 1H), 1.74 (m, 2H), 1.41 (m, 2H).

Mass [M+H] = 395.2 (M+1)

### Example 5: 4-[5-(2-methoxyethoxymethyl)-2-phenyl-1H-indol-7-yl]morpholine

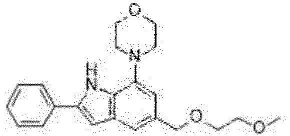

The 5-(2-methoxyethoxymethyl)-2-phenyl-1H-indol-7-amine obtained in Preparation Example 4 (50 mg, 0.153 mmol) was dissolved in DMF (200 L), and K₂CO₃ (23 mg, 0.168 mmol) and 1-bromo-2-(2-bromoethoxy)ethane (21 uL, 0.168 mmol) were added. After heating to 80°C, the mixture was stirred for 22 hours. After confirming termination of the reaction, the mixture was cooled to room temperature. EA and saturated NH₄Cl aqueous solution was added, and the organic layer was isolated. The organic layer was dried with MgSO₄ and filtered. The filtrate was concentrated under reduced pressure, and the concentration residue was purified by silica gel column chromatography (Hex/EA, 2/1) to obtain the title compound as a yellow solid (15 mg, 27%).

¹H NMR (400MHz, CDCl₃); δ 8.37 (br, 1H), 7.70 (d, 2H), 7.47 (t, 2H), 7.32 (t, 1H), 7.31 (s, 1H), 6.89 (s, 1H), 6.80 (s, 1H), 4.66 (s, 2H), 3.66-3.60 (m, 4H), 3.42 (s, 3H), 3.13 (br, 4H), 1.85 (br, 4H), 1.66 (m, 2H).

Mass [M+H] = 365.2 (M+1)

### Example 6: Example 4: 5-(2-methoxyethoxymethyl)-2-phenyl-N-tetrahydrofuran-3-yl-1H-indol-7-amine

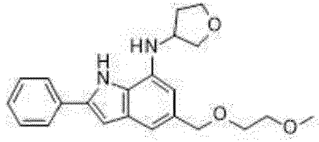

The 5-(2-methoxyethoxymethyl)-2-phenyl-1H-indol-7-amine obtained in Preparation Example 4 and commercially available tetrahydrofuran-3-one were reacted using the method of Example 1 to obtain the title compound.

¹H NMR (400MHz, CDCl₃); δ 8.92 (br, 1H), 7.71 (d, 2H), 7.46 (t, 2H), 7.32 (t, 1H), 7.10 (s, 1H), 6.79 (s, 1H), 6.49 (s, 1H), 4.65 (s, 2H), 4.25 (br, 1H), 4.10-3.90 (m, 4H), 3.64 (m, 4H), 3.41 (s, 3H), 2.31 (m, 2H), 2.13 (m, 2H).

Mass [M+H] = 367.1 (M+1)

### Example 7: 5(2-methoxyethoxymethyl)-N-(oxetane-3-yl)-2-phenyl-1H-indol-7-amine

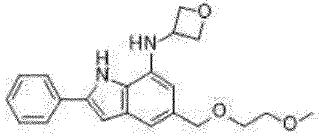

The 5-(2-methoxyethoxymethyl)-2-phenyl-1H-indol-7-amine obtained in Preparation Example 4 and commercially available oxetan-3-one were reacted using the method of Example 1 to obtain the title compound.

¹H NMR (400MHz, CDCl₃); δ 8.57 (br, 1H), 7.68 (d, 2H), 7.42 (t, 2H), 7.30 (t, 1H), 7.07 (s, 1H), 6.77 (s, 1H), 6.09 (s, 1H), 5.04 (m, 2H), 4.55 (m, 5H), 3.59 (m, 4H), 3.38 (s, 3H).

Mass [M+H] = 353.2 (M+1)

### Example 8: N-cyclobutyl-5-(2-methoxyethoxymethyl)-2-phenyl-1H-indol-7-amine

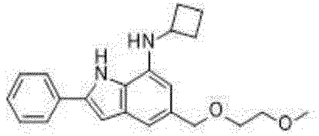

The 5-(2-methoxyethoxymethyl)-2-phenyl-1H-indol-7-amine obtained in Preparation Example 4 and commercially available cyclobutanone (1 equivalent) were reacted using the method of Example 1 to obtain the title compound.

¹H NMR (400MHz, CDCl₃); δ 8.55 (br s, 1H), 7.66 (d, 2H), 7.40 (t, 2H), 7.27 (t, 1H), 7.01 (s, 1H), 6.72 (s, 1H), 6.37 (s, 1H), 4.59 (s, 2H), 3.97 (m, 1H), 3.63-3.57 (m, 4H), 3.36 (s, 3H), 2.47 (m, 2H), 1.83 (m, 4H).

Mass [M+H] = 351.3 (M+1)

### Example 9: N,N-dicyclobutyl-5-(2-methoxyethoxymethyl)-2-phenyl-1H-indol-7-amine

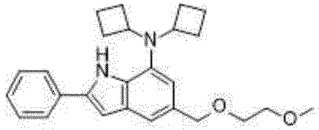

The 5-(2-methoxyethoxymethyl)-2-phenyl-1H-indol-7-amine obtained in Preparation Example 4 and commercially available cyclobutanone (5 equivalents) were reacted using the method of Example 1 to obtain the title compound.

¹H NMR (400MHz, CDCl₃); δ 8.55 (br, 1H), 7.66 (d, 2H), 7.42 (t, 2H), 7.33 (s, 1H), 7.27 (t, 1H), 6.83 (s, 1H), 6.75 (s, 1H), 4.62 (s, 2H), 3.77 (m, 2H), 3.54 (m, 4H), 3.37 (s, 3H), 1.97 (br, 4H), 1.77 (m, 4H), 1.51 (m, 4H).

Mass [M+H] = 405.4 (M+1)

### Preparation Example 5: 5-(2-methoxyethoxymethyl)-1-methyl-7-nitro-2-phenyl-indole

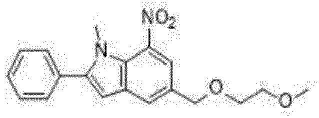

The 5-(2-methoxyethoxymethyl)-7-nitro-2-phenyl-1H-indole (200 mg, 0.612 mmol) obtained in Preparation Example 3 was added to THF and the mixture was cooled to 0°C. NaH (60% in mineral oil dispersion, 28 mg, 1.735 mmol) was added in small amounts, followed by stirring for 30 minutes at the same temperature. CH₃I(130 mg, 0.918 mmol) was added dropwise before raising the temperature to room temperature and stirring for 15 hours. H₂O was added to terminate the reaction, followed by extracting 2 times with EA. The organic layer was collected, dried with MgSO₄, and filtered. The filtrate was concentrated under reduced pressure, and the concentration residue was purified by silica gel column chromatography (Hex/EA, 4/1) to obtain the title compound (88 mg, 42%) as a yellow solid.

Mass [M+H] =341.3 (M+1)

### Preparation Example 6: 5-(2-methoxyethoxymethyl)-1-methyl-2-phenyl-indol-7-amine

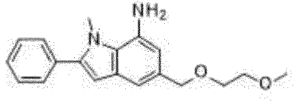

The {5-(2-methoxyethoxymethyl)-1-methyl-7-nitro-2-phenyl-indole} (88 mg, 0.258 mmol) obtained in Preparation Example 5 was added to THF/MeOH/H₂O (1.76 mL / 1.76 mL / 1.76 mL), and Fe powder (144 mg, 2.585 mmol) and NH₄Cl (69 mg, 1.29 mmol) were added. The mixture was heated to 60°C and stirred for 2 hours, then termination of the reaction was confirmed. The mixture was cooled to room temperature and filtered through a celite pad, then washed with EA. The filtrate was concentrated under reduced pressure, extracted with EA and saturated NH₄Cl aqueous solution, and the water layer was re-extracted with EA. The EA layer was collected and dried with MgSO₄, then filtered. The filtrate was concentrated under reduced pressure, and the concentration residue was purified by silica gel column chromatography (Hex/EA, 1/1) to obtain the title compound (32 mg, 40%) as a brown solid.

Mass [M+H] = 311.5 (M+1)

### Example 10: N-cyclobutyl-5-(2-methoxyethoxymethyl)-1-methyl-2-phenyl-indol-7-amine

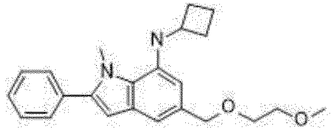

The {5-(2-methoxyethoxymethyl)-1-methyl-2-phenyl-indol-7-amine} (32 mg, 0.103 mmol) obtained in Preparation Example 6 was dissolved in DCM (1.6 mL), and cyclobutanone (7 µL, 0.093 mmol) was added. NaBH(OAc)₃ (65 mg, 0.309 mmol) was added, and the mixture was stirred for 15 hours at room temperature. After confirming the completion of the reaction, 1N NaOH aqueous solution was added and stirred for 30 minutes. The layers were purified, and the organic layer was dried with MgSO₄ and filtered. The filtrate was concentrated under reduced pressure, and the concentration residue was purified by silica gel column chromatography (Hex/EA, 2/1) to obtain the title compound (27 mg, 70%) as a light brown solid.

¹H NMR (400MHz, CDCl₃); δ 7.47-7.30 (m, 5H), 7.00 (s, 1H), 6.44 (s,1H), 6.40 (s, 1H), 4.59 (s, 2H), 4.01 (m, 1H), 3.94 (s, 3H), 3.55 (m, 4H), 3.38 (s, 3H), 2.62 (m, 2H), 1.85 (m, 4H).

Mass [M+H] = 365.2 (M+1)

### Example 11: N-(2,2-dimethylpropyl)-5-(2-methoxyethoxymethyl)-2-phenyl-1H-indol-7-amine

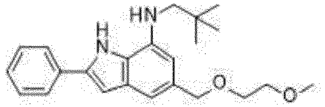

The 5-(2-methoxyethoxymethyl)-2-phenyl-1H-indol-7-amine obtained in Preparation Example 4 and commercially available pivalaldehyde were reacted using the method of Example 1 to obtain the title compound.

¹H NMR (400MHz, CDCl₃); δ 8.17 (br, 1H), 7.67 (d, 2H), 7.42 (t, 2H), .28 (t, 1H), 7.04 (s, 1H), 6.75 (s, 1H), 6.57 (s, 1H), 4.60 (s, 2H), 3.60-3.53 (m, 4H), 3.38 (s, 3H), 3.03 (s, 2H), 1.07 (s, 9H).

Mass [M+H] = 367.3 (M+1)

### Example 12: N-(cyclopentylmethyl)-5-(2-methoxyethoxymethyl)-2-phenyl-1H-indol-7-amine

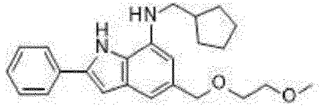

The 5-(2-methoxyethoxymethyl)-2-phenyl-1H-indol-7-amine obtained in Preparation Example 4 and commercially available cyclopentanecarbaldehyde were reacted using the method of Example 1 to obtain the title compound.

¹H NMR (400MHz, CDCl₃); δ 8.41 (br, 1H), 7.72 (d, 2H), 7.46 (t, 2H), 7.30 (t, 1H), 7.10 (s, 1H), 6.79 (s, 1H), 6.58 (s, 1H), 4.64 (s, 2H), 3.63 (m, 4H), 3.42 (s, 3H), 3.20 (d, 2H), 2.29 (m, 1H), 1.90 (m, 2H), 1.67 (m, 4H), 1.37 (m, 2H).

Mass [M+H] = 379.2 (M+1)

### Example 13: N-cyclohexyl-5-(2-methoxyethoxymethyl)-2-phenyl-1H-indol-7-amine

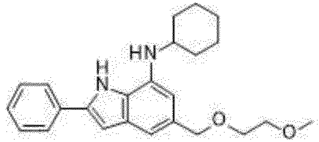

The 5-(2-methoxyethoxymethyl)-2-phenyl-1H-indol-7-amine obtained in Preparation Example 4 and commercially available cyclohexanone were reacted using the method of Example 1 to obtain the title compound.

¹H NMR (400MHz, CDCl₃); δ 8.46 (br, 1H), 7.66 (d, 2H), 7.39 (t, 2H), 7.27 (t, 1H), 7.00 (s, 1H), 6.72 (s, 1H), 6.50 (s, 1H), 4.60 (s, 2H), 3.60 (m, 4H), 3.36 (s, 3H), 3.34 (m, 1H), 2.13 (m, 2H), 1.76 (br m, 2H), 1.65 (m, 1H), 1.36 (m, 2H), 1.21 (m, 3H).

Mass [M+H] = 379.2 (M+1)

### Example 14: N-(4,4-difluorocyclohexyl)-5-(2-methoxyethoxymethyl)2-phenyl-1H-indol-7-amine

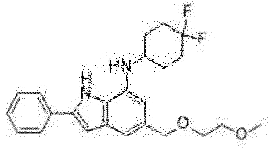

The 5-(2-methoxyethoxymethyl)-2-phenyl-1H-indol-7-amine obtained in Preparation Example 4 and commercially available 4,4-difluorocyclohexan-1-one were reacted using the method of Example 1 to obtain the title compound.

¹H NMR (400MHz, CDCl₃); δ 8.36 (br, 1H), 7.67 (d, 2H), 7.41 (t, 2H), 7.30 (t, 1H), 7.07 (s, 1H), 6.75 (s, 1H), 6.54 (s, 1H), 4.60 (s, 2H), 3.57 (m, 5H), 3.37 (s, 3H), 2.16 (m, 4H), 1.90-1.55 (m, 4H).

Mass [M+H] = 415.2 (M+1)

### Example 15: N-isobutyl-5-(2-methoxyethoxymethyl)-2-phenyl-1H-indol-7-amine

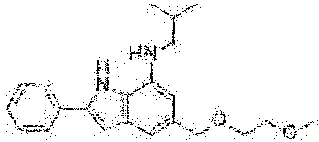

The 5-(2-methoxyethoxymethyl)-2-phenyl-1H-indol-7-amine obtained in Preparation Example 4 and commercially available isobutylaldehyde were reacted using the method of Example 1 to obtain the title compound.

¹H NMR (400MHz, CDCl₃); δ 8.34 (br, 1H), 7.67 (d, 2H), 7.41 (t, 2H), 7.30 (t, 1H), 7.05 (s, 1H), 6.74 (s, 1H), 6.52 (s, 1H), 4.59 (s, 2H), 3.57 (m, 4H), 3.37 (s, 3H), 3.06 (d, 2H), 1.97 (m, 1H), 1.04 (d, 6H).

Mass [M+H] = 353.2 (M+1)

### Example 16: 5-(2-methoxyethoxymethyl)-2-phenyl-N-propyl-1H-indol-7-amine

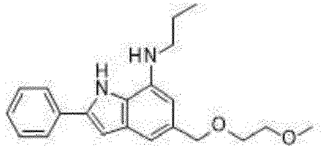

The 5-(2-methoxyethoxymethyl)-2-phenyl-1H-indol-7-amine obtained in Preparation Example 4 and commercially available propylaldehyde were reacted using the method of Example 1 to obtain the title compound.

¹H NMR (400MHz, CDCl₃); δ 8.30 (br, 1H), 7.63 (d, 2H), 7.39 (t, 2H), 7.30 (t, 1H), 7.04 (s, 1H), 6.75 (s, 1H), 6.52 (s, 1H), 4.61 (s, 2H), 3.58 (m, 4H), 3.37 (s, 3H), 3.20 (t, 2H), 1.73 (m, 2H), 1.04 (t, 6H).

Mass [M+H] = 339.2 (M+1)

### Example 17: N-butyl-5-(2-methoxyethoxymethyl)-2-phenyl-1H-indol-7-amine

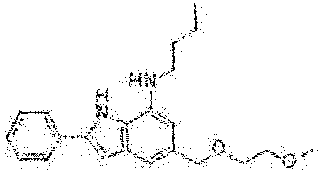

The 5-(2-methoxyethoxymethyl)-2-phenyl-1H-indol-7-amine obtained in Preparation Example 4 and commercially available n-butanal were reacted using the method of Example 1 to obtain the title compound.

¹H NMR (400MHz, CDCl₃); δ 8.20 (br, 1H), 7.66 (d, 2H), 7.41 (t, 2H), 7.30 (t, 1H), 7.05 (s, 1H), 6.75 (s, 1H), 6.54 (s, 1H), 4.61 (s, 2H), 3.58 (m, 4H), 3.37 (s, 3H), 3.24 (t, 2H), 1.73 (m, 2H), 1.50 (m, 2H), 1.04 (t, 6H).

Mass [M+H] = 353.2 (M+1)

### Example 18: N-(2-ethylbutyl)-5-(2-methoxyethoxymethyl)-2-phenyl-1H-indol-7-amine

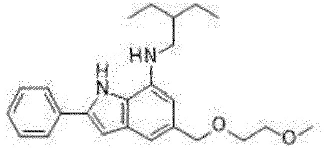

The 5-(2-methoxyethoxymethyl)-2-phenyl-1H-indol-7-amine obtained in Preparation Example 4 and commercially available 2-ethylbutanal were reacted using the method of Example 1 to obtain the title compound.

¹H NMR (400MHz, CDCl₃); δ 8.29 (br, 1H), 7.67 (d, 2H), 7.41 (t, 2H), 7.28 (t, 1H), 7.02 (s, 1H), 6.73 (s, 1H), 6.49 (s, 1H), 4.60 (s, 2H), 3.60 (m, 4H), 3.37 (s, 3H), 3.10 (m, 2H), 1.59 (m, 1H), 1.47 (m, 4H), 0.93 (m, 6H).

Mass [M+H] = 381.2 (M+1)

### Example 19: N-isopentyl-5-(2-methoxyethoxymethyl)-2-phenyl-1H-indol-7-amine

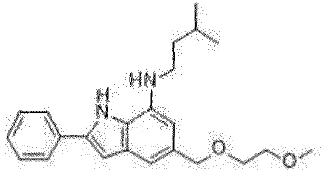

The 5-(2-methoxyethoxymethyl)-2-phenyl-1H-indol-7-amine obtained in Preparation Example 4 and commercially available 3-methylbutanal were reacted using the method of Example 1 to obtain the title compound.

¹H NMR (400MHz, CDCl₃); δ 8.29 (br, 1H), 7.70 (d, 2H), 7.45 (t, 2H), 7.31 (t, 1H), 7.08 (s, 1H), 6.79 (s, 1H), 6.55 (s, 1H), 4.65 (s, 2H), 3.60 (m, 4H), 3.42 (s, 3H), 3.27 (t, 2H), 1.82 (m, 1H), 1.65 (q, 2H), 1.02 (d, 6H).

Mass [M+H] = 367.2 (M+1)

### Example 20: 5-(2(methoxyethoxymethyl)-2-phenyl)-N-sec-butyl-1H-indol-7-amine

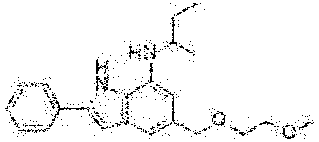

The 5-(2-methoxyethoxymethyl)-2-phenyl-1H-indol-7-amine obtained in Preparation Example 4 and commercially available butane-2-one were reacted using the method of Example 1 to obtain the title compound.

¹H NMR (400MHz, CDCl₃); δ 8.20 (br, 1H), 7.66 (d, 2H), 7.41 (t, 2H), 7.26 (t, 1H), 7.03 (s, 1H), 6.75 (s, 1H), 6.53 (s, 1H), 4.61 (s, 2H), 3.60-3.55 (m, 5H), 3.37 (s, 3H), 1.70 (m, 1H), 1.54 (m, 1H), 1.25 (d, 3H), 1.02 (t, 3H).

Mass [M+H] = 353.2 (M+1)

### Example 21: N-cyclopropylmethyl)-5-(2-methoxyethoxymethyl)-2-phenyl-1H-indol-7-amine

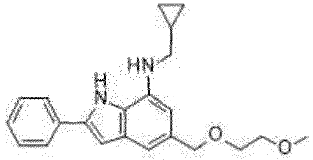

The 5-(2-methoxyethoxymethyl)-2-phenyl-1H-indol-7-amine obtained in Preparation Example 4 and commercially available cyclopropane carbaldehyde were reacted using the method of Example 1 to obtain the title compound.

¹H NMR (400MHz, CDCl₃); δ 8.36 (br, 1H), 7.67 (d, 2H), 7.41 (t, 2H), 7.26 (t, 1H), 7.05 (s, 1H), 6.75 (s, 1H), 6.48 (s, 1H), 4.60 (s, 2H), 3.60-3.55 (m, 4H), 3.37 (s, 3H), 3.07 (d, 2H), 1.20 (m, 1H), 0.59 (m, 2H), 0.29 (m, 2H).

Mass [M+H] = 351.2 (M+1)

### Example 22: 5-(2-methoxyethoxymethyl)-N-(1-methylbutyl)-2-phenyl-1H-indol-7-amine

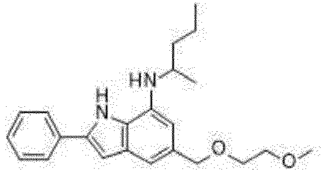

The 5-(2-methoxyethoxymethyl)-2-phenyl-1H-indol-7-amine obtained in Preparation Example 4 and commercially available pentane-2-one were reacted using the method of Example 1 to obtain the title compound.

¹H NMR (400MHz, CDCl₃); δ 8.32 (br, 1H), 7.70 (d, 2H), 7.45 (t, 2H), 7.30 (t, 1H), 7.06 (s, 1H), 6.78 (s, 1H), 6.56 (s, 1H), 4.65 (s, 2H), 3.67-3.58 (m, 5H), 3.41 (s, 3H), 1.68 (m, 1H), 1.51 (m, 3H), 1.28 (d, 3H), 0.98 (t, 3H).

Mass [M+H] = 367.2 (M+1)

### Example 23: N-(1-ethylpropyl)-5-(2-methoxyethoxymethyl)-2-phenyl-1H-indol-7-amine

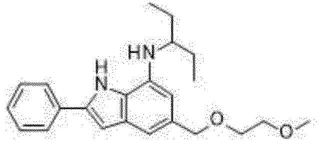

The 5-(2-methoxyethoxymethyl)-2-phenyl-1H-indol-7-amine obtained in Preparation Example 4 and commercially available pentane-3-one were reacted using the method of Example 1 to obtain the title compound.

¹H NMR (400MHz, CDCl₃); δ 8.21 (br, 1H), 7.70 (d, 2H), 7.45 (t, 2H), 7.35 (t, 1H), 7.05 (s, 1H), 6.78 (s, 1H), 6.55 (s, 1H), 4.65 (s, 2H), 3.65-3.55 (m, 5H), 3.45 (m, 1H), 3.41 (s, 3H), 1.67 (m, 4H), 1.02 (t, 6H).

Mass [M+H] = 367.3 (M+1)

### Example 24: (E,Z)-1,1,1-trifluoro-N-[5-(2-methoxyethoxymethyl)-2-phenyl-1H-indol-7-yl]propane-2-imine

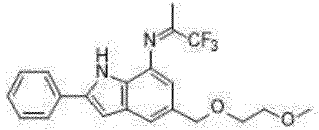

The 5-(2-methoxyethoxymethyl)-2-phenyl-1H-indol-7-amine obtained in Preparation Example 4 (50 mg, 0.169 mmol) was dissolved in DCM (2.5 mL), and 1,1,1-trifluoropropane-2-one (14 ul, 0.152 mmol) was added. NaBH(OAc)₃ (107 mg, 0.507 mmol) was added, then the mixture was stirred for 15 hours at room temperature. After confirming termination of the reaction, 1N NaOH aqueous solution was added, followed by stirring for 30 minutes. After separating the layers, the organic layer was dried with MgSO₄ and filtered. The filtrate was concentrated under reduced pressure, and the concentration residue was purified with silica gel column chromatography (Hex/EA, 2/1) to obtain the title compound as a light brown solid (39mg, 60%).

¹H NMR (400MHz, CDCl₃); δ 8.52 (br, 1H), 7.70 (d, 2H), 7.47 (t, 2H), 7.47 (s, 1H), 7.38 (t, 1H), 6.85 (s, 1H), 6.68 (s, 1H), 4.69 (s, 2H), 3.65-3.60 (m, 5H), 3.41 (s, 3H), 2.28 (s, 3H).

Mass [M] = 391.2 (M+1)

### Example 25: 5-(2-methoxyethoxymethyl)-2-phenyl-N-(2,2,2-trifluoro-1-mthyl-ethyl)-1H-indol-7-amine 1H-indol-7-amine

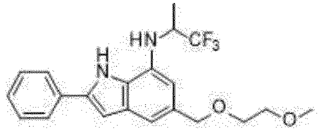

The (E,Z)-1,1,1-trifluoro-N-[5-(2-methoxyethoxymethyl)-2-phenyl-1H-indol-7-yl]propane-2-imine obtained in Example 24 (39 mg, 0.100 mmol) was dissolved in EtOH (2.5 mL), then NaBH₄ (11 mg, 0.300 mmol) was added, followed by stirring for 15 minutes at room temperature. H₂O was slowly added dropwise to terminate the reaction. EA was added to extract two times, and the organic layer was dried with MgSO₄ and filtered. The filtrate was concentrated under reduced pressure and the residue was purified by silica gel column chromatography (Hex/EA, 2/1) to obtain the title compound as a light brown solid (30 mg, 77%).

¹H NMR (400MHz, CDCl₃); δ 8.36 (br, 1H), 7.70 (d, 2H), 7.47 (t, 2H), 7.37 (t, 1H), 7.20 (s, 1H), 6.80 (s, 1H), 6.67 (s, 1H), 4.64 (s, 2H), 4.14 (m, 1H), 3.66-3.59 (m, 4H), 3.42 (s, 3H), 1.50 (d, 3H).

Mass [M] =392.9 (M+1)

### Example 26: N-(1,2-dimethylpropyl)-5-(2-methoxyethoxymethyl)-2-phenyl-1H-indol-7-amine

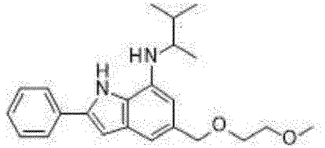

The 5-(2-methoxyethoxymethyl)-2-phenyl-1H-indol-7-amine obtained in Preparation Example 4 and commercially available 3-methylbutane-2-one were reacted using the method of Example 1 to obtain the title compound.

¹H NMR (400MHz, CDCl₃); δ 8.20 (br, 1H), 7.70 (d, 2H), 7.46 (t, 2H), 7.31 (t, 1H), 7.07 (s, 1H), 6.79 (s, 1H), 6.58 (s, 1H), 4.64 (s, 2H), 3.65- 3.55 (m, 5H), 3.41 (s, 3H), 2.01 (m, 1H), 1.22 (d, 3H), 1.09 (d, 3H), 1.02 (d, 3H).

Mass [M+H] = 367.2 (M+1)

### Example 27: N-isopropyl-5-(2-methoxyethoxymethyl)-2-phenyl-1H-indol-7-amine

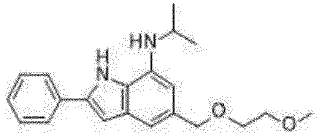

The 5-(2-methoxyethoxymethyl)-2-phenyl-1H-indol-7-amine obtained in Preparation Example 4 and acetone were reacted using the method of Example 1 to obtain the title compound.

¹H NMR (400MHz, CDCl₃); δ 8.27 (br, 1H), 7.66 (d, 2H), 7.40 (t, 2H), 7.28 (t, 1H), 7.03 (s, 1H), 6.73 (s, 1H), 6.53 (s, 1H), 4.61 (s, 2H), 3.76 (m, 1H), 3.60-3.57 (m, 4H), 3.37 (s, 3H), 2.96 (br, 1H), 1.27 (d, 6H).

Mass [M+H] = 339 (M+1)

### Example 28: N-benzyl-5-(2-methoxyethoxymethyl)-2-phenyl-1H-indol-7-amine

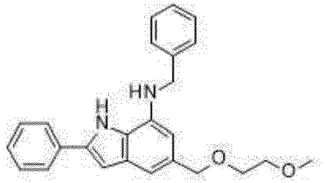

The 5-(2-methoxyethoxymethyl)-2-phenyl-1H-indol-7-amine obtained in Preparation Example 4 and commercially available benzaldehyde were reacted using the method of Example 1 to obtain the title compound.

¹H NMR (400MHz, CDCl₃); δ 8.47 (br s, 1H), 7.63 (d, 2H), 7.43 (t, 2H), 7.36 (m, 4H), 7.28 (m, 2H), 7.04 (s, 1H), 6.75 (s, 1H), 6.45 (s, 1H), 4.54 (s, 2H), 4.30 (s, 2H), 3.57-3.46 (m, 4H), 3.31 (s, 3H).

Mass [M+H] = 387.3 (M+1)

### Example 29: tert-butyl 4-[[5-(2-methoxyethoxymethyl)-2-phenyl-1H-indol-7-yl]amino]piperidine-1-carboxylate

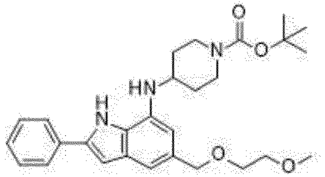

The 5-(2-methoxyethoxymethyl)-2-phenyl-1H-indol-7-amine obtained in Preparation Example 4 (100 mg, 0.337 mmol) was dissolved in DCM (5 mL), and 1-tert-butoxycarbonyl-piperidine-4-one (81 mg, 0.405 mmol) was added. After stirring for 30 minutes, NaBH(OAc)₃ (286 mg, 1.35 mmol) was added. After stirring for 15 hours at room temperature, 1N NaOH aqueous solution was added, followed by stirring for 1 hour. After separating the layers, the organic layer was dried with MgSO₄ and filtered. The filtrate was concentrated under reduced pressure, and the concentration residue was purified by silica gel column chromatography (Hex/EA, 4/1) to obtain the title indicated in the title (20 mg, 12%) as a yellow solid.

¹H NMR (400MHz, CDCl₃); δ 9.36 (br s, 1H), 7.73 (d, 2H), 7.42 (t, 2H), 7.30 (t, 1H), 7.03 (s, 1H), 6.78 (s, 1H), 6.52 (s, 1H), 4.64 (s, 2H), 4.18 (m, 2H), 3.65-3.57 (m, 5H), 3.39 (s, 3H), 2.97 (m, 2H), 2.16 (m, 2H), 1.48 (s, 9H), 1.40 (m, 2H).

Mass [M+H] = 480.3 (M+1)

### Example 30: 1-[4-[[5-(2-methoxyethoxymethyl)-2-phenyl-1H-indol-7-yl]amino]piperidyl]-1-ethanone

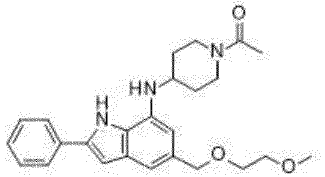

The 5-(2-methoxyethoxymethyl)-2-phenyl-1H-indol-7-amine obtained in Preparation Example 4 and commercially available 1-acetylpiperidine-4-one were reacted using the method of Example 1 to obtain the title compound.

¹H NMR (400MHz, CDCl₃); δ 10.70 (br s, 1H), 7.73 (d, 2H), 7.38 (t, 2H), 7.26 (t, 1H), 6.98 (s, 1H), 6.76 (s, 1H), 6.49 (s, 1H), 4.81 (m, 1H), 4.61 (ABq, 2H), 3.89 (m, 1H), 3.75 (m, 1H), 3.58-3.53 (m, 4H), 3.37 (s, 3H), 3.30 (m, 1H), 2.83 (m, 1H), 2.45 (m, 1H), 2.14 (s, 3H), 2.13 (m, 1H), 1.42 (m, 2H), 1.24 (m, 1H).

Mass [M+H] = 422.3 (M+1)

### Example 31: 5-(2-methoxyethoxymethyl)-N-(1-methylsulfonyl-4-piperidyl)-2-phenyl-1H-indol-7-amine

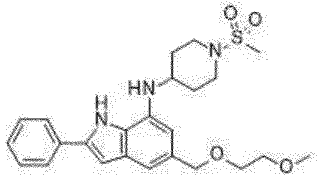

The 5-(2-methoxyethoxymethyl)-2-phenyl-1H-indol-7-amine obtained in Preparation Example 4 and commercially available 1-(methylsulfonyl)piperidine-4-one were reacted using the method of Example 1 to obtain the title compound.

¹H NMR (400MHz, CDCl₃); δ 8.73 (br s, 1H), 7.73 (d, 2H), 7.45 (t, 2H), 7.32 (t, 1H), 7.07 (s, 1H), 6.79 (s, 1H), 6.54 (s, 1H), 4.64 (s, 2H), 3.88 (m, 2H), 3.64-3.57 (m, 5H), 3.39 (s, 3H), 2.89 (m, 2H), 2.86 (s, 3H), 2.30 (m, 2H), 1.70 (m, 2H).

Mass [M+H] = 458.2 (M+1)

### Example 32: N-(1,1-dioxothian-4-yl)-5-(2-methoxyethoxymethyl)-2-phenyl-1H-indol-7-amine

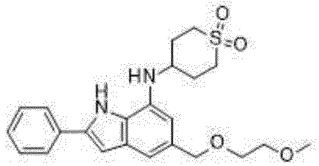

The 5-(2-methoxyethoxymethyl)-2-phenyl-1H-indol-7-amine obtained in Preparation Example 4 and commercially available tetrahydro-4H-thiopyran-4-one 1,1-dioxide were reacted using the method of Example 1 to obtain the title compound.

¹H NMR (400MHz, CDCl₃); δ 9.07 (br s, 1H), 7.72 (d, 2H), 7.41 (t, 2H), 7.29 (t, 1H), 7.02 (s, 1H), 6.74 (s, 1H), 6.22 (s, 1H), 4.54 (s, 2H), 3.67-3.61 (m, 4H), 3.37 (m, 1H), 3.33 (s, 3H), 3.14 (m, 2H), 2.92 (m, 3H), 2.28 (m, 2H), 2.10 (m, 2H).

Mass [M+H] = 429.2 (M+1)

### Example 33: 5-(2-methoxyethoxymethyl)-N-(1-oxothian-4-yl)-2-phenyl-1H-indol-7-amine

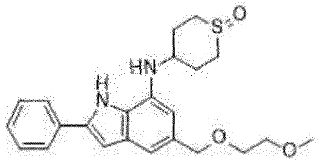

The 5-(2-methoxyethoxymethyl)-2-phenyl-1H-indol-7-amine obtained in Preparation Example 4 and commercially available tetrahydro-4H-thiopyran-4-one 1-oxide were reacted using the method of Example 1 to obtain the title compound.

¹H NMR (400MHz, CDCl₃); δ 10.68 (br s, 1H), 7.82 (d, 2H), 7.46 (t, 2H), 7.31 (t, 1H), 6.99 (s, 1H), 6.76 (s, 1H), 6.45 (s, 1H), 4.64 (s, 2H), 3.69 (m, 1H), 3.62-3.56 (m, 4H), 3.38 (s, 3H). 3.28 (m, 2H), 2.74 (m, 2H), 2.47 (m, 2H), 2.31 (m, 2H).

Mass [M+H] = 413.2 (M+1)

### Example 34: 5-(2-methoxyethoxymethyl)-N-(1-methyl-4-piperidyl)-2-phenyl-1H-indol-7-amine

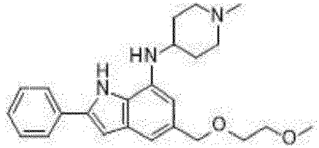

The 5-(2-methoxyethoxymethyl)-2-phenyl-1H-indol-7-amine obtained in Preparation Example 4 and commercially available 1-methylpiperidine-4-one were reacted using the method of Example 1 to obtain the title compound.

¹H NMR (400MHz, CDCl₃); δ 8.81 (br s, 1H), 7.66 (d, 2H), 7.37 (t, 2H), 7.26 (t, 1H), 6.99 (s, 1H), 6.71 (s, 1H), 6.46 (s, 1H), 4.59 (s, 2H), 3.59-3.53 (m, 4H), 3.37 (m, 1H), 3.34 (s, 3H), 2.86 (m, 2H), 2.28 (s, 3H), 2.13 (m, 4H), 1.60 (m, 2H).

Mass [M+H] = 394.3 (M+1)

### Preparation Example 7: 5-(2-ethoxyethoxymethyl)-7-nitro-2-phenyl-1H-indole

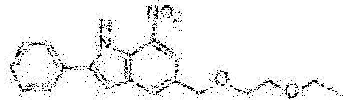

The 5-(bromomethyl)-7-nitro-2-phenyl-1H-indole (1.0 g, 3.019 mmol) obtained in Preparation Example 2 was added to 2-ethoxyethanol (20mL). K₂CO₃ (625 mg, 4.528 mmol) was added, followed by stirring for three hours. After confirming termination of the reaction, EA and H₂O were added to extract. The organic layer was dried with MgSO₄ and filtered. The filtrate was concentrated under reduced pressure, and the concentration residue was purified by silica gel column chromatography (Hex/EA, 4/1) to obtain the title compound as a yellow solid (646 mg, 58%).

Mass [M+H] = 341.4 (M+1)

### Preparation Example 8: 5-(2-ethoxyethoxymethyl)-2-phenyl-1H-indol-7-amine

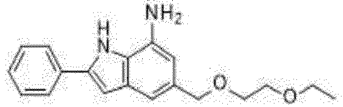

The 5-(2-ethoxyethoxymethyl)-7-nitro-2-phenyl-1H-indol-7-amine (481 mg, 1.413 mmol) obtained in Preparation Example 7 was added to THF/MeOH/H₂O (10 mL/ 10 mL/ 10 mL), then Fe powder (789 mg, 14.131 mmol) and NH₄Cl (377 mg, 7.065 mmol) were added. The mixture was heated to 60°C and stirred for 2 hours, then termination of the reaction was confirmed. The mixture was cooled to room temperature, filtered through a celite pad, and washed with EA. The filtrate was concentrated under reduced pressure and extracted with EA and saturated NH₄Cl aqueous solution, and the water layer was re-extracted with EA. The EA layer was collected and dried with MgSO₄, then filtered. The filtrate was concentrated under reduced pressure, then the concentration residue was purified by silica gel column chromatography (Hex/EA, 2/1) to obtain the title compound as a brown solid (220mg, 50%).

Mass [M+H] = 311.4 (M+1)

### Example 35: 5-(2-ethoxyethoxymethyl)-2-phenyl-N-tetrahydropyran-4-yl-1H-indol-7-amine

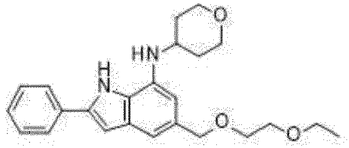

The 5-(2-ethoxyethoxymethyl)-2-phenyl-1H-indol-7-amine obtained in Preparation Example 8 and commercially available tetrahydro-4H-pyran-4-one were reacted using the method of Example 1 to obtain the title compound.

¹H NMR (400MHz, CDCl₃); δ 8.83 (br s, 1H), 7.73 (d, 2H), 7.44 (t, 2H), 7.34 (t, 1H), 7.02 (s, 1H), 6.76 (s, 1H), 6.40 (s, 1H), 4.60 (s, 2H), 4.03 (m, 2H), 3.68 (s, 4H), 3.57-3.48 (m, 5H), 2.08 (m, 2H), 1.53 (m, 2H), 1.19 (t, 3H).

Mass [M+H] = 395.3 (M+1)

### Example 36: N-cyclopentyl-5-(2-ethoxyethoxymethyl)-2-phenyl-1H-indol-7-amine

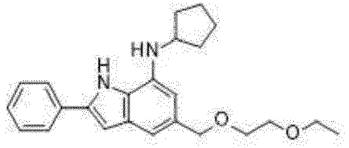

The 5-(2-ethoxyethoxymethyl)-2-phenyl-1H-indol-7-amine obtained in Preparation Example 8 and commercially available cyclopentanone were reacted using the method of Example 1 to obtain the title compound.

¹H NMR (400MHz, CDCl₃); δ 8.41 (br s, 1H), 7.72 (d, 2H), 7.44 (t, 2H), 7.30 (t, 1H), 7.05 (s, 1H), 6.78 (s, 1H), 6.52 (s, 1H), 4.64 (s, 2H), 3.92 (m, 1H), 3.68 (s, 4H), 3.57 (qt, 2H), 2.10 (m, 2H), 1.79-1.55 (m, 6H), 1.23 (t, 3H).

Mass [M+H] = 379.3 (M+1)

### Preparation Example 9: 5-(2-isopropoxyethoxymethyl)-7-nitro-2-phenyl-1H-indole

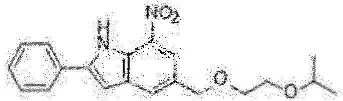

The 5-(bromomethyl)-7-nitro-2-phenyl-1H-indole (1.0 g, 3.019 mmol) obtained in Preparation Example 2 was added to 2-isopropoxyethanol (20mL). K₂CO₃ (834 mg, 6.039 mmol) was added, followed by stirring for two hours. After confirming termination of the reaction, EA and H₂O were added to extract. The organic layer was washed with brine then dried with MgSO₄ and filtered. The filtrate was concentrated under reduced pressure, and the concentration residue was purified by silica gel column chromatography (Hex/EA, 4/1) to obtain the title compound as a yellow solid (630 mg, 63%).

Mass [M+H] = 355.4 (M+1)

### Preparation Example 10: 5-(2-isopropoxyethoxymethyl)-2-phenyl-1H-indol-7-amine

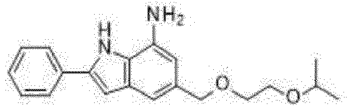

The 5-(2-isopropoxyethoxymethyl)-7-nitro-2-phenyl-1H-indole (630mg, 1.777 mmol) obtained in Preparation Example 9 was added to THF/MeOH/H₂O (12 mL/12 mL/12 mL), then Fe powder (992 mg, 17.776 mmol) and NH₄Cl (475 mg, 8.885 mmol) were added. The mixture was heated to 60°C and stirred for 2 hours, then termination of the reaction was confirmed. The mixture was cooled to room temperature, filtered through a celite pad, and washed with EA. The filtrate was concentrated under reduced pressure and extracted with EA and saturated NH₄Cl aqueous solution, and the water layer was re-extracted with EA. The EA layer was collected and dried with MgSO₄, then filtered. The filtrate was concentrated under reduced pressure, then the concentration residue was purified by silica gel column chromatography (Hex/EA, 1/1) to obtain the title compound as a brown solid (310mg, 53%).

Mass [M+H] = 325.4 (M+1)

### Example 37: N-cyclopentyl-5-(2-isopropoxyethoxymethyl)-2-phenyl-1H-indol-7-amine

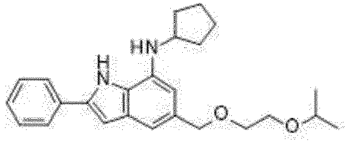

The 5-(2-isopropoxyethoxymethyl)-2-phenyl-1H-indol-7-amine obtained in Preparation Example 10 and commercially available cyclopentanone were reacted using the method of Example 1 to obtain the title compound.

¹H NMR (400MHz, CDCl₃); δ 8.63 (br s, 1H), 7.73 (d, 2H), 7.43 (t, 2H), 7.31 (t, 1H), 7.02 (s, 1H), 6.76 (s, 1H), 6.45 (s, 1H), 4.62 (s, 2H), 3.85 (m, 1H), 3.68 (m, 4H), 3.60 (s, 4H), 3.66 (m, 1H), 1.80 (m, 2H), 1.76 (m, 2H), 1.66 (m, 4H).

Mass [M+H] = 393.3 (M+1)

### Example 38: 5-(2-isopropoxyethoxymethyl)-2-phenyl-Ntetrahydropyran-4-yl-1H-indol-7-amine

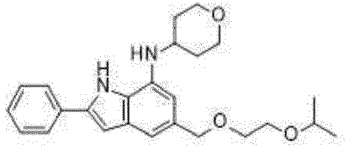

The 5-(2-isopropoxyethoxymethyl)-2-phenyl-1H-indol-7-amine obtained in Preparation Example 10 and commercially available tetrahydro-4H-pyran-4-one were reacted using the method of Example 1 to obtain the title compound.

¹H NMR (400MHz, CDCl₃); δ 8.95 (br s, 1H), 7.75 (d, 2H), 7.43 (t, 2H), 7.30 (t, 1H), 7.00 (s, 1H), 6.75 (s, 1H), 6.35 (s, 1H), 4.59 (s, 2H), 4.02 (m, 2H), 3.70 (m, 4H), 3.60 (m, 1H), 3.50 (m, 3H), 2.07 (m, 2H), 1.51 (m, 2H), 1.14 (d, 6H).

Mass [M+H] = 409.3 (M+1)

### Preparation Example 11: 5-[2-(2-methoxyethoxy)ethoxymethyl]-7-nitro-2-phenyl-1H-indole

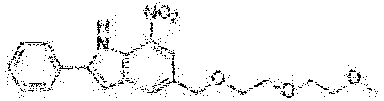

The 5-(bromomethyl)-7-nitro-2-phenyl-1H-indole (1.0 g, 3.019 mmol) obtained in Preparation Example 2 was added to diethylene glycol monomethyl ether (20mL). K₂CO₃ (834 mg, 6.039 mmol) was added, followed by stirring for two hours. After confirming termination of the reaction, EA and H₂O were added to extract. The organic layer was washed with brine then dried with MgSO₄ and filtered. The filtrate was concentrated under reduced pressure, and the concentration residue was purified by silica gel column chromatography (Hex/EA, 2/1) to obtain the title compound as a yellow solid (646 mg, 58%).

Mass [M+H] = 371.4 (M+1)

### Preparation Example 12: 5-[2-(2-methoxyethoxy)ethoxymethyl]-2-phenyl-1H-indol-7-amine

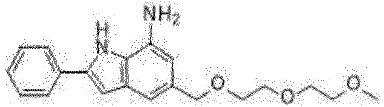

The 5-[2-(2-methoxyethoxy)ethoxymethyl]-7-nitro-2-phenyl-1H-indole (646mg, 1.742 mmol) obtained in Preparation Example 11 was added to THF/MeOH/H₂O (6.5 mL/6.5 mL/6.5 mL), then Fe powder (973 mg, 17.426 mmol) and NH₄Cl (465 mg, 8.71 mmol) were added. The mixture was heated to 60°C and stirred for 2 hours, then termination of the reaction was confirmed. The mixture was cooled to room temperature, filtered through a celite pad, and washed with EA. The filtrate was concentrated under reduced pressure and extracted with EA and saturated NH₄Cl aqueous solution, and the water layer was re-extracted with EA. The EA layer was collected and dried with MgSO₄, then filtered. The filtrate was concentrated under reduced pressure, then the concentration residue was purified by silica gel column chromatography (Hex/EA, 1/4) to obtain the title compound as a brown liquid (140 mg, 24%).

Mass [M+H] = 341.4 (M+1)

### Example 39: N-cyclopentyl-5-[2-(2-methoxyethoxy)ethoxymethyl]-2-phenyl-1H-indol-7-amine

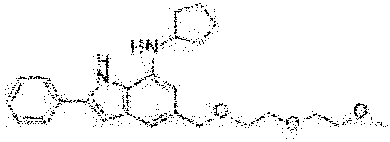

The 5-[2-(2-methoxyethoxy)ethoxymethyl]-2-phenyl-1H-indol-7-amine obtained in Preparation Example 12 and commercially available cyclopentanone were reacted using the method of Example 1 to obtain the title compound.

¹H NMR (400MHz, CDCl₃); δ 8.68 (br s, 1H), 7.72 (d, 2H), 7.43 (t, 2H), 7.30 (t, 1H), 7.01 (s, 1H), 6.75 (s, 1H), 6.47 (s, 1H), 4.60 (s, 2H), 3.85 (m, 1H), 3.69 (m, 6H), 3.57 (m, 2H), 3.47 (m, 1H), 3.34 (s, 3H), 2.07 (m, 2H), 1.75 (m, 2H), 1.70-1.54 (m, 4H).

Mass [M+H] = 408.8 (M+1)

### Example 40: 5-[2-(2-methoxyethoxy)ethoxymethyl]-2-phenyl-N-tetrahydropyran-4-yl-1H-indol-7-amine

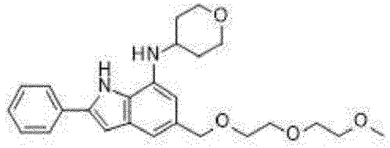

The 5-[2-(2-methoxyethoxy)ethoxymethyl]-2-phenyl-1H-indol-7-amine obtained in Preparation Example 12 and commercially available tetrahydro-4H-pyran-4-one were reacted using the method of Example 37 to obtain the title compound.

¹H NMR (400MHz, CDCl₃); δ 8.47 (br s, 1H), 7.72 (d, 2H), 7.46 (t, 2H), 7.31 (t, 1H), 7.08 (s, 1H), 6.79 (s, 1H), 6.54 (s, 1H), 4.61 (s, 2H), 4.05 (m, 2H), 3.69 (m, 6H), 3.60-3.52 (m, 5H), 3.37 (s, 3H), 2.11 (m, 2H), 1.60 (m, 2H).

Mass [M+H] = 424.8 (M+1)

### Preparation Example 13: N-(3,5-bis(trifluoromethyl)phenyl)-4-oxopiperidine-1-carboxamide

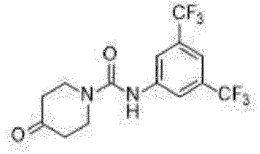

Piperidine-4-one · HCl monohydrate (200 mg, 1.3 mmol) was added to DCM, followed by addition of 1-isocyanato-3,5-bis(trifluoromethyl)benzene (270 ul, 1.56 mmol). K₂CO₃ (539 mg, 3.9 mmol) was added, then stirred for 15 hours. After confirming termination of the reaction, the reaction mixture was filtered. The filtrate was washed with 2N HCl aqueous solution, then dried with MgSO₄ and filtered. The filtrate was concentrated under reduced pressure and azeotropically concentrated with n-Hexane, then n-Hexane was added again and crystallized. The solid was filtered and dried to obtain the title compound as a white solid (70 mg, 15%) which was used as-is in the subsequent reaction.

### Example 41: N-[3,5-bis(trifluoromethyl)phenyl]-4-[[5-(2-methoxyethoxymethyl)-2-phenyl-1H-indol-7-yl]amino]piperidine-1-carboxamide

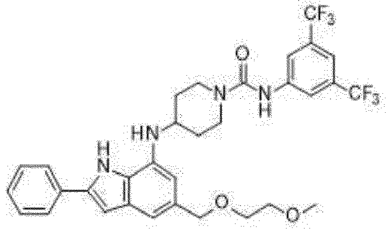

The 5-(2-methoxyethoxymethyl)-2-phenyl-1H-indol-7-amine obtained in Preparation Example 4 (50 mg, 0.135 mmol) was added to DCM, then the N-(3,5-bis(trifluoromethyl)phenyl)-4-oxopiperidine-1-carboxamide (57 mg, 0.162 mmol) obtained in Preparation Example 13 was added. NaBH(OAc)₃ (114 mg, 0.54 mmol) was added, followed by stirring for 15 minutes). After confirming termination of the reaction, 1N NaOH aqueous solution was added, followed by stirring for 1 hour. After separating the layers, the organic layer was dried with MgSO₄ and filtered. The filtrate was concentrated under reduced pressure and purified with silica gel column chromatography (Hex/EA, 1/1) to obtain the title compound (13 mg, 15%) as a white solid.

¹H NMR (400MHz, CDCl₃); δ 9.21 (br s, 1H), 7.86 (s, 2H), 7.62 (d, 2H), 7.45 (s, 1H), 7.36 (t, 2H), 7.27 (t, 1H), 7.01 (m, 2H), 6.70 (s, 1H), 6.44 (s, 1H), 4.64 (s, 2H), 3.95 (m, 2H), 3.74 (m, 3H), 3.63 (m, 2H), 3.45 (m, 1H), 3.32 (s, 3H), 2.94 (m, 2H), 2.04 (m, 2H), 1.33 (m, 2H).

Mass [M+H] = 635.3 (M+1)

### Preparation Example 14: N-(3,5-dichlorophenyl)-4-oxopiperidine-1-carboxamide

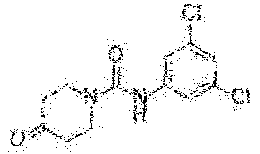

Piperidine-4-one · HCl monohydrate (500 mg, 3.255 mmol) was added to DCM, followed by addition of 1,3-dichloro-5-isocyanatobenzene (795 mg, 4.232 mmol). K₂CO₃ (1.35 g, 9.77 mmol) was added, then stirred for 15 hours. After confirming termination of the reaction, the reaction mixture was filtered. The filtrate was washed with 2N HCl aqueous solution, then dried with MgSO₄ and filtered. The filtrate was concentrated under reduced pressure and azeotropically concentrated with n-Hexane, then n-Hexane was added again and crystallized. The solid was filtered and dried to obtain the title compound as a white solid (217 mg, 23%) which was used as-is in the subsequent reaction.

### Example 42: N-(3,5-dichlorophenyl)-4-[[5-(2-methoxyethoxymethyl)-2-phenyl-1H-indol-7-yl]amino]piperidine-1-carboxamide

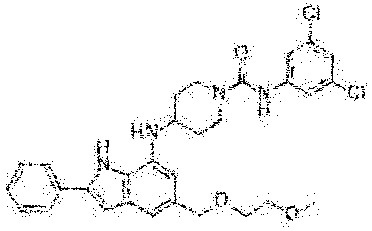

DCM was added to 5-(2-methoxyethoxymethyl)-2-phenyl-1H-indol-7-amine (47 mg, 0.157 mmol) obtained in Preparation Example 4, and then the N-(3,5-dichlorophenyl)-4-oxopiperidine-1-carboxamide (55 mg, 0.190 mmol) obtained in Preparation Example 14 was added. NaBH(OAc)₃ (134 mg, 0.634 mmol) was added, followed by stirring for 15 hours. After confirming termination of the reaction, 1N NaOH aqueous solution was added, and stirred for 1 hour. The layers were purified, and the organic layer was dried with MgSO₄ and filtered. The filtrate was concentrated under reduced pressure and filtered using silica gel column chromatography (Hex/EA, 1/1) to obtain the title compound (20 mg, 22%) as a white solid.

¹H NMR (400MHz, CDCl₃); δ 9.46 (br s, 1H), 7.56 (d, 2H), 7.31 (t, 2H), 7.24 (m, 3H), 6.96 (s, 1H), 6.87 (s, 1H), 6.68 (s, 1H), 6.64 (br, 1H), 6.43 (s, 1H), 4.59 (s, 2H), 3.95 (m, 2H), 3.63-3.56 (m, 4H), 3.52 (m, 1H), 3.31 (s, 3H). 2.95 (m, 2H), 2.06 (m, 2H), 1.35 (m, 2H).

Mass [M] = 566.2 (M)

### Example 43: 5-(2-methoxyethoxymethyl)-2-phenyl-N-(2-thienylmethyl)-1H-indol-7-amine

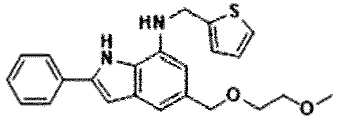

The 5-(2-methoxyethoxymethyl)-2-phenyl-1H-indol-7-amine obtained in Preparation Example 4 and commercially available thiophene-2-carbaldehyde were reacted using the method of Example 1 to obtain the title compound.

¹H NMR (400MHz, CDCl₃); δ 8.45 (br s, 1H), 7.67 (d, 2H), 7.44 (t, 2H), 7.31 (m, 2H), 7.13 (m, 2H), 7.03 (m, 1H), 6.80 (s, 1H), 6.57 (s, 1H), 4.62 (s, 2H), 4.57 (s, 2H), 3.66-3.55 (m, 4H), 3.40 (s, 3H).

Mass [M+H] = 393.2 (M+1)

### Example 44: Ethyl 4-[[5-(2-methoxyethoxymethyl)-2-phenyl-1H-indol-7-yl]amino]piperidine-1-carboxylate

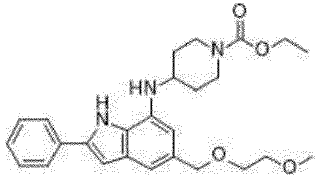

The 5-(2-methoxyethoxymethyl)-2-phenyl-1H-indol-7-amine obtained in Preparation Example 4 and commercially available ethyl 4-oxopiperidine-1-carboxylate were reacted using the method of Example 1 to obtain the title compound.

¹H NMR (400MHz, CDCl₃); δ 10.09 (br s, 1H), 7.76 (d, 2H), 7.43 (t, 2H), 7.28 (t, 1H), 7.02 (s, 1H), 6.79 (s, 1H), 6.53 (s, 1H), 4.65 (s, 2H), 4.39-4.10 (br m, 2H), 4.17 (qt, 2H), 3.79 (m, 1H), 3.65-3.57 (m, 4H), 3.66-3.55 (m, 4H), 3.41 (s, 3H), 3.03 (m, 2H), 2.42-2.10 (br m, 2H), 1.45 (br,2H), 1.21 (t, 3H).

Mass [M+H] = 452.3 (M+1)

### Example 45: 5-(2-methoxyethoxymethyl)-2-phenyl-N-(4-pyridylmethyl)-1H-indol-7-amine

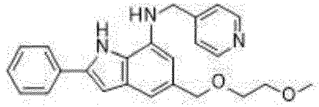

The 5-(2-methoxyethoxymethyl)-2-phenyl-1H-indol-7-amine obtained in Preparation Example 4 and commercially available iso-nicotinic aldehyde were reacted using the method of Example 1 to obtain the title compound.

¹H NMR (400MHz, DMSO-d₆); δ 11.05 (br s, 1H), 8.52 (d, 2H), 7.81 (d, 2H), 7.47 (t, 2H), 7.44 (d, 2H), 7.32 (t, 1H), 6.81 (m, 2H), 6.13 (s, 2H), 4.53 (d, 2H), 4.36 (s, 2H), 3.45-3.35 (m, 4H), 3.20 (s, 3H).

Mass [M+H] = 388.2 (M+1)

### Preparation Example 15: 2-methoxy-N-[5-(2-methoxyethoxymethyl)-2-phenyl-1H-indol-7-yl]acetamide {2-methoxy-N-[5-(2-methoxyethoxymethyl)-2-phenyl-1H-indol-7-yl] acetamide}

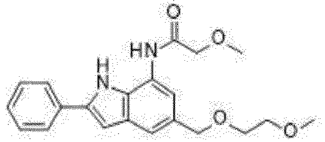

The 5-(2-methoxyethoxymethyl)-2-phenyl-1H-indol-7-amine obtained in Preparation Example 4 (100 mg, 0.337 mmol) was added to Acetonitrile (1.5 mL), then DIPEA (176 uL, 1.011 mmol) was added. HATU (192 mg, 0.506 mmol) and 2-methoxyacetic acid (28 uL, 0.371 mmol) were added to the reaction mixture, then stirred for 15 hours at room temperature. The reaction mixture was concentrated under reduced pressure, and the concentration residue was purified by silica gel column chromatography (Hex/EA, 1/2) to obtain the title compound as a light brown solid (130 mg, quantitative yield).

Mass [M] = 369.2 (M+1)

### Example 46: 5-(2-methoxyethoxymethyl)-N-(2-methoxyethyl)-2-phenyl-1H-indol-7-amine

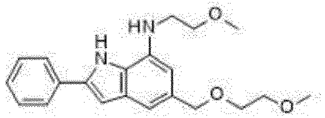

The 2-methoxy-N-[5-(2-methoxyethoxymethyl)-2-phenyl-1H-indol-7-yl]acetamide obtained in Preparation Example 15 (124 mg, 0.337 mmol) was dissolved in THF (1.2 mL). The mixture was cooled to 0°C, and BH₃ · SMe₂ 2M in THF (0.5 mL, 1.011 mmol) was added dropwise. The mixture was warmed up to room temperature, and stirred for 17 hours. After confirming that the reaction was terminated, the mixture was cooled to 0°C, and a 1N NaOH aqueous solution was slowly added dropwise. The mixture was warmed to room temperature, stirred for 30 minutes, then extracted with EA. The organic layer was dried with MgSO₄ and filtered. The filtrate was concentrated under reduced pressure, and the concentration residue was purified by silica gel column chromatography (Hex/EA, 1/1) to obtain the title compound (96 mg, 80%) as a light brown solid.

¹H NMR (400MHz, CDCl₃); δ 9.29 (br s, 1H), 7.68 (d, 2H), 7.43 (t, 2H), 7.30 (t, 1H), 7.12 (s, 1H), 6.77 (s, 1H), 6.58 (s, 1H), 4.64 (s, 2H), 3.76 (m, 2H), 3.65-3.55 (m, 4H), 3.52 (m, 2H), 3.50 (s, 3H). 3.41 (s, 3H).

Mass [M + H] = 355.2 (M +1)

### Preparation Example 16: 3-methoxy-N-[5-(2-methoxyethoxymethyl)-2-phenyl-1H-indol-7-yl]propanamide

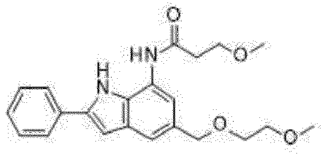

The 5-(2-methoxyethoxymethyl)-2-phenyl-1H-indol-7-amine obtained in Preparation Example 4 (100 mg, 0.337 mmol) was added to Acetonitrile (1.5 mL), then DIPEA (176 uL, 1.011 mmol) was added. HATU (192 mg, 0.506 mmol) and 3-methoxypropanoic acid (35 uL, 0.371 mmol) were added to the reaction mixture, then stirred for 15 hours at room temperature. The reaction mixture was concentrated under reduced pressure, and the concentration residue was purified by silica gel column chromatography (Hex/EA, 1/2) to obtain the title compound as a light brown solid (135 mg, quantitative yield).

Mass [M] = 383.2 (M+1)

### Example 47: 5-(2-methoxyethoxymethyl)-N-(3-methoxypropyl)-2-phenyl-1H-indol-7-amine

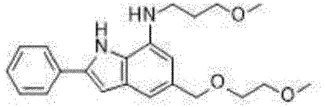

The 3-methoxy-N-[5-(2-methoxyethoxymethyl)-2-phenyl-1H-indol-7-yl]propamide (128 mg, 0.337 mmol) obtained in Preparation Example 16 was dissolved in THF (1.2 mL). The mixture was cooled to 0°C, and BH₃ · SMe₂ 2M in THF (0.5 mL, 1.011 mmol) was added dropwise. The mixture was warmed up to room temperature, and stirred for 17 hours. After confirming that the reaction was terminated, the mixture was cooled to 0°C, and a 1N NaOH aqueous solution was slowly added dropwise. The mixture was warmed to room temperature, stirred for 30 minutes, then extracted with EA. The organic layer was dried with MgSO₄ and filtered. The filtrate was concentrated under reduced pressure, and the concentration residue was purified by silica gel column chromatography (Hex/EA, 1/1) to obtain the title compound (55 mg, 44%) as a light brown solid.

¹H NMR (400MHz, CDCl₃); δ 8.91 (br s, 1H), 7.73 (d, 2H), 7.45 (t, 2H), 7.35 (t, 1H), 7.11 (s, 1H), 6.77 (s, 1H), 6.56 (s, 1H), 4.64 (s, 2H), 3.69 (t, 2H), 3.63-3.58 (m, 4H), 3.41 (s, 6H). 3.39 (m, 2H), 1.99 (m, 2H).

Mass [M + H] = 369.2 (M +1)

### Preparation Example 17: Methyl 2-(3-fluorophenyl)-7-nitro-1H-indole-5-carboxylate

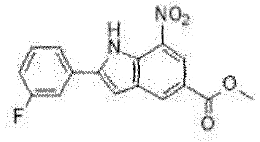

Methyl 4-amino-3-iodo-5-nitrobenzene (1.03 g, 3.105 mmol) was dissolved in Dioxane (100 mL), then TEA (1.3 mL, 9.315 mmol), 3-fluorophenylacetilene (0.4 mL, 3.726 mmol), CuI (6 mg, 0.031 mmol), PdCh (PPh₃)₂ (22 mg, 0.031 mmol) were added in the same order. The mixture was heated to 60°C, then stirred for 3 hours. Generation of an intermediate was confirmed with TLC, then DBU (2.6 mL, 18.63 mmol) was added dropwise before heating to 110°C and stirring for 15 hours. After confirming termination of the reaction, the reaction mixture was cooled to room temperature. After extracting with DCM and H₂O, the organic layer was dried with MgSO₄ and filtered. The filtrate was concentrated under reduced pressure and purified with silica gel column chromatography to obtain the title compound (653 mg, 58%) as a yellow solid.

Mass [M+H] = 315.3 (M+1)

### Preparation Example 18: 2-(3-fluorophenyl)-7-nitro-1H-indole-5-carboxylic acid

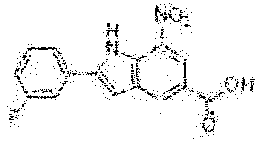

The methyl 2-(3-fluorophenyl)-7-nitro-1H-indole-5-carboxylate (570mg, 1.813 mmol) obtained in Preparation Example 17 was added to THF/MeOH/H₂O (11 mL/5.7 mL/5.7 mL), then the mixture was cooled to 0°C. LiOH · H₂O (152 mg, 3.627 mmol) was added, then the mixture was warmed up to room temperature and stirred for 15 hours. The mixture was then cooled to 0°C, neutralized with a 2N HCl aqueous solution, and additionally stirred for 1 hour. The generated solid was filtered and washed with water. The filtered solid was vacuum dried and the title compound (440 mg, 80%) as a yellow solid.

Mass [M+H] = 301.3 (M+1)

### Preparation Example 19: (2-(3-fluorophenyl)-7-nitro-1H-indol-5-yl)methanol

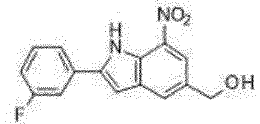

The 2-(3-fluorophenyl)-7-nitro-1H-indole-5-carboxylic acid (440 mg, 1.465 mmol) obtained in Preparation Example 18 was dissolved in THF (4.4 mL). The mixture was cooled to 0°C, and BH₃ · SMe₂ 2M in THF (2.2 mL, 4.396 mmol) was added dropwise. The mixture was warmed up to room temperature, and stirred for 15 hours. After confirming that the reaction was terminated, the mixture was cooled to 0°C, and a 1N NaOH aqueous solution was slowly added dropwise. The mixture was warmed to room temperature, stirred for 1 hour, then extracted with EA. The organic layer was washed with brine, then dried with MgSO₄ and filtered. The filtrate was concentrated under reduced pressure, and the concentration residue was purified by silica gel column chromatography to obtain the title compound (285 mg, 68%) as a yellow solid.

Mass [M+H] = 287.2 (M+1)

### Preparation Example 20: 5-(bromomethyl)-2-(3-fluorophenyl)-7-nitro-1H-indole

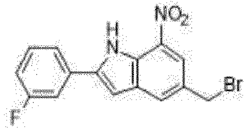

The (2-(3-fluorophenyl)-7-nitro-1H-indol-5-yl)methanol (285 mg, 0.995 mmol) obtained in Preparation Example 19 was dissolved in THF (3 mL). The mixture was cooled to 0°C, and PBr₃ (0.06 mL, 0.597 mmol) was slowly added dropwise. The mixture was warmed up to room temperature, and stirred for 2 hours. After confirming that the reaction was terminated, the reaction mixture was poured over ice, then extracted 2 times with EA. The organic layer was collected, then dried with MgSO₄ and filtered. The filtrate was concentrated under reduced pressure, and diethyl ether was added to the concentration residue to crystallize and filter to obtain the title compound (287mg, 83%) as a yellow solid.

Mass [M+H] = 350.2 (M+1)

### Preparation Example 21: 2-(3-fluorophenyl)-5-(2-methoxyethoxymethyl)-7-nitro-1H-indole

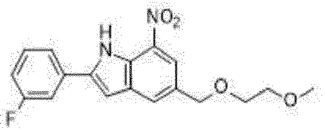

The 5-(bromomethyl)-2-(3-fluorophenyl)-7-nitro-1H-indole obtained in Preparation Example 20 (287 mg, 0.822 mmol) was dissolved in 2-methoexyethanol (6 mL). K₂CO₃ (163 mg, 1.178 mmol) was added, and the mixture was stirred for 1 hour and 30 minutes at room temperature. After confirming termination of the reaction, EA and H₂O were added to extract. The organic layer was washed with brine, and the organic layer was dried with MgSO₄ and filtered. The filtrate was concentrated under reduced pressure, and the concentration residue was purified by silica gel column chromatography to obtain the title compound (47 mg, 16%) as a yellow solid.

Mass [M+H] = 345.3 (M+1)

### Preparation Example 22: 2-(3-fluorophenyl)-5-(2-methoxyethoxymethyl)-1H-indol-7-amine

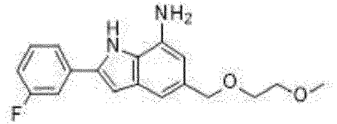

The 2-(3-fluorophenyl)-5-(2-methoxyethoxymethyl)-7-nitro-1H-indole obtained in Preparation Example 21 (43 mg, 0.125 mmol) was added to THF/MeOH/H₂O (0.86 mL/0.86 mL/0.86 mL), then Fe powder (69 mg, 1.249 mmol) and NH₄Cl (33 mg, 0.625 mmol) were added. The mixture was heated to 60°C and stirred for 2 hours, then termination of the reaction was confirmed. The mixture was cooled to room temperature and filtered through a celite pad, and the filtrate was concentrated. The concentration residue was extracted with EA and H₂O, and the organic layer was dried with MgSO₄, then filtered. The filtrate was concentrated under reduced pressure, then purified by silica gel column chromatography to obtain the title compound as a brown liquid (39 mg, 90%).

Mass [M+H] = 315.3 (M+1)

### Example 48: 2-(3-fluorophenyl)-5-(2-methoxyethoxymethyl)-N-tetrahydropyran-4-yl-1H-indol-7-amine

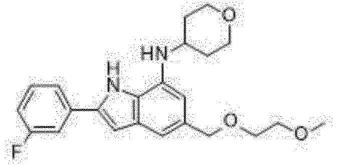

The 2-(3-fluorophenyl)-5-(2-methoxyethoxymethyl)-1H-indol-7-amine obtained in Preparation Example 22 (39 mg, 0.124 mmol) was added to DCM (2 mL), then tetrahydro-4H-pyran-4-one (11 µL, 0.372 mmol) and NaBH(OAc)₃ (79 mg, 0.372 mmol). After stirring for 15 hours at room temperature, termination of the reaction was confirmed. 1N NaOH aqueous solution was added, followed by stirring for 30 minutes. The layers were purified, and the organic layer was dried with MgSO₄ and filtered. The filtrate was concentrated under reduced pressure and purified by silica gel column chromatography to obtain the title compound as a light brown solid (45 mg, 91%).

¹H NMR (400MHz, CDCl₃); δ 8.53 (br s, 1H), 7.46 (d, 1H), 7.37 (m, 2H), 7.35 (t, 1H), 7.04 (s, 1H), 6.97 (t, 1H), 6.75 (s, 1H), 6.55 (s, 1H), 4.58 (s, 2H), 4.01 (m, 2H), 3.60-3.50 (m, 7H), 3.37 (s, 3H). 2.10 (m, 2H), 1.56 (m, 2H).

Mass [M+H] = 399.2 (M+1)

### Preparation Example 23: Methyl 4-amino-3-nitro-5-(2-trimethylsilylethynyl)benzoate

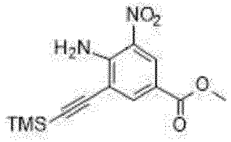

Methyl 4-amino-3-iodo-5-nitro-benzoate (6.56 g, 20.37 mmol, 1 eq.) was dissolved in MeCN (70 mL), then ethinyl(trimethyl)silane (2.40 g, 24.44 mmol, 1.2 eq). and TEA (6.18 g, 61.11 mmol, 3 eq.) were added. After stirring for 30 minutes in a nitrogen atmosphere at 25°C, Pd(dppf)C12 (149.04 mg, 203.69 mol, 0.01 eq.) and CuI (38.79 mg, 203.69 mol, 0.01 eq.) were added, then the mixture was heated to 60°C and stirred for 3 hours to obtain a brown suspension. After confirming termination of the reaction with LCMS, the reaction mixture was added to ice water (100 mL) and filtered. EtOAc (200 mL) was added to the filtrate to extract. The organic layer was washed with water (150 mL) and brine (150 mL), then dried with MgSO₄ and filtered. The filtrate was concentrated under reduced pressure and purified by silica gel column chromatography (EtOAc in PE = 0-10%) to obtain the title compound as a yellow solid (4.46g, 74.67%).

¹H NMR (400MHz, CDCl₃); δ 8.81 (s, 1H), 8.18 (s, 1H), 3.90 (s, 3H), 0.30 (s, 9H).

### Preparation Example 24: methyl 7-nitro-1H-indole-5-carboxylate

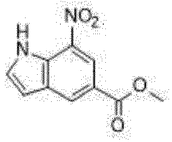

The methyl 4-amino-3-nitro-5-(2-trimethylsilylethynyl)benzoate (4.46 g, 15.26 mmol,. 1 eq) obtained in Preparation Example 23 was added to THF (45 mL), then TBAF (1 M, 30.51 mL, 2 eq.) was added at 0°C, then heated to 65°C for 30 minutes to obtain a brown solution. After confirming termination of the reaction with TLC (PE/EtOAc = 9/1), concentration was carried out under vacuum. The concentration residue was treated with H₂O (500 mL) and EtOAc (500 mL × 2), and the organic layer was again washed with H₂O (800 mL) and brine (500 mL) followed by drying with Na₂SO₄ and filtering. The filtrate was concentrated under reduced pressure and purified by silica gel column chromatography to obtain the title compound as a brown solid (3.46 g, crude). This was used in the next reaction without additional purification.

¹H NMR (400MHz, DMSO-d6); δ 12.27 (s, 1H), 8.67 (s, 1H), 8.57 (s, 1H), 7.65 (t, 1H), 6.92 (t, 1H), 3.92 (s, 3H).

### Preparation Example 25: (7-nitro-1H-indol-5-yl)methanol

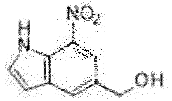

The methyl 7-nitro-1H-indole-5-carboxylate (3.16 g, 14.35 mmol, 1 eq.) was dissolved in THF (30 mL), then 1M DIBAL-H in toluene (43.06 mL, 3 eq.) was added at 0°C. After stirring for 30 minutes at the same temperature, termination of the reaction was confirmed with TLC (PE/EtOAc = 2/1). H₂O (100 mL) was added to terminate the reaction, then the reaction mixture was filtered. The filtrate was extracted with EtOAc (150mL × 2), and the organic layer was washed with H₂O (200 mL) and brine (150mL), then dried with Na₂SO₄ and filtered. The filtrate was concentrated under reduced pressure and purified by silica gel column chromatography PE/EtOAc = 0-25%) to obtain the title compound (1.79 g, 64.90% yield) as a yellow solid.

¹H NMR (400MHz, DMSO-d₆); δ 11.82 (br s, 1H), 8.09 (s, 1H), 8.00 (s, 1H), 7.51 (t, 1H), 6.70 (t, 1H), 5.37 (t, 1H), 4.65 (d, 2H).

### Preparation Example 26: 5-(bromomethyl)-7-nitro-1H-indole

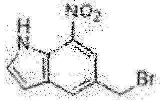

The (7-nitro-1H-indol-5-yl)methanol obtained in Preparation Example 25 (1.98 g, 10.30 mmol, 1 eq.) was dissolved in DCM (20 mL), then PBr₃ (5.58 g, 20.61 mmol, 2 eq.) was added at 0°C. The reaction mixture was stirred for 3 hours at 15°C to obtain a brown suspension. Termination of the reaction was confirmed with TLC (PE/EtOAc = 2/1). H₂O (5 mL) was added to terminate the reaction. After extraction with DCM (25 mL), the organic layer was washed with H₂O (20 mL) and brine (15 mL) then dried with Na₂SO₄ and filtered. The filtrate was concentrated under reduced pressure, and purified by silica gel flash column chromatography (PE/EtOAc = 0-15%) to obtain the title compound (1.56 g, 59.36% yield) as a yellow solid.

¹H NMR (400MHz, DMSO-d₆); δ 11.98 (br s, 1H), 8.22 (s, 1H), 8.17 (s, 1H), 7.57 (s, 1H), 6.75 (s, 1H), 4.96 (s, 2H).

### Preparation Example 27: 5-(2-methoxyethoxymethyl)-7-nitro-1H-indole

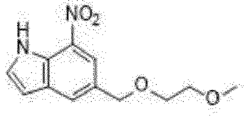

To 2-methoxyethanol (46.54 g, 611.20 mmol 100 eq), K₂CO₃ (5.58 g, 20.61 mmol, 2 eq.) was added at 15°C, then a THF solution (5 mL) of the 5-(bromomethyl)-7-nitro-1H-indole (1.56 g, 6.12 mmol, 1 eq.) obtained in Preparation Example 26 was added at the same temperature. The reaction mixture was stirred for 3 hours at 15°C to obtain a brown suspension. After confirming termination of the reaction with TLC (PE/EtOAc = 10/1), H₂O (10 mL) and EtOAc (30mL) were added and work-up was carried out. The extracted organic layer was washed with H₂O (25 mL) and brine (25 mL), then dried with Na₂SO₄ and filtered. The filtrate was concentrated under reduced pressure and purified by silica gel flash column chromatography PE/EtOAc = 0-25%) to obtain the title compound (610.6 mg, 39.9% yield) as a yellow solid.

¹H NMR (400MHz, DMSO-d₆); δ 11.88 (br s, 1H), 8.07 (s, 1H), 8.02 (s, 1H), 7.53 (m, 1H), 6.72 (m, 1H), 4.64 (s, 2H), 3.61-3.49 (m, 4H), 3.32 (s, 3H).

### Preparation Example 28: 3-bromo-5-(2-methoxyethoxymethyl)-7-nitro-1H-indole

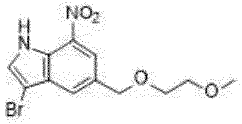

The 5-(2-methoxyethoxymethyl)-7-nitro-1H-indole (610 mg, 2.44 mmol, 1 eq.) obtained in Preparation Example 27 was dissolved in DMF (12 mL), then NBS (433.85 mg, 2.44 mmol, 2 eq.) was added at 0°C. The reaction mixture was stirred for 1 hour at 15°C to obtain a yellow solution. After confirming termination of the reaction using TLC (PE/EtOAc = 2/1), H₂O (30 mL) was added and the reaction mixture was filtered. To the obtained filter cake, THF (20 mL) was added, and the organic layer was dried with Na₂SO₄ and filtered. The filtrate was concentrated under reduced pressure, and the title compound was obtained as a yellow solid (798.2 mg, 94.02% yield), then used in the subsequent reaction without additional purification.

¹H NMR (400MHz, DMSO-d₆); δ 12.20 (br s, 1H), 8.16 (s, 1H), 7.89 (s, 1H), 7.73 (d, 1H), 4.69 (s, 2H), 3.63-3.51 (m, 4H), 3.27 (s, 3H).

### Preparation Example 29: 5-(2-methoxyethoxymethyl)-3-methyl-7-nitro-1H-indole

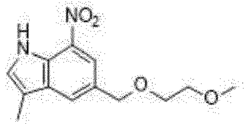

2,4,6-trimethyl-1,3,5,2,4,6-trioxatriborinane (3.43 g, 13.67 mmol, 50% purity, 15 eq.), the 3-bromo-5-(2-methoxyethoxymethyl)-7-nitro-1H-indole obtained in Preparation Example 28 (300 mg, 911.45 mol, 1 eq.), K₂CO₃ (377.90 mg, 2.73 mmol, 3 eq.) and Pd(PPh₃)₄ (105.32 mg, 91.14 mol, 0.1 eq.) were placed in a microwave tube, then THF (3 mL) was added. The sealed tube was reacted for 6 hours at 80°C in a microwave reactor (2 bar) to obtain a black suspension. TLC (PE/EtOAc = 2/1) was used to confirm termination of the reaction. EtOAc (5 mL) was added to the suspension, which was then filtered. EtOAc (5 mL) and H₂O (3 mL) was added to the filtrate, and the organic layer was washed with H₂O (10mL) and brine (5 mL), then dried with Na₂SO₄ and filtered. The filtrate was concentrated under reduced pressure, then purified by silica gel flash column chromatography (PE/EtOAc = 0-15%) to obtain the title compound (94.3 mg, 39.15% yield) as a yellow solid.

¹H NMR (400MHz, DMSO-d₆); δ 11.59 (br s, 1H), 8.06 (s, 1H), 7.97 (s, 1H), 7.31 (s, 1H), 4.66 (s, 2H), 3.60-3.49 (m, 4H), 3.26 (s, 3H), 2.31 (s, 3H).

### Preparation Example 30: 5-(2-methoxyethoxymethyl)-3-methyl-1H-indol-7-amine

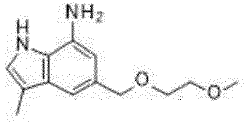

To the 5-(2-methoxyethoxymethyl)-3-methyl-7-nitro-1H-indole obtained in Preparation Example 29 (154 mg, 582.72 µmol, 1 eq.), THF (1.2 mL), H₂O (1 mL) and EtOH (4 mL) were added, followed by addition of Fe powder (390.51 mg, 6.99 mmol, 12 eq.) and NH₄Cl (623.41 mg, 11.65 mmol, 20 eq.) at 15°C, heating to 80°C and stirring for 3 hours. After confirming termination of the reaction with LCMS, the reaction mixture was filtered using celite. EtOAc (10 mL) and H₂O (20 mL) were added to the filtrate before extracting. The organic layer was washed with H₂O (20 mL) and brine (10 mL), then dried with Na₂SO₄ and filtered. The filtrate was concentrated under reduced pressure and purified with silica gel flash column chromatography (PE/EtOAc = 0-60%) to obtain the title compound (100 mg) as a yellow solid.

¹H NMR (400MHz, DMSO-d₆); δ 10.24 (br s, 1H), 6.99 (s, 1H), 6.67 (s, 1H), 6.30 (s, 1H), 4.40 (s, 2H), 3.49-3.45 (m, 4H), 3.24 (s, 3H), 2.18 (s, 3H).

### Example 49: N-cyclopentyl-5-(2-methoxyethoxymethyl)-3-methyl-1H-indol-7-amine

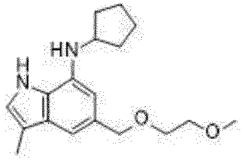

The 5-(2-methoxyethoxymethyl)-3-methyl-1H-indol-7-amine obtained in Preparation Example 30 and commercially available cyclopentanone were reacted using the method of Example 1 to obtain the title compound.

¹H NMR (400MHz, DMSO-d₆); δ 10.33 (br s, 1H), 6.98 (s, 1H), 6.68 (s, 1H), 6.20 (s, 1H), 5.15 (d, 1H), 4.44 (s, 2H), 3.85 (m, 1H), 3.49-3.43 (m, 4H), 3.24 (s, 3H), 2.18 (s, 3H), 1.97 (m, 2H), 1.71 (m, 2H), 1.61-1.50 (m, 4H).

Mass [M+H] = 303.2 (M+1)

### Example 50: 5-(2-methoxyethoxymethyl)-3-methyl-N-tetrahydropyran-4-yl-1H-indol-7-amine

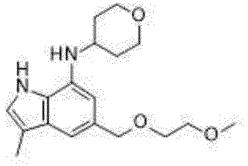

The 5-(2-methoxyethoxymethyl)-3-methyl-1H-indol-7-amine obtained in Preparation Example 30 and commercially available tetrahydro-4H-pyran-4-one were reacted using the method of Example 1 to obtain the title compound.

¹H NMR (400MHz, DMSO-d₆); δ 10.35 (br s, 1H), 7.00 (s, 1H), 6.70 (s, 1H), 6.27 (s, 1H), 5.08 (d, 1H), 4.45 (s, 2H), 3.89 (m, 2H), 3.65 (m, 1H), 3.49-3.45 (m, 6H), 3.24 (s, 3H), 2. 18 (s, 3H), 1.99 (m, 2H), 1.43 (m, 2H).

Mass [M+H] = 319.2 (M+1)

### Preparation Example 31: 5-(2-methoxyethoxymethyl)-7-nitro-3-phenyl-1H-indole

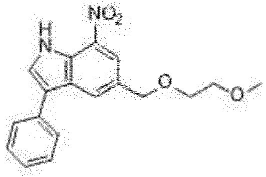

The 3-bromo-5-(2-methoxyethoxymethyl)-7-nitro-1H-indole obtained in Preparation Example 28 (300 mg, 911.45 µmol, 1 eq.) was dissolved in dioxane (3 mL) /H₂O (0.6 mL), then K₂CO₃ (377.90 mg, 2.73 mmol, 3 eq.), PhB(OH)₂ (222.26 mg, 1.82 mmol, 2 eq.) and Pd(dppf)Cl₂ (6.67 mg, 9.11 µmol, 0.01 eq.) were added followed by stirring for 2 hours at 100°C and confirmation of termination of the reaction using LCMS. H₂O (15 mL) and EtOAc (20mL) were added to the reaction mixture before extracting. The obtained organic layer was washed with H₂O (20 mL) and brine (15 mL), then dried with Na₂SO₄ and filtered. The filtrate was concentrated under reduced pressure, then purified by silica gel flash column chromatography (PE/EtOAc = 0-20%) to obtain the title compound (286.7 mg, 96.39% yield) as a yellow solid.

¹H NMR (400MHz, DMSO-d₆); δ 12.10 (br s, 1H), 8.26 (s, 1H), 8.14 (s, 1H), 7.80 (s, 1H), 7.69 (d, 2H), 7.48 (t, 2H), 7.33 (t, 1H), 4.70 (s, 2H), 3.62-3.49 (m, 4H), 3.25 (s, 3H).

### Preparation Example 32: 5-(2-methoxyethoxymethyl)-3-phenyl-1H-indol-7-amine

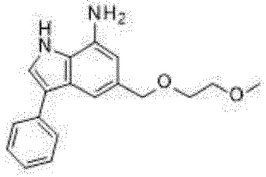

To the 5-(2-methoxyethoxymethyl)-7-nitro-3-phenyl-1H-indole obtained in Preparation Example 31 (286.7 mg, 878.52 µmol, 1 eq.), THF (1.5 mL), H₂O (1 mL) and EtOH (5 mL) were added, followed by addition of Fe powder (588.73 mg, 10.54 mmol, 12 eq.) and NH₄Cl (939.86 mg, 17.57 mmol, 20 eq.) and heating to 80°C for stirring for 3 hours. After confirming termination of the reaction with LCMS, filtration was performed using celite. EtOAC (10 mL) and H₂O (20 mL) were added to the filtrate before extracting. The organic layer was washed with H₂O (20 mL) and brine (10 mL), then dried with Na₂SO₄ and filtered. The filtrate was concentrated under reduced pressure and purified by silica gel flash column chromatography (PE/EtOAc = 0-60%) to obtain the title compound (216.2 mg, 97.2%) as a yellow solid.

¹H NMR (400MHz, DMSO-d₆); δ 10.90 (br s, 1H), 7.65 (d, 2H), 7.59 (s, 1H), 7.41 (t, 2H), 7.20 (t, 1H), 7.08 (s, 1H), 6.40 (s, 1H), 5.14 (br s, 2H), 4.44 (s, 2H), 3.52-3.45 (m, 4H), 3.24 (s, 3H).

### Example 51: N-cyclopentyl-5-(2-methoxyethoxymethyl)-3-phenyl-1H-indol-7-amine

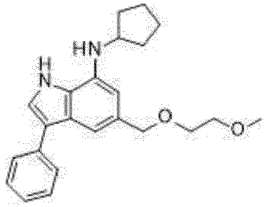

The 5-(2-methoxyethoxymethyl)-3-phenyl-1H-indol-7-amine obtained in Preparation Example 32 and commercially available cyclopentanone were reacted using the method of Example 1 to obtain the title compound.

¹H NMR (400MHz, DMSO-d₆); δ 11.37 (br s, 1H), 7.68 (br, 1H), 7.65 (d, 2H), 7.45 (t, 2 H), 7.30 (s, 1H), 7.25 (t, 1H), 7.17 (s, 1H), 7.04 (s, 1H), 4.54 (s, 2H), 3.98 (m, 1H), 3.54-3.48 (m, 4H), 3.25 (s, 3H), 1.97 (m, 2H), 1.77 (m, 2H), 1.67-1.59 (m, 4H).

Mass [M+H] = 365.4 (M+1)

### Example 52: 5-(2-methoxyethoxymethyl)-3-phenyl-N-tetrahydropyran-4-yl-1H-indol-7-amine

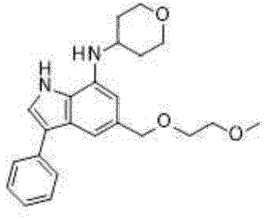

The 5-(2-methoxyethoxymethyl)-3-phenyl-1H-indol-7-amine obtained in Preparation Example 32 and commercially available tetrahydro-4H-pyran-4-one were reacted using the method of Example 1 to obtain the title compound.

¹H NMR (400MHz, DMSO-d₆); δ 11.49 (br s, 1H), 7.67 (s, 1H), 7.66 (d, 2H), 7.51 (s, 1 H), 7.43 (t, 2H), 7.25 (t, 1H), 6.88 (s, 1H), 4.58 (s, 2H), 3.93 (m, 2H), 3.77 (m, 1H), 3.61-3.48 (m, 4H), 3.41 (m, 2H), 3.27 (s, 3H), 1.97 (m, 2H), 1.77 (m, 2H).

Mass [M+H] = 381.3 (M+1)

### Example 53: 5-((2-methoxyethoxy)methyl)-1-methyl-2-phenyl-N-(tetrahydro-2H-pyran-4-yl)-1H-indol-7-amine

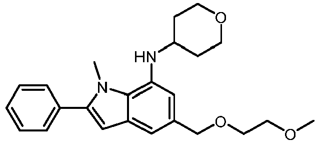

5-((2-methoxyethoxy)methyl)-1-methyl-2-phenyl-indol-7-amine obtained in Preparation Example 6 and commercially available tetrahydro-4H-pyran-4-one were reacted in the same method as in Example 1 to obtain the desired title compound.

¹H NMR (400MHz, CDCl₃); δ 7.51-7.46 (m, 4H), 7.41 (m, 1H), 7.07 (s, 1H), 6.59 (s, 1 H), 6.48 (s, 1H), 4.61 (s, 2H), 4.01 (m, 2H), 3.96 (s, 3H) 3.61-3.54 (m, 7H), 3.39 (s, 3H), 2.14-2.11 (m, 2H), 1.59 (m, 2H).

ESI-MS (*m*/*z*): 394.5 [M+H]⁺

### Preparation Example 33: 2-(4-bromophenyl)-5-((2-methoxyethoxy)methyl)-7-nitro-1H-indole

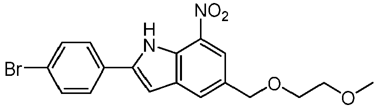

From 2-(4-bromophenyl)-7-nitro-1H-indole-5-carboxylic acid obtained by the known method of International Patent Publication No. WO2009-025478 using 4-bromophenylacetylene, the desired title compound was obtained using the same methods as in Preparation Examples 1, 2 and 3.

¹H NMR (400MHz, DMSO-d₆); δ 11.72 (s, 1H), 8.07 (s, 1H), 8.01 (s, 1H), 7.98 (d, 2H), 7.68 (d, 2H), 7.20 (s, 1 H), 4.66 (s, 2H), 3.61 (m, 2H), 3.51 (m, 2H), 3.27 (s, 3H).

### Preparation Example 34: 2-(4-bromophenyl)-5-((2-methoxyethoxy)methyl)-1H-indol-7-amine

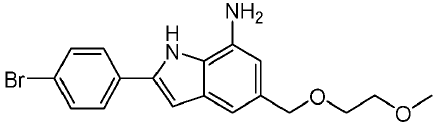

The compound obtained in Preparation Example 33 was used immediately in the next reaction without further purification using the same method as in Preparation Example 4.

### Example 54: 2-(4-bromophenyl)-N-cyclopentyl-5-((2-methoxyethoxy)methyl)-1H-indol-7-amine

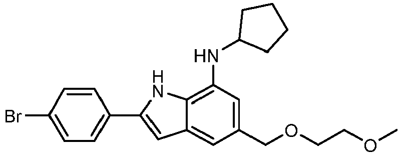

2-(4-bromophenyl)-5-((2-methoxyethoxy)methyl)-1H-indol-7-amine obtained in Preparation Example 34 and commercially available cyclopentanone (1 equivalent) were reacted in the same method as in Example 1 to obtain the title compound.

¹H NMR (400 MHz, DMSO-d₆); δ 10.94 (s, 1 H), 7.75 (d, *J* = 8.8 Hz, 2 H), 7.66 (d, *J* = 8.4 Hz, 2 H), 6.81 (d, *J* = 2.0 Hz, 1 H), 6.75 (s, 1 H), 6.24 (s, 1 H), 5.39 (d, *J* = 6.0 Hz, 1 H), 4.44 (s, 2 H), 3.93 - 3.83 (m, 1 H), 3.53 - 3.43 (m, 4 H), 3.25 (s, 3 H), 2.10 - 1.98 (m, 2 H), 1.81 - 1.71 (m, 2 H), 1.66 - 1.51 (m, 4 H).

LCMS: 96.60%, MS (ESI): m/z 445.1 [M+H]⁺

### Example 55: 2-(4-bromophenyl)-5-((2-methoxyethoxy)methyl)-N-(tetrahydro-2H-pyran-4-yl)-1H-indol-7-amine

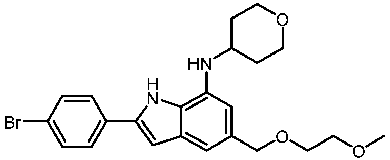

5-((2-methoxyethoxy)methyl)-2-phenyl-1H-indol-7-amine obtained in Preparation Example 34 and commercially available tetrahydro-4H-pyran-4-one (1 equivalent) was reacted in the same method as in Example 1 to obtain the title compound.

¹H NMR (400 MHz, DMSO-d₆); δ 11.02 (s, 1 H), 7.83 - 7.77 (m, 2 H), 7.74 - 7.67 (m, 2 H), 6.87 (d, *J* = 2 Hz, 1 H), 6.82 (s, 1 H), 6.36 (s, 1 H), 5.38 (d, *J* = 7.6 Hz, 1 H), 4.48 (s, 2 H), 4.03 - 3.94 (m, 2 H), 3.72 - 3.64 (m, 1 H), 3.58 - 3.53 (m, 4 H), 3.51 (m, 2 H), 3.29 (s, 3 H), 2.15 - 2.06 (m, 2 H), 1.57 - 1.45 (m, 2 H).

LCMS: 93.05%, MS (ESI): m/z 461.1 [M+H]⁺

### Preparation Example 35: (4-(5-((2-methoxyethoxy)methyl))-7-nitro-1H-indol-2-yl)phenyl)dimethylphosphine oxide

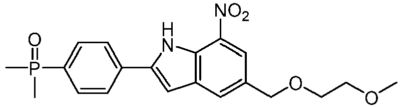

Methylphosphonylmethane (138.67 mg, 1.78 mmol, 1.2 eq.) was dissolved in DMF (10 mL), and then the compound (600 mg, 1.48 mmol, 1 eq.) of Preparation Example 33, Pd(OAc)₂ (26.59 mg, 118.45 µmol, 0.08 eq.), K₃PO₄ (345.71 mg, 1.63 mmol, 1.1 eq.) and Xantphos (85.67 mg, 148.06 µmol, 0.1 eq.) were sequentially added thereto under nitrogen. The reaction mixture was stirred in a microwave reactor under nitrogen conditions at 150°C (1 atm) for 2 hours. After confirming the progress of the reaction by LCMS, the reaction mixture was concentrated, and subjected to flash column chromatography ((SiO₂, MeOH /DCM = 0-10%) to obtain the desired title compound (550 mg, 92.32%) as a brown oil.

¹H NMR (400 MHz, DMSO-d₆); δ 11.77 (s, 1 H), 8.15 (m, 2 H), 8.09 (s, 1 H), 8.04 (s, 1 H), 7.87 (m, 2H), 7.30 (s, 1 H), 4.67 (s, 2 H), 3.62 - 3.52 (m, 4 H), 3.27 (s, 3 H), 1.71 (s, 3 H), 1.68 (s, 3 H).

### Preparation Example 36: (4-(7-amino-5-((2-methoxyethoxy)methyl)-1H-indol-2-yl)phenyl)dimethylphosphine oxide

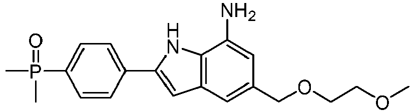

(4-(5-((2-methoxyethoxy)methyl))-7-nitro-1H-indol-2-yl)phenyl)dimethylphosphine oxide obtained in Preparation Example 35 was dissolved in THF (10 mL), and then Pd/C (120 mg, 10% purity) was added thereto, and stirred in hydrogen (15 psi) at room temperature for 2 hours. The reactant was filtered using Celite and concentrated to obtain the desired title compound (290 mg). It was used in the next reaction without further purification.

### Example 56: (4-(7-(cyclopentylamino)-5-((2-methoxyethoxy)methyl)-1H-indol-2-yl)phenyl)dimethylphosphine oxide

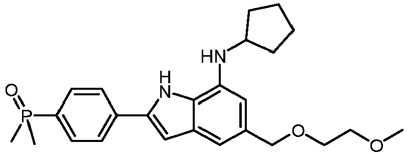

(4-(7-amino-5-((2-methoxyethoxy)methyl)-1H-indol-2-yl)phenyl)dimethylphosphine oxide obtained in Preparation Example 36 and commercially available cyclopentanone (1 equivalent) were reacted in the same method as in Example 1 to obtain the title compound.

¹H NMR (400 MHz, DMSO-d₆): δ 11.07 (s, 1 H), 7.97 - 7.90 (m, 2 H), 7.88 - 7.80 (m, 2 H), 6.94 (d, *J* = 1.6 Hz, 1 H), 6.84 (s, 1 H), 6.33 (s, 1 H), 4.46 (s, 2 H), 3.97 - 3.89 (m, 1 H), 3.53 - 3.51 (m, 4 H), 3.25 (s, 3 H), 2.10 - 1.99 (m, 2 H), 1.81 - 1.73 (m, 2 H), 1.70 (s, 3 H), 1.66 (s, 3H), 1.65 (m, 4 H)

LCMS: 94.69%, MS (ESI): m/z 441.3 [M+H]⁺

### Example 57: (4-(5-((2-methoxyethoxy)methyl)-7-((tetrahydro-2H-pyran-4-yl)amino-1H-indol-2-yl)phenyl)dimethylphosphine oxide

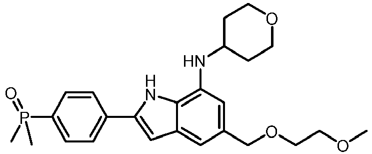

(4-(7-amino-5-((2-methoxyethoxy)methyl)-1H-indol-2-yl)phenyl)dimethylphosphine oxide obtained in Preparation Example 36 and commercially available tetrahydro-4H-pyran-4-one (1 equivalent) were reacted in the same method as in Example 1 to obtain the title compound.

¹H NMR (400 MHz, DMSO-d₆); δ 11.07 (s, 1 H), 7.97 - 7.91 (m, 2 H), 7.89 - 7.80 (m, 2 H), 6.93 (d, *J* = 2.0 Hz, 1 H), 6.81 (s, 1 H), 6.35 (s, 1 H), 4.45 (s, 2 H), 4.00 - 3.90 (m, 2 H), 3.70 - 3.61 (m, 1 H), 3.53 - 3.48 (m, 4 H), 3.48 - 3.45 (m, 3 H), 3.25 (s, 3 H), 2.10 - 2.00 (m, 2H), 1.68 (d, *J* = 13.6 Hz, 6 H), 1.55 - 1.41 (m, 2 H)

LCMS: 97.30%, MS (ESI): m/z 457.3 [M+H]⁺

### Example 58: 4-(4-(7-(cyclopentylamino)-5-((2-methoxyethoxy)methyl)-1H-indol-2-yl)phenyl)-2-methylbut-3-yn-2-ol

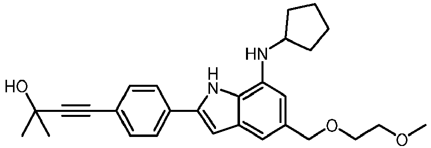

2-(4-bromophenyl)-N-cyclopentyl-5-((2-methoxyethoxy)methyl)-1H-indol-7-amine obtained in Example 54 was dissolved in DMF (3 mL), and CuI (3.87 mg, 20.30 µmol, 0.1 eq.), 2-methylbut-3-yn-2-ol (51.22 mg, 608.96 µmol, 59.49 µL, 3 eq.), K₂CO₃ (84.16 mg, 608.96 µmol, 3 eq.), and dichloropalladium; tricyclohexylphosphane (14.98 mg, 20.30 µmol, 0.1 eq.) were added thereto. This reaction mixture was stirred in a microwave reactor at 120°C for 2 hours. The reaction was confirmed by LCMS, and the reaction was terminated by adding water. The organic layer was washed with H₂O (20 mL) and brine (10 mL) by adding EA thereto, dried over Na₂SO₄, and filtered. The filtrate was concentrated under reduced pressure and purified by prep TLC PE/EtOAc = 1/1) and titration [MTBE/PE=20/1 (2 mL)] to obtain the desired title compound (14.9 mg, 7.82%) as a gray solid.

¹H NMR (400 MHz, DMSO-d₆); δ 10.95 (d, *J* = 1.6 Hz, 1 H), 7.83 - 7.74 (m, 2 H), 7.52 - 7.43 (m, 2 H), 6.82 (d, *J* = 2 Hz, 1 H), 6.75 (s, 1 H), 6.23 (s, 1 H), 5.49 (s, 1 H), 5.42 (d, *J* = 6.4 Hz, 1 H), 4.44 (s, 2 H), 3.93 - 3.83 (m, 1 H), 3.54 - 3.42 (m, 4 H), 3.25 (s, 3 H), 2.10 - 1.99 (m, 2 H), 1.81 - 1.71 (m, 2 H), 1.67 - 1.53 (m, 4 H), 1.48 (s, 6 H)

LCMS: 93.75%, MS (ESI): m/z 447.2 [M+H]⁺

### Example 59: 4-(4-(5-((2-methoxyethoxy)methyl)-7-((tetrahydro-2H-pyran-4-yl)amino)-1H-indol-2-yl)phenyl)-2-methylbut3-yn-2-ol

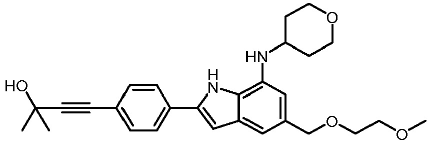

2-(4-bromophenyl)-5-((2-methoxyethoxy)methyl)-N-(tetrahydro-2H-pyran-4-yl)-1H-indole-7-amine (100 mg, 217.69 µmol, 1 eq.) obtained in Example 55 was used in the same method as in Example 58 to obtain the desired title compound (31.6 mg, 14.89%) as a green solid.

¹H NMR (400 MHz, DMSO-d₆); δ 10.98 (s, 1 H), 7.86 - 7.73 (m, 2 H), 7.54 - 7.43 (m, 2 H), 6.84 (d, *J* = 2 Hz, 1 H), 6.77 (s, 1 H), 6.31 (s, 1 H), 5.49 (s, 1 H), 5.37 (d, *J* = 7.6 Hz, 1 H), 4.44 (s, 2 H), 3.98 - 3.90 (m, 2 H), 3.69 - 3.59 (m, 1 H), 3.54 - 3.44 (m, 6 H), 3.25 (s, 3 H), 2.11 - 2.00 (m, 2 H), 1.49 (s, 6 H), 1.46 - 1.43 (m, 1 H), 1.33 - 1.30 (m, 1 H).

LCMS: 95.04%, MS (ESI): m/z 463.3 [M+H]⁺

### Example 60: 5-((2-methoxyethoxy)methyl)-N-(tetrahydro-2H-pyran-4-yl)-2-(p-tolyl)-1H-indol-7-amine

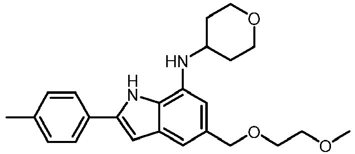

2-(4-bromophenyl)-5-((2-methoxyethoxy)methyl)-N-(tetrahydro-2H-pyran-4-yl)-1H-indole-7-amine (70 mg, 152.38 µmol, 1 eq.) obtained in Example 55 was dissolved in dioxane (4 mL) and H₂O (1 mL), and then methylboronic acid (10.95 mg, 182.86 µmol, 1.2 eq.), Pd(dppf)Cl₂ (11.15 mg, 15.24 µmol, 0.1 eq.) and K₂CO₃ (63.18 mg, 457.14 µmol, 3 eq.) were added and stirred under nitrogen at 100°C for 12 hours. After confirming the completion of the reaction by LCMS, EA was added, and the filtrate was concentrated after filtering with Celite. The concentrated residue was subjected to prep-TLC (PE/EA = 1/2) and prep-HPLC (column: Welch Xtimate C18 150*30 mm*5 µm; mobile phase: [water (NH₄HCO₃)-ACN]; B%: 27% -73%, 12min) to obtain the desired title compound (12.4 mg, 19.80% yield, 96% purity) as a blue solid.

¹H NMR (400 MHz, DMSO-d₆); δ 10.87 (s, 1 H), 7.69 (d, *J* = 8.0 Hz, 2 H), 7.28 (d, *J* = 7.6 Hz, 2 H), 6.77 - 6.68 (m, 2 H), 6.28 (s, 1 H), 5.34 (d, *J* = 7.6 Hz, 1 H), 4.43 (s, 2 H), 3.97 - 3.89 (m, 2 H), 3.69 - 3.56 (m, 1 H), 3.51 (m, 6 H), 3.24 (s, 3 H), 2.36 - 2.32 (m, 3 H), 2.09 - 2.01 (m, 2 H), 1.52 - 1.40 (m, 2 H).

LCMS: 96.29%, MS (ESI): m/z 395.1 [M+H]⁺

### Preparation Example 37: 5-((3-methoxypropyl)methyl)-7-nitro-2-phenyl-1H-indole

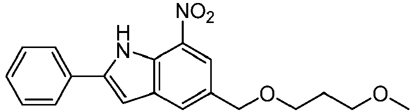

5-(bromomethyl)-7-nitro-2-phenyl-1H-indole obtained in Preparation Example 2 and commercially available 3-methoxypropan-1-ol were reacted in the same method as in Preparation Example 2 to obtain the desired title compound.

¹H NMR (400MHz, CDCl₃); δ 10.94 (br, 1H), 8.16 (s, 1H), 7.95 (s, 1H), 7.76 (d, 2H), 7.53 (t, 2H), 7.45 (t, 1H), 6.95 (s, 1H), 4.67 (s, 2H), 3.65 (t, 2H), 3.53 (t, 2H), 3.37 (s, 3H), 1.95 (m, 2H).

### Preparation Example 38: 5-((3-methoxypropyl)methyl)-2-phenyl-1H-indol-7-amine

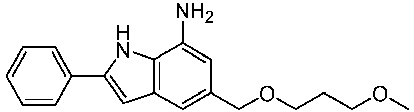

5-((3-methoxypropyl)methyl)-7-nitro-2-phenyl-1H-indole obtained in Preparation Example 37 was treated in the same method as in Preparation Example 4 to obtain the desired title compound.

¹H NMR (400MHz, CDCl₃); δ 8.25 (br, 1H), 7.70 (d, 2H), 7.46 (t, 2H), 7.36 (t, 1H), 7.14 (s, 1H), 6.80, (s, 1H), 6.64 (s, 1H), 4.55 (s, 2H), 3.60 (t, 2H), 3.52 (t, 2H), 3.36, (s, 3H), 1.92 (m, 2H).

### Example 61: N-cyclopentyl-5-((3-methoxypropoxy)methyl)-2-phenyl-1H-indol-7-amine

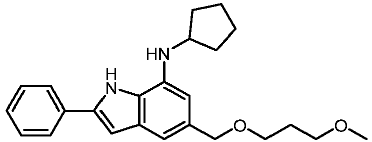

5-((3-methoxypropyl)methyl)-2-phenyl-1H-indol-7-amine obtained in Preparation Example 38 and commercially available cyclopentanone (1 equivalent) were reacted in the same method as in Example 1 to obtain the title compound.

¹H NMR (400MHz, CDCl₃); δ 8.27 (s, 1H), 7.70 (d, 2H), 7.45 (t, 2H), 7.34 (t, 1H), 7.07 (s, 1H), 6.79 (s, 1H), 6.57 (s, 1H), 4.59 (s, 2H), 3.39 (m, 1H), 3.59 (t, 2H), 3.53 (t, 2H), 3.36 (s, 3H), 2.14 (m, 2H), 1.92 (m, 2H), 1.81 (m, 2H), 1.75 -1.60 (m, 4H).

ESI-MS (*m*/*z*): 379.3 [M+H]⁺

### Example 62: 5-((3-methoxypropoxy)methyl)-N-(oxetan-3-yl)-2-phenyl-1H-indol-7-amine

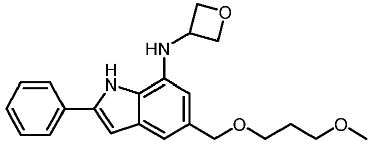

5-((3-methoxypropyl)methyl)-2-phenyl-1H-indol-7-amine obtained in Preparation Example 38 and commercially available oxetan-3-one (1 equivalent) were reacted in the same method as in Example 1 to obtain the title compound.

¹H NMR (400MHz, CDCl₃); δ 8.64 (br, 1H), 7.73 (d, 2H), 7.48 (t, 2H), 7.37 (t, 1H), 7.12 (s, 1H), 6.81 (s, 1H), 3.17 (s, 1H), 5.13 (m, 2H), 4.66 (m, 1H), 4.64 (m, 2H), 4.55 (s, 2H), 4.30 (br, 1H), 3.59 (t, 2H), 3.53 (t, 2H), 3.36 (s, 3H), 1.92 (m, 2H).

ESI-MS (*m*/*z*): 366.2 [M+H]⁺

### Example 63: 5-((3-methoxypropoxy)methyl)-2-phenyl-N-(tetrahydro-2H-pyran-4-yl)-1H-indol-7-amine

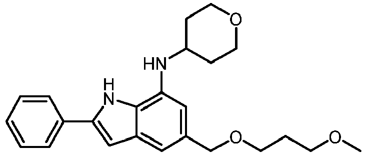

5-((3-methoxypropyl)methyl)-2-phenyl-1H-indol-7-amine obtained in Preparation Example 38 and commercially available tetrahydro-4H-pyran-4-one (1 equivalent) were reacted in the same method as in Example 1 to obtain the title compound.

¹H NMR (400 MHz, CDCl₃); δ 8.69 (br, 1 H), 7.73 (d, 2 H), 7.46 (t, 2 H), 7.35 (t, 1 H), 7.15 (s, 1 H), 6.80 (s, 1 H), 6.64 (s, 1 H), 4.56 (s, 2 H), 4.05 (d, 2 H), 3.69 (m, 1 H), 3.59-3.49 (m, 6 H), 3.35 (s, 3 H), 2.13 (d, 2 H), 1.91 (m, 2 H), 1.68 (m, 2 H).

MS (ESI): *m*/*z* 395.3 [M+H]⁺

### Example 64: 5-((2-methoxyethoxy)methyl)-2-phenyl-N-(piperidin-4-yl)-1H-indol-7-amine

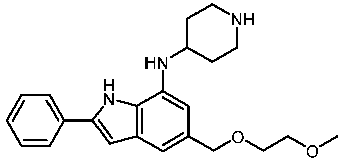

tert-Butyl-4-[[5-(2-methoxyethoxymethyl)-2-phenyl-1H-indol-7-yl]amino]piperidine-1-carboxylate obtained in Example 29 was subjected to deprotection using 2M hydrogen chloride/dioxane to obtain the desired title compound.

¹H NMR (400MHz, DMSO-d₆); δ 11.71 (br, 1H), 8.85 (br, 1H), 8.77 (br, 1H), 7.94 (d, 2H), 7.47 (t, 2H), 7.32 (t, 1H), 7.02 (br, 1H), 6.87 (s, 1H), 6.55 (br, 1H), 4.49 (s, 2H), 3.81 (m, 1H), 3.55-3.43 (m, 6H), 3.26 (s, 3H), 3.05 (m, 2H), 2.19 (m, 2H), 1.89 (m, 2H).

ESI-MS (*m*/*z*): 380.3 [M+H]⁺

### Preparation Example 39: tert-Butyl 5-(3-hydroxypropyl)-7-nitro-2-phenyl-1H-indole-1-carboxylate

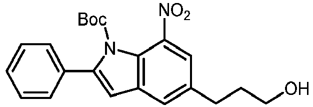

### Step 1: 7-nitro-2-phenyl-1H-indole-carbaldehyde

(7-nitro-2-phenyl-1H-indol-5-yl)methanol (5 g, 18.6 mmol) obtained in Preparation Example 1 was dissolved in DCM (300 mL) and acetone (100 mL), and pyridinium chlorochromate (5.63 g, 26.12 mmol) was introduced thereto. The mixture was heated to reflux overnight. After confirming the completion of the reaction by TLC, it was filtered through silica gel and then concentrated to obtain 7-nitro-2-phenyl-1H-indole-carbaldehyde (5 g, yield 100%) as a yellow solid.

¹H NMR (400MHz, CDCl₃); δ 10.34 (br, 1H), 10.15 (s, 1H), 8.68 (s, 1H), 8.51 (s, 1H), 7.79 (d, 2H), 7.57 (t, 2H), 7.49 (t, 1H), 7.12 (s, 1H).

Mass [M + H] = 267.07 (M +1)

### Step 2: tert-Butyl 5-formyl-7-nitro-2-phenyl-1H-indole-1-carboxylate

5 g (18.7 mmol) of 7-nitro-2-phenyl-1H-indole-carbaldehyde obtained in Step 1 was dissolved in DCM (250 mL), and TEA (6.54 mL, 46.95 mmol), (Boc)₂O (8.19 g, 37.56 mmol) and DMAP (0.229 g, 1.878 mmol) were introduced thereto. The mixture was stirred at room temperature for two days. The completion of the reaction was confirmed by TLC, and the concentrated residue was purified by column chromatography (Hex/EA, 10/1) to obtain tert-butyl 5-formyl-7-nitro-2-phenyl-1H-indole-1-carboxylate (5.2 g, yield 75%).

¹H NMR (400MHz, CDCl₃); δ 10.12 (s, 1H), 8.38 (d, 2H), 7.49 (m, 5H), 6.77 (s, 1H), 1.31 (s, 9H).

Mass [M + H] = 367.12 (M +1)

### Step 3: 3-(1-(tert-butoxycarbonyl)-7-nitro-2-phenyl-1H-indol-5-yl)propionic acid

Formic acid (1.63 mL, 42.57 mmol), TEA (2.57 mL, 18.45 mmol), 2,2-dimethyl-1,3-dioxane-4,6-dione (2.045 g, 14.19 mmol) were introduced to dimethylformamide (20 mL) at 10°C. tert-Butyl 5-formyl-7-nitro-2-phenyl-1H-indole-1-carboxylate (5.2 g, 14.19 mmol) obtained in Step 2 was added, and then stirred at 80°C overnight. After confirming the completion of the reaction by TLC, a saturated aqueous NH₄Cl solution was added to the reaction solution at 10°C and stirred for 30 minutes. The organic layer was washed three times with a saturated aqueous NH₄Cl solution, washed again with water, and washed with a saturated NaCl solution. After drying over MgSO₄, the filtered filtrate was concentrated. 3-(1-(tert-butoxycarbonyl)-7-nitro-2-phenyl-1H-indol-5-yl)propionic acid (6 g, yield 104%) was obtained as an orange oil.

¹H NMR (400MHz, CDCl₃); δ 7.85 (s, 1H), 7.78 (s, 1H), 7.51 (m, 2H), 7.46 (m, 2H), 6.61 (s, 1H), 3.84 (s, 2H), 1.30 (s, 9H).

Mass [M + H] = 410.15 (M +1)

### Step 4: tert-Butyl 5-(3-hydroxypropyl)-7-nitro-2-phenyl-1H-indole-1-carboxylate

3-(1-(tert-butoxycarbonyl)-7-nitro-2-phenyl-1H-indol-5-yl)propionic acid (6 g, 14.76 mmol) obtained in Step 3 was dissolved in THF (120 mL) and cooled to 0°C. BH₃·SMe₂ 2M in THF (22.05 mL, 44.28 mmol) was added, and the mixture was warmed to room temperature, and stirred for 5 hours. The completion of the reaction was confirmed by TLC and the mixture was cooled to 0°C. 1 N NaOH aqueous solution was slowly added dropwise and stirred for 1 hour. The mixture was extracted twice with EA, and the organic layer was dried over MgSO₄, filtered, and concentrated under reduced pressure. The concentrated residue was purified by silica gel column chromatography (Hex/EA, 2/1) to obtain tert-butyl 5-(3-hydroxypropyl)-7-nitro-2-phenyl-1H-indole-1-carboxylate (3.2 g, yield 55%) as a yellow oil.

¹H NMR (400MHz, CDCl₃); δ 7.76 (s, 1H), 7.67 (s, 1H), 7.54 (m, 2H), 7.44 (m, 3H), 6.58 (s, 1H), 3.75 (t, 2H), 2.91 (t, 2H), 1.99 (m, 2H), 1.30 (s, 9H).

Mass [M + H] = 397.17 (M +1)

### Preparation Example 40: 5-(3-(2-methoxyethoxy)propyl)-2-phenyl-1H-indol-7-amine

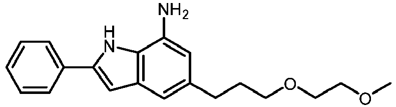

### Step 1: tert-Butyl 5-(3-((methylsulfonyl)oxy)propyl)-7-nitro-2-phenyl-1H-indole-1-carboxylate

tert-Butyl 5-(3-hydroxypropyl)-7-nitro-2-phenyl-1H-indole-1-carboxylate (300 mg, 0.756 mmol) obtained in Preparation Example 39 was dissolved in DCM (6 mL), TEA (0.255 mL, 1.82 mmol) was added dropwise, followed by cooling to 0°C. Methanesulfonyl chloride (0.07 mL, 0.908 mmol) was added dropwise and stirred at room temperature for 2 hours. After confirming the completion of the reaction by TLC, the mixture was extracted twice with EA and washed with a saturated NaHCO₃ solution. The organic layer was dried over MgSO₄, filtered, and concentrated under reduced pressure. The concentrated residue was purified by silica gel column chromatography (Hex/EA, 1/1) to obtain a yellow oil (342 mg, yield 95%) of tert-butyl 5-(3-((methylsulfonyl)oxy)propyl)-7-nitro-2-phenyl-1H-indole-1-carboxylate.

¹H NMR (400MHz, CDCl₃); δ 7.74 (s, 1H), 7.70 (s, 1H), 7.54 (m, 2H), 7.46 (m, 3H), 6.59 (s, 1H), 4.29 (t, 2H), 3.06 (s, 3H), 2.96 (t, 2H), 2.20 (m, 2H), 1.30 (s, 9H).

### Step 2: 5-(3-(2-methoxyethoxy)propyl)-7-nitro-2-phenyl-1H-indole

tert-Butyl 5-(3-((methylsulfonyl)oxy)propyl)-7-nitro-2-phenyl-1H-indole-1-carboxylate (342 mg, 0.72 mmol) obtained in Step 1 was dissolved in 2-methoxyethan-1-ol (6 mL), and potassium iodide (12 mg, 0.075 mmol) was added, and then heated to reflux overnight. The reaction completion was confirmed by TLC, and the mixture was cooled to room temperature. It was extracted twice with EA, washed with a saturated NaHCO₃ solution, and then washed with a saturated aqueous NaCl solution. The organic layer was dried over MgSO₄, filtered, and concentrated under reduced pressure. The concentrated residue was purified by silica gel column chromatography (Hex/EA, 5/1) to obtain a yellow solid (261 mg, yield 96%) of 5-(3-(2-methoxyethoxy)propyl)-7-nitro-2-phenyl-1H-indole.

¹H NMR (400MHz, CDCl₃); δ 10.00 (s, 1H), 7.99 (s, 1H), 7.78 (s, 1H), 7.74 (d, 2H), 7.50 (t, 2H), 7.41 (t, 1H), 6.88 (s, 1H), 3.59 (m, 4H), 3.51 (t, 2H), 3.42 (s, 3H), 2.88 (t, 2H), 2.00 (m, 2H).

### Step 3: 5-(3-(2-methoxyethoxy)propyl)-2-phenyl-1H-indol-7-amine

5-(3-(2-methoxyethoxy)propyl)-7-nitro-2-phenyl-1H-indole (100 mg, 0.283 mmol) obtained in Step 2 was dissolved in THF (2 mL) and EA (2 mL), and palladium carbon 50%/water (30 mg, 30 wt%) was introduced thereto. A hydrogen balloon was inserted therein and the mixture was stirred for 2 hours and 40 minutes. The reaction solution was filtered through Celite, and then concentrated under reduced pressure, and filtered through silica gel, and then concentrated under reduced pressure to obtain the title compound 5-(3-(2-methoxyethoxy)propyl)-2-phenyl-1H-indol-7-amine (89 mg, yield 96%) as a pale brown solid.

¹H NMR (400MHz, CDCl₃); δ 8.30 (br, 1H), 7.67 (d, 2H), 7.42 (t, 2H), 7.30 (t, 1H), 6.97 (s, 1H), 6.73 (s, 1H), 6.44 (s, 1H), 3.58 (m, 4H), 3.49 (t, 2H), 3.40 (s, 3H), 2.70 (t, 2H), 1.95 (t, 2H).

### Example 65: 5-(3-(2-methoxyethoxy)propyl)-2-phenyl-N-(tetrahydro-2H-pyran-4-yl)-1H-indol-7-amine

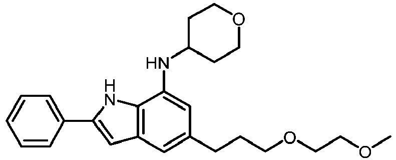

5-(3-(2-methoxyethoxy)propyl)-2-phenyl-1H-indol-7-amine obtained in Preparation Example 40 and commercially available tetrahydro-4H-pyran-4-one (1 equivalent) were reacted in the same method as in Example 1 to obtain the title compound.

¹H NMR (400MHz, CDCl₃); δ 8.47 (br, 1H), 7.72 (d, 2H), 7.46 (t, 2H), 7.32 (t, 1H), 7.03 (s, 1H), 6.76 (s, 1H), 6.51 (s, 1H), 4.06 (m, 2H), 3.61 (m, 9H), 3.43 (s, 3H), 2.76 (t, 2H), 2.13 (s, 2H), 1.99 (m, 2H), 1.69 (m, 2H).

Mass [M + H] = 409.42 (M +1)

### Preparation Example 41: ethyl 2-(7-nitro-2-phenyl-1H-indol-5-yl)acetate

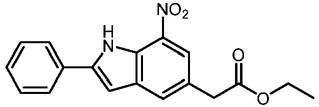

### Step 1: Ethyl 2-(4-aminophenyl)acetate

Commercially available ethyl 2-(4-nitrophenyl)acetate (20 g, 95.6 mmol) was dissolved in 300 mL of a 1:1:1 mixed solution of tetrahydrofuran, methanol, and water. Iron powder (26.7 g, 478 mmol) and NH₄Cl (25.6 g, 478 mmol) were added, and the mixture was stirred at 60°C for 2 hours. The completion of the reaction was confirmed by TLC, and the mixture was filtered through Celite at room temperature, and then concentrated under reduced pressure. The organic layer was diluted with EA, washed with a saturated NH₄Cl solution, extracted with EA, and then dried over MgSO₄, and then the filtered filtrate was concentrated under reduced pressure. Ethyl 2-(4-aminophenyl)acetate (17 g, yield 99%) was obtained.

Mass [M + H] = 180.09 (M +1)

### Step 2: Ethyl 2-(4-acetamidophenyl) acetate

Ethyl 2-(4-aminophenyl)acetate (17 g, 94.87 mmol) obtained in Step 1 was dissolved in DCM (170 mL), and TEA (19.8 mL, 142.3 mmol) was introduced thereto, and then acetic anhydride (11.6 g, 113.84 mmol) was slowly added dropwise at 0°C. The mixture was stirred at room temperature for 1 hour. After confirming the completion of the reaction by TLC, the reaction mixture was diluted with DCM, and washed with a saturated K₂CO₃ solution and a saturated NaCl solution in this order. The organic layer was dried over MgSO₄, and the filtered filtrate was concentrated under reduced pressure. The concentrated residue was recrystallized from hexane and then filtered. A pinkish solid of ethyl 2-(4-acetamidophenyl)acetate (20.6 g, yield 98%) was obtained.

¹H NMR (400MHz, CDCl₃); δ 7.48 (d, 2H), 7.32 (br, 1H), 7.26 (d, 2H), 4.17 (q, 2H), 3.59 (s, 2H), 2.18 (s, 3H), 1.27 (t, 3H).

Mass [M + H] = 222.11 (M +1)

### Step 3: Ethyl 2-(4-acetamido-3-nitrophenyl)acetate

Ethyl 2-(4-acetamidophenyl)acetate (20.6 g, 93.1 mmol) obtained in Step 2 was dissolved in acetic anhydride (309 mL), and red fuming nitric acid was slowly added dropwise at 0°C. The mixture was warmed to room temperature and stirred for 4 hours. The completion of the reaction was confirmed by TLC, water was added thereto, and the resulting solid was filtered and dried. Ethyl 2-(4-acetamido-3-nitrophenyl)acetate (24 g, 92%) was obtained.

¹H NMR (400MHz, CDCl₃); δ 10.31 (br, 1H), 8.76 (d, 1H), 8.17 (s, 1H), 7.60 (d, 1H), 4.20 (q, 2H), 3.67 (s, 2H), 2.31 (s, 3H), 1.29 (t, 3H).

Mass [M + H] = 267.09 (M +1)

### Step 4: Ethyl 2-(4-amino-3-nitrophenyl)acetate

Ethyl 2-(4-acetamido-3-nitrophenyl)acetate (24 g, 86 mmol) obtained in Step 3 was dissolved in ethanol (300 mL), and 1N HCl (160 mL) was added dropwise thereto. After stirring at room temperature overnight, the mixture was further heated to reflux overnight. After confirming the completion of the reaction by TLC, the reaction solution was concentrated, and then the organic layer was washed with saturated NaHCO₃, and dried over MgSO₄, and then the filtered filtrate was concentrated. Ethyl 2-(4-amino-3-nitrophenyl)acetate (16 g, 82%) was obtained as an orange solid.

¹H NMR (400MHz, CDCl₃); δ 8.05 (s, 1H), 7.35 (d, 1H), 6.82 (d, 1H), 6.06 (br, 1H), 4.19 (q, 2H), 3.56 (s, 2H), 1.29 (t, 3H).

Mass [M + H] = 225.08 (M +1)

### Step 5: Ethyl 2-(4-amino-3-iodo-5-nitrophenyl)acetate

Ethyl 2-(4-amino-3-nitrophenyl)acetate (16 g, 71.27 mmol) obtained in Step 4 was dissolved in ethanol (480 mL), and silver nitrate (14.5 g, 85.52 mmol) and iodine (21.7 g, 85.52 mmol) were introduced thereto, and stirred at room temperature overnight. After confirming the completion of the reaction by TLC, the reaction solution was concentrated, and then diluted with EA. The organic layer was washed three times with saturated sodium thiosulfate, dried over MgSO₄, and then the filtered filtrate was concentrated. The concentrated residue was purified by column chromatography (Hex/EA, 5/1) to obtain ethyl 2-(4-amino-3-iodo-5-nitrophenyl)acetate (17 g, yield 68%).

¹H NMR (400MHz, CDCl₃); δ 8.11 (s, 1H), 7.91 (s, 1H), 6.65 (br, 1H), 4.20 (q, 2H), 3.53 (s, 2H), 1.30 (t, 3H).

Mass [M + H] = 350.98 (M +1)

### Step 6: Ethyl 2-(7-nitro-2-phenyl-1H-indol-5-yl)acetate

Ethyl 2-(4-amino-3-iodo-5-nitrophenyl)acetate (5.67 g, 16.2 mmol) obtained in Step 5 was dissolved in anhydrous dioxane (100 mL), and TEA (6.77 mL, 48.6 mmol), and phenylacetylene (2.14 mL, 19.44 mmol), bis(triphenyl phosphine) palladium dichloride (114 mg, 0.162 mmol), and copper iodide (30.85 mg, 0.162 mmol) were introduced thereto in this order. The mixture was reacted at 60°C for 18 hours, and 1,8-diazabicyclo[5,4,0]undec-7-ene (18.3 mL, 129.6 mmol) was added to the reaction solution, and reacted at 110°C for 48 hours. The mixture was filtered through Celite at room temperature, and the filtrate was distilled under reduced pressure. EA was added to the concentrated residue, which was washed with water. The aqueous layer was re-extracted with EA, and the combined organic layers were dried over MgSO₄ and filtered. The filtrate was concentrated under reduced pressure, and the concentrated residue was purified by silica column chromatography to obtain ethyl 2-(7-nitro-2-phenyl-1H-indol-5-yl)acetate (3.3 g, yield 63%) as an orange solid.

¹H NMR (400MHz, CDCl₃); δ 10.08 (br, 1H), 8.10 (s, 1H), 7.90 (s, 1H), 7.77 (d, 2H), 7.52 (t, 2H), 7.42 (t, 1H), 6.93 (s, 1H), 4.22 (q, 2H), 3.80 (2, 1H), 1.30 (t, 3H).

### Preparation Example 42: 5-(2-(2-methoxyethoxy)ethyl)-2-phenyl-1H-indol-7-amine

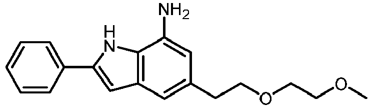

### Step 1: 2-(7-nitro-2-phenyl-1H-indol-5-yl)ethan-1-ol

Ethyl-2-(7-nitro-2-phenyl-1H-indol-5-yl)acetate (420 mg, 1.295 mmol) obtained in Step 6 of Preparation Example 41 was dissolved in THF (20 mL) and cooled to 5°C. 2M LiAlH₄/THF (1.3 mL, 2.59 mmol) was added dropwise and reacted at the same temperature for 1 hour. At the same temperature, an aqueous sodium potassium tartrate solution (5 mL) was added dropwise to terminate the reaction, and warmed to room temperature. Water and ethyl acetate were added to separate the layers, and the aqueous layer was re-extracted with EA. The combined organic layers were dried over MgSO₄ and filtered. The filtrate was concentrated under reduced pressure and dried to obtain 2-(7-nitro-2-ethyl-1H-indol-5-yl)ethan-1-ol (255 mg, yield 70%) as an orange solid.

¹H NMR (400MHz, CDCl₃); δ 10.05 (br, 1H), 8.06 (s, 1H), 7.84 (s, 1H), 7.77 (d, 2H), 7.53 (t, 2H), 7.45 (t, 1H), 6.92 (s, 1H), 4.00 (t, 2H), 3.07 (s, 3H), 1.13 (t, 1H).

### Step 2: 2-(7-nitro-2-phenyl-1H-indol-5-yl)ethyl methanesulfonate

2-(7-nitro-2-ethyl-1H-indol-5-yl)ethan-1-ol (366 mg, 1.295 mmol) obtained in Step 1 was dissolved in DCM, and TEA (546 µL, 3.89 mmol) was added dropwise and then cooled to 5°C. Methanesulfonyl chloride (150 µL, 1.94 mmol) was added thereto and reacted at room temperature for 30 minutes. The reaction solution was concentrated under reduced pressure and purified by silica column chromatography to obtain 2-(7-nitro-2-phenyl-1H-indol-5-yl)ethyl methanesulfonate (291 mg, yield 89%) as a yellow solid.

¹H NMR (400MHz, CDCl₃); δ 10.06 (br, 1H), 8.02 (s, 1H), 7.84 (s, 1H), 7.75 (d, 2H), 7.52 (t, 2H), 7.45 (t, 1H), 6.91 (s, 1H), 4.50 (t, 2H), 3.25 (t, 2H), 2.95 (s, 3H).

### Step 3: 5-(2-(2-methoxyethoxy)ethyl)-7-nitro-2-phenyl-1H-indole

2-(7-nitro-2-phenyl-1H-indol-5-yl)ethyl methane sulfonate (290 mg, 0.805 mmol) obtained in Step 2 was introduced to ethylene glycol (20 mL), and 19 mg (0.1165 mmol) of potassium iodide was added thereto. The mixture was reacted by heating to reflux for 40 hours, and then cooled to room temperature, and EA and water were added thereto. The layers were purified, and the aqueous layer was re-extracted with EA. The combined organic layers were dried over MgSO₄ and filtered. The filtrate was concentrated under reduced pressure and purified by silica column chromatography to obtain 5-(2-(2-methoxyethoxy)ethyl)-7-nitro-2-phenyl-1H-indole (120 mg, yield 44%) as an orange solid.

¹H NMR (400MHz, CDCl₃); δ 10.04 (br, 1H), 8.06 (s, 1H), 7.85 (s, 1H), 7.77 (d, 2H), 7.53 (t, 2H), 7.45 (t, 1H), 6.91 (s, 1H), 3.81 (t, 2H), 3.65 (t, 2H), 3.59 (t, 2H), 3.42 (s, 3H), 3.10 (t, 2H).

### Step 4: 5-(2-(2-methoxyethoxy)ethyl)-2-phenyl-1H-indol-7-amine

5-(2-(2-methoxyethoxy)ethyl)-7-nitro-2-phenyl-1H-indole (120 mg, 0.352 mmol) obtained in Step 3 was dissolved in an EA and methanol (1:1) solution, and palladium carbon (10%)/water 50% (50 mg) was added thereto. The mixture was reacted for 4 hours under a hydrogen balloon, filtered through Celite, and washed with EA. The filtrate was concentrated under reduced pressure and dried to obtain 5-(2-(2-methoxyethoxy)ethyl)-2-phenyl-1H-indol-7-amine (108 mg, yield 99%) as a brown solid.

Mass [M + H] = 311.1 (M+1)

### Example 66: N-cyclopentyl-5-(2-(2-methoxyethoxy)ethyl)-2-phenyl-1H-indol-7-amine

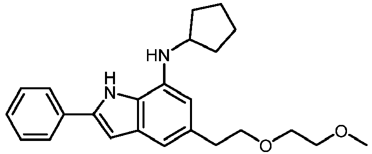

5-(2-(2-methoxyethoxy)ethyl)-2-phenyl-1H-indol-7-amine obtained in Preparation Example 42 and commercially available cyclopentanone (1 equivalent) were reacted in the same method as in Example 1 to obtain the title compound.

¹H NMR (400MHz, CDCl₃); δ 8.27 (br, 1H), 7.69 (d, 2H), 7.42 (t, 2H), 7.31 (t, 1H), 6.96 (s, 1H), 6.73 (s, 1H), 6.45 (s, 1H), 3.98 (m, 1H), 3.72 (t, 2H), 3.63 (t, 2H), 3.58 (t, 2H), 3.40 (s, 3H), 2.98 (t, 2H), 2.11 (m, 2H), 1.78 (m, 2H), 1.66 (m, 4H).

Mass [M + H] = 379.2 (M +1)

### Example 67: 5-(2-(2-methoxyethoxy)ethyl)-2-phenyl-N-(tetrahydro-2H-pyran-4-yl)-1H-indole-7-amine

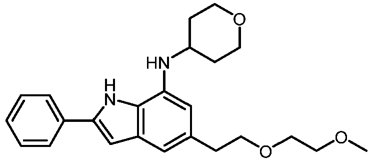

5-(2-(2-methoxyethoxy)ethyl)-2-phenyl-1H-indol-7-amine obtained in Preparation Example 42 and commercially available tetrahydro-4H-pyran-4-one (1 equivalent) were reacted in the same method as in Example 1 to obtain the title compound.

¹H NMR (400MHz, CDCl₃); δ 8.62 (br, 1H), 7.71 (d, 2H), 7.43 (t, 2H), 7.33 (t, 1H), 7.03 (s, 1H), 6.74 (s, 1H), 6.51 (s, 1H), 4.04 (m, 2H), 3.71 (t, 2H), 3.63 (t, 2H), 3.57 (t, 2H), 3.46 (m, 1H), 3.40 (s, 3H), 2.96 (t, 2H), 2.13 (m, 2H), 1.67 (m, 2H).

Mass [M + H] = 395.2 (M +1)

### Example 68: 5-((2-methoxyethoxy)methyl)-2-phenyl-7-(piperidin-1-yl)-1H-indole

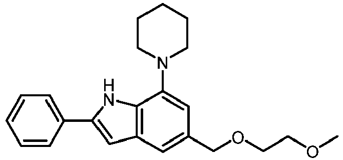

5-((2-methoxyethoxy)methyl)-2-phenyl-1H-indol-7-amine (50 mg, 0.1687 mmol) obtained in Preparation Example 4 was dissolved in DMF, and K₂CO₃ (28 mg, 0.202 mmol) was introduced thereto. 1,5-Dibromopentane (27.6 µL, 0.202 mmol) was added thereto, and the mixture was reacted at 80°C for 18 hours. The reaction solution was cooled to room temperature, and EA and water were added thereto, and the layers were purified. The organic layer was dried over MgSO₄ and filtered. The filtrate was concentrated under reduced pressure, and purified by silica column chromatography to obtain 5-((2-methoxyethoxy)methyl)-2-phenyl-7-(piperidin-1-yl)-1H-indole (30 mg, yield 49%).

¹H NMR (400MHz, CDCl₃); δ 8.37 (br, 1H), 7.70 (d, 2H), 7.48 (t, 2H), 7.37 (t, 1H), 7.31 (s, 1H), 6.89 (s, 1H), 6.81 (s, 1H), 4.66 (s, 2H), 3.66 - 3.60 (m, 4H), 3.42 (s, 3H), 3.13 (br s, 4H), 1.85 (br s, 4H), 1.66 (br s, 2H).

ESI-MS (*m*/*z*): 365.2 [M+H]⁺

### Examples 69-1 and 69-2: 4-((5-((2-methoxyethoxy)methyl)-2-phenyl-1H-indol-7-yl)amino)cyclohexan-1-ol

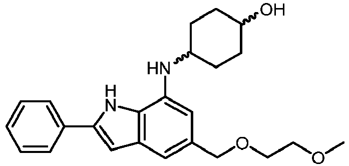

Using 5-((2-methoxyethoxy)methyl)-2-phenyl-1H-indol-7-amine and 4-hydroxycyclohexan-1-one obtained in Preparation Example 4, the title compound was purified in the same method as in Example 2 into two isomers, respectively, by silica column chromatography.

### Isomer 1: Example 69-1

¹H NMR (400MHz, DMSO-d₆); δ 10.99 (br, 1H), 7.81 (d, 2H), 7.48 (t, 2H), 7.31 (t, 1H), 6.78 (s, 1H), 6.74 (s, 1H), 6.24 (s, 1H), 5.31 (d, 1H), 4.47 (d, 1H), 4.44 (s, 2H), 3.78 (m, 1H), 3.51 (m, 2H), 3.48 (m, 2H), 3.26 (s, 3H), 1.76 (m, 6H), 1.63 (m, 2H).

### Isomer 2: Example 69-2

¹H NMR (400MHz, DMSO-d₆); δ 10.91 (br, 1H), 7.80 (d, 2H), 7.47 (t, 2H), 7.31 (t, 1H), 6.77 (s, 1H), 6.74 (s, 1H), 6.24 (s, 1H), 5.26 (d, 1H), 4.60 (d, 1H), 4.45 (s, 2H), 3.51 (m, 2H), 3.48 (m, 2H), 3.37 (m, 1H), 3.27 (s, 3H), 2.14 (m, 2H), 1.93 (m, 2H), 1.34 (m, 4H).

### Examples 70-1 and 70-2: 4-((5-((2-methoxyethoxy)methyl)-2-phenyl-1H-indol-7-yl)amino)-1-methylcyclohexane-1-ol

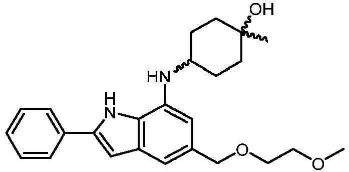

Using 5-((2-methoxyethoxy)methyl)-2-phenyl-1H-indol-7-amine obtained in Preparation Example 4 and 4-hydroxy-4-methylcyclohexan-1-one, the title compound was purified in the same method as in Example 2 into two isomers, respectively, by silica column chromatography.

### Isomer 1: Example 70-1

¹H NMR (400MHz, DMSO-d₆); δ 10.93 (br, 1H), 7.81 (d, 2H), 7.47 (t, 2H), 7.31 (t, 1H), 6.77 (s, 1H), 6.73 (s, 1H), 6.24 (s, 1H), 5.31 (d, 1H), 4.43 (s, 2H), 4.12 (s, 1H), 3.51 (m, 2H), 3.48 (m, 2H), 3.33 (m, 1H), 3.26 (s, 3H), 1.85 (m, 2H), 1.65 (m, 4H), 1.49 (m, 2H), 1.16 (s, 3H).

### Isomer 2: Example 70-2

¹H NMR (400MHz, DMSO-d₆); δ 10.94 (br, 1H), 7.81 (d, 2H), 7.48 (t, 2H), 7.31 (t, 1H), 6.77 (s, 1H), 6.74 (s, 1H), 6.24 (s, 1H), 5.27 (d, 1H), 4.44 (s, 2H), 4.30 (s, 1H), 3.52 - 3.42 (m, 5H), 3.26 (s, 3H), 2.03 (m, 2H), 1.65 (m, 2H), 1.52 - 1.42 (m, 4H), 1.20 (s, 3H).

### Experimental Example 1: Measuring protective effect with regard to heart cells (H9C2, white mouse cardiomyocytes)

In order to identify the protective effect with regard to heart cells, H9C2 cells were divided into 96-well plates by 1.5 × 10⁴, and cultured for 24 hours. The respective wells were treated with compounds of the examples 3x serial diluted to final concentrations of 30, 10, 3, 1, 0.3, 0.1, 0.03 and 0.01 µM, then incubated for 15 to 20 minutes. After treating with t-BHP with a final concentration of 400µM, incubation was performed for 2 hours. To confirm the protective effect of each compound, a Cytotoxicity LDH assay kit [Dojindo; CK12] was used to measure the degree of extracellular secretion of LDH. After 2-hour treatment of the cells with t-BHP was completed, 100µL each of the supernatant in which the cells were placed was collected and moved to a new 96-well plate. 100µL/well of the assay buffer included in the kit (same amount as the sample) was added, followed by wrapping with foil and reacting for 30 minutes at room temperature. Observing the change of color in the sample, 50µL/well of stop solution was added, then an iD3 spectrometer was used to measure absorbance at 490nm and calculate the EC₅₀ values. The EC₅₀ of the compounds of the examples is preferably no more than 1.0 µM, and more preferably no more than 0.3 µM.

Table 1 shows the protective effect of the compounds of the examples against the necrosis-inducing substance t-BHP.

**Table 1: Cell Protection Effect against t-BHP in Heart Cells**

| Example | EC₅₀ (µM) | Example | EC₅₀ (µM) | Example | EC₅₀ (µM) |
|---|---|---|---|---|---|
| 1 | A | 25 | A | 49 | B |
| 2 | A | 26 | A | 50 | C |
| 3 | A | 27 | A | 51 | A |
| 4 | A | 28 | A | 52 | A |
| 5 | C | 29 | A | 53 | B |
| 6 | A | 30 | A | 54 | A |
| 7 | A | 31 | A | 55 | A |
| 8 | A | 32 | A | 56 | A |
| 9 | A | 33 | A | 57 | C |
| 10 | B | 34 | A | 58 | B |
| 11 | A | 35 | A | 59 | B |
| 12 | A | 36 | A | 60 | A |
| 13 | A | 37 | A | 61 | A |
| 14 | A | 38 | A | 62 | A |
| 15 | A | 39 | A | 63 | B |
| 16 | A | 40 | B | 64 | A |
| 17 | A | 41 | C | 65 | A |
| 18 | A | 42 | B | 66 | A |
| 19 | A | 43 | B | 67 | A |
| 20 | A | 44 | A | 68 | C |
| 21 | A | 45 | A | 69-1 | A |
| 22 | A | 46 | A | 69-2 | A |
| 23 | A | 47 | A | 70-1 | A |
| 24 | A | 48 | A | 70-2 | A |
| A indicates EC₅₀ ≤ 0.3 µM, B indicates 0.3 µM < EC₅₀ ≤ 1µM, C indicates EC₅₀ > 1.0 µM | | | | | |

### Experimental Example 2: The Maintenance Effect on Intracellular Calcium Level in Heart Cells (H9C2, white mouse cardiomyocytes) treated with t-BHP

To examine the maintenance effect on calcium level in heart cells, H9C2 cells were divided into a 96-well plate at 1.5 × 10⁴ cells/well and cultured for 24 hours. To measure intracellular calcium level, a FLIPR Calcium 6 assay kit (Molecular devices; #R8190) was used. Following the experimental method provided by the manufacturer, cells were treated with probenecid and calcium-sensitive dye for 1.75 hours, and then the cells were treated with the compound of Example 2 for 0.25 hours (the final concentration of the compound was 0.02 or 0.01 µM). And the cells were treated with t-BHP to a final concentration of 150 µM, then intracellular calcium level was measured every 30 seconds in real time.

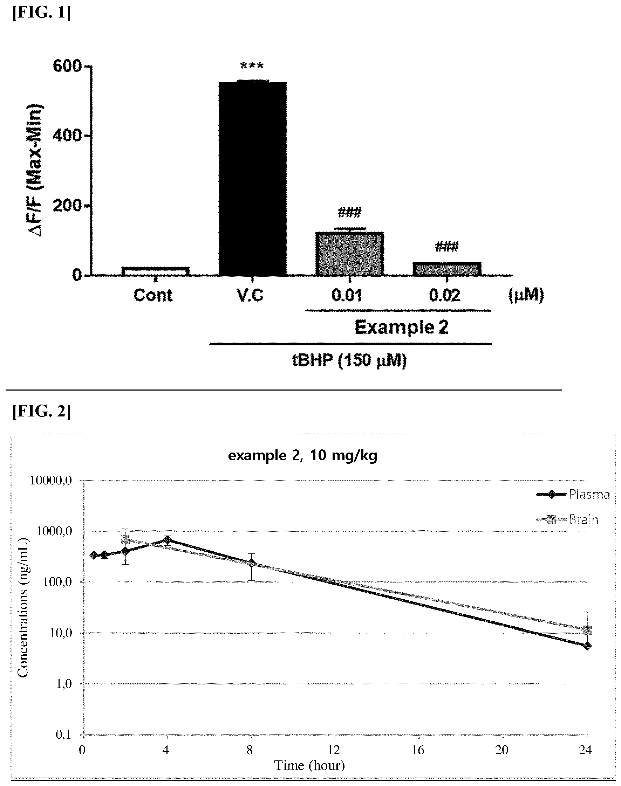

FIG. 1 is a graph regarding the regulatory effect of the compound of Example 2 on calcium level under t-BHP treatment. The ΔF/F(Max-Min) value on the vertical axis is the difference between the maximum and minimum fluorescence intensity values of the calcium-sensitive dye after treatment with t-BHP (150 µM), and this value increases as intracellular calcium level increases. The horizontal axis represents the concentration of the compound of Example 2, and vehicle (V.C.) represents a control group with treatment of DMSO (the same volume of the DMSO in which the compound was diluted). t-BHP increased the intracellular calcium level in the V.C group, whereas the combination of compound of Example 2 (0.01 µM) and t-BHP showed significantly decreased the intracellular calcium level. Moreover, the intracellular calcium level exhibited within normal range under 0.02 µM treatment of the compound of Example 2. Accordingly, it was confirmed that treating with the compound of Example 2 effectively reduced the abnormal increased in intracellular calcium level due to t-BHP treatment.

### Experimental Example 3: Cell death protective effect on fibroblasts (NIH/3T3, mouse embryonic fibroblasts)

In order to identify the protective effect on fibroblasts, NIH/3T3 cells were divided into 96-well plates by 8 × 10³ and cultured for 18 to 24 hours. The compounds of Examples were diluted to final concentrations of 0.1 and 1 µM, treated in each well, and incubated for 15 to 20 minutes. Erastin, glutamate, and RSL3 were treated to have final concentrations of 3 µM, 30 mM, and 3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 2 showed cell death reduction rates according to the respective concentrations of the compounds of Examples for Erastin, glutamate, and RSL3 as ferroptosis-inducing substances.

**Table 2: Cell death protective effect in fibroblasts (NIH/3T3) against Erastin, glutamate and RSL3**

| [Table 2] | | | | | | |
|---|---|---|---|---|---|---|
| | Erastin, 3 µM | | Glutamate, 30 mM | | RSL3, 3 µM | |
| Example | 0.1 µM | 1.0 µM | 0.1 µM | 1.0 µM | 0.1 µM | 1.0 µM |
| | inhibition (%) | | | | | |
| Ferrostatin-1 | 17 | 99 | 97 | 97 | 100 | 100 |
| 1 | 99 | 100 | 100 | 100 | 100 | 100 |
| 4 | 99 | 99 | 100 | 100 | 100 | 100 |
| 30 | 6 | 100 | 100 | 100 | 100 | 100 |
| 36 | 99 | 100 | 100 | 100 | 100 | 100 |
| 39 | 99 | 100 | 99 | 100 | 100 | 100 |
| 45 | 100 | 100 | 0 | 97 | 0 | 100 |
| 50 | 0 | 8 | 99 | 100 | 100 | 100 |
| 64 | 99 | 100 | 99 | 100 | 100 | 100 |

### Experimental Example 4: Cell death protective effect on adrenal cells (PC-12, white mouse adrenal cells)

In order to identify the protective effect on adrenal cells, PC-12 cells were divided into 96-well plates by 1 × 10⁴ and cultured for 18 to 24 hours. The compounds of Examples were diluted to final concentrations of 0.1 and 1.0 µM in Erastin, 0.01 and 0.1 µM in glutamate, and 0.3 and 3.0 µM in RSL3, treated in each well, and incubated for 15 to 20 minutes. Erastin, glutamate, and RSL3 were treated to have final concentrations of 1 µM, 10 mM, and 0.3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 3 showed cell death reduction rates according to the respective concentrations of the compounds of Examples for Erastin, glutamate, and RSL3 as ferroptosis-inducing substances.

**Table 3: Cell death protective effect in adrenal cells (PC-12) against Erastin, glutamate, and RSL3**

| [Table 3] | | | | | | |
|---|---|---|---|---|---|---|
| | Erastin, 1 µM | | Glutamate, 10 mM | | RSL3, 0.3 µM | |
| Example | 0.1 µM | 1.0 µM | 0.01 µM | 0.1 µM | 0.3 µM | 3.0 µM |
| | inhibition (%) | | | | | |
| Ferrostatin-1 | 100 | 100 | 35 | 98 | 100 | 100 |
| 1 | 100 | 100 | 53 | 98 | 100 | 100 |
| 4 | 100 | 100 | 53 | 96 | 100 | 100 |
| 30 | 86 | 100 | 33 | 95 | 100 | 100 |
| 36 | 100 | 100 | 61 | 95 | 100 | 100 |
| 39 | 100 | 100 | 52 | 97 | 100 | 100 |
| 45 | 100 | 100 | 57 | 100 | 100 | 100 |
| 50 | 41 | 68 | 16 | 25 | 0 | 100 |
| 64 | 100 | 100 | 39 | 97 | 78 | 100 |

### Experimental Example 5: Cell death protective effect on cardiac cells (H9C2, white mouse cardiac myocytes)

In order to identify the protective effect on cardiac cells, H9C2 cells were divided into 96-well plates by 1 × 10⁴ and cultured for 18 to 24 hours. The compounds of Examples were diluted to final concentrations of 0.1 and 1 µM, treated in each well, and incubated for 15 to 20 minutes. Erastin, glutamate, and RSL3 were treated to have final concentrations of 1 µM, 25 mM, and 0.5 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 4 showed cell death reduction rates according to the respective concentrations of the compounds of Examples for Erastin, glutamate, and RSL3 as ferroptosis-inducing substances.

**Table 4: Cell death protective effect in cardiac myocytes (H9C2) against Erastin, glutamate and RSL3**

| [Table 4] | | | | | | |
|---|---|---|---|---|---|---|
| | Erastin, 1 µM | | Glutamate, 25 mM | | RSL3, 0.5 µM | |
| Example | 0.1 µM | 1.0 µM | 0.1 µM | 1.0 µM | 0.1 µM | 1.0 µM |
| | inhibition (%) | | | | | |
| Ferrostatin-1 | 100 | 100 | 98 | 97 | 96 | 96 |
| 1 | 100 | 100 | 96 | 97 | 100 | 98 |
| 4 | 100 | 100 | 97 | 96 | 97 | 96 |
| 30 | 0 | 100 | 90 | 98 | 0 | 96 |
| 36 | 100 | 100 | 99 | 97 | 98 | 99 |
| 39 | 100 | 100 | 96 | 96 | 96 | 96 |
| 45 | 100 | 100 | 98 | 96 | 99 | 97 |
| 50 | 16 | 18 | 0 | 49 | 7 | 4 |
| 64 | 100 | 100 | 97 | 97 | 90 | 95 |

### Experimental Example 6: Cell death protective effect on renal fibroblasts (NRK-49F, white mouse renal fibroblasts)

In order to identify the protective effect on renal fibroblasts, NRK-49F cells were divided into 96-well plates by 1 × 10⁴ and cultured for 18 to 24 hours. The compounds of Examples were diluted to final concentrations of 0.1 and 1 µM, treated in each well, and incubated for 15 to 20 minutes. Erastin, glutamate, and RSL3 were treated to have final concentrations of 3 µM, 75 mM, and 3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, glutamate, and RSL3 for 24 hours is completed, the supernatants containing the cells are taken by 70 µl and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 5 showed cell death reduction rates according to the respective concentrations of the compounds of Examples for Erastin, glutamate, and RSL3 as ferroptosis-inducing substances.

**Table 5: Cell death protective effect in renal fibroblasts (NRK-49F) against Erastin, glutamate and RSL3**

| [Table 5] | | | | | | |
|---|---|---|---|---|---|---|
| | Erastin, 3 µM | | Glutamate, 75 mM | | RSL3, 3 µM | |
| Example | 0.1 µM | 1.0 µM | 0.1 µM | 1.0 µM | 0.1 µM | 1.0 µM |
| | inhibition (%) | | | | | |
| Ferrostatin-1 | 99 | 98 | 95 | 100 | 94 | 99 |
| 1 | 99 | 99 | 100 | 100 | 99 | 99 |
| 4 | 99 | 99 | 99 | 100 | 98 | 99 |
| 30 | 98 | 98 | 97 | 100 | 52 | 98 |
| 36 | 99 | 99 | 100 | 100 | 98 | 99 |
| 39 | 99 | 98 | 100 | 100 | 98 | 99 |
| 45 | 99 | 98 | 100 | 98 | 99 | 98 |
| 50 | 3 | 99 | 4 | 73 | 16 | 18 |
| 64 | 100 | 100 | 7 | 99 | 70 | 100 |

### Experimental Example 7: Cell death protective effect on retinal epithelial cells (ARPE-19, human retinal epithelial cells)

In order to identify the protective effect on retinal epithelial cells, ARPE-19 cells were divided into 96-well plates by 1 × 10⁴ and cultured for 18 to 24 hours. The compounds of Examples were diluted to final concentrations of 0.03 and 0.3 µM, treated in each well, and incubated for 20 minutes. Erastin, and RSL3 were treated to have final concentrations of 10 µM, and 0.3 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with Erastin, and RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 6 showed cell death reduction rates according to the respective concentrations of the compounds of Examples for Erastin, and RSL3 as ferroptosis-inducing substances.

**Table 6: Cell death protective effect in retinal epithelial cells (ARPE-19) against Erastin and RSL3**

| [Table 6] | | | | |
|---|---|---|---|---|
| | Erastin, 10 µM | | RSL3, 0.3 µM | |
| Example | 0.03 µM | 0.3 µM | 0.03 µM | 0.3 µM |
| | inhibition (%) | | | |
| Ferrostatin-1 | 86 | 100 | 61 | 100 |
| 1 | 100 | 100 | 95 | 100 |
| 4 | 100 | 100 | 100 | 100 |
| 30 | 80 | 100 | 60 | 100 |
| 36 | 98 | 100 | 100 | 100 |
| 39 | 100 | 100 | 100 | 100 |
| 45 | 100 | 100 | 100 | 100 |
| 50 | 19 | 86 | 4 | 45 |
| 64 | 100 | 100 | 67 | 100 |

### Experimental Example 8: Cell death protective effect on lung epithelial cells (MLE-12, rat lung epithelial cells)

In order to identify the protective effect on lung epithelial cells, MLE-12 cells were divided into 96-well plates by 5 × 10³ and cultured for 18 to 24 hours. The compounds of Examples were diluted to final concentrations of 0.1 and 1.0 µM, treated in each well, and incubated for 20 minutes. RSL3 was treated to have final concentrations of 1.0 µM, respectively, and cultured for 24 hours. In order to identify the protective effect of each compound, the degree of LDH extracellular secretion was measured using cytotoxicity LDH assay kit [Dojindo; CK12]. Briefly, at the time when the treatment of the cells with RSL3 for 24 hours is completed, the supernatants containing the cells are taken by 70 µL and transferred to a new 96-well plate. The assay buffer included in the kit is added in an amount of 70 µL/well (the same amount as the sample) thereto, and then the plate is wrapped with a foil and the reaction is performed at room temperature for 15 minutes. After observing the changed color of the sample, the stop solution was added in an amount of 35 µL/well thereto, and then the % inhibition value was calculated by measuring the absorbance value at a wavelength of 490 nm using an iD3 spectrophotometer. Table 7 showed cell death reduction rates according to the respective concentrations of the compounds of Examples for RSL3 as ferroptosis-inducing substances.

**Table 7: Cell death protective effect in lung epithelial cells (MLE-12) against RSL3**

| [Table 7] | | |
|---|---|---|
| | RSL3, 1.0 µM | |
| Example | 0.1 µM | 1.0 µM |
| | inhibition (%) | |
| Ferro statin-1 | 100 | 100 |
| 1 | 100 | 100 |
| 4 | 90 | 91 |
| 30 | 84 | 95 |
| 36 | 89 | 94 |
| 39 | 89 | 89 |
| 45 | 98 | 100 |
| 50 | 0 | 56 |
| 64 | 100 | 98 |

### Experimental Example 9: Pharmacodynamic Comparison of the Compounds of the Examples and Experimental Examples

### Measuring the pharmacodynamics of the compound of Example 2, (N-cyclopentyl-5-(2-methoxyethoxymethyl)-2-phehyl-1H-indol-7-amine)

Pharmacodynamic parameters can be represented as maximum concentration in plasma (Cₘₐₓ), time taken until maximum concentration in plasma (Tₘₐₓ), area under the plasma concentration-time curve (AUCₗₐₛₜ and AUC_{inf}), and half-life (t_{1/2}), and the like.

The compound of Example 2 was orally administered to ICR mice at a dose of 10 mg/kg, and drug concentration in mouse plasma was measured using HPLC-MS/MS spectroscopy at 0.5, 1, 2, 4, 8 and 24 hours. The mouse brains were removed at 2 or 24 hours, and drug concentration in the brain was measured using HPLC-MS/MS spectroscopy. The area under the plasma concentration v. time curve (AUC_{inf}) was measured to be 5420 ng/mL, and at 2 hours after administration drug concentration in plasma was 398 ng/mL and brain drug concentration was measured at 683 ng/mL. The brain-plasma drug concentration ratio was 1.71, exhibiting very high exposure in the brain. Further, at 24 hours after administration, for Example 2, drug concentration in plasma was measured to be 5.6 ng/mL and drug concentration in the brain was measured at 11.4 ng/mL. With the brain-plasma drug concentration ratio at 2.04, drug exposure in the brain was still high.

### Measuring the pharmacodynamics of the Comparative Example compound [7-cyclopentylamino]-2-phenyl-1H-indol-5-yl]methanol {[7-cyclopentylamino]-2-phenyl-1H-indol-5-yl]methanol}

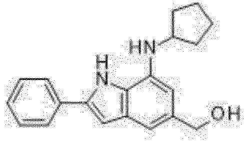

The Comparative Example compound [7-cyclopentylamino]-2-phenyl-1H-indol-5-yl]methanol was synthesized and used in accordance with Korean Registered Patent KR 10-1511771.

The Comparative Example compound was orally administered to ICR mice at a dose of 10 mg/kg, and drug concentration in mouse plasma was measured using HPLC-MS/MS spectroscopy at 0.5, 1, 2, 4, 8 and 24 hours. The mouse brains were removed at 2 or 24 hours, and drug concentration in the brain was measured using HPLC-MS/MS spectroscopy.

For the Comparative Example compound, the area under the plasma concentration v. time curve (AUC_{inf}) was measured to be 5458 ng/mL, and at 2 hours after administration drug concentration in plasma was 1122 ng/mL and brain drug concentration was measured at 95 ng/mL. The brain-plasma drug concentration ratio was 0.08, and further, at 24 hours after administration, no drug concentration was measured in the plasma and brain.

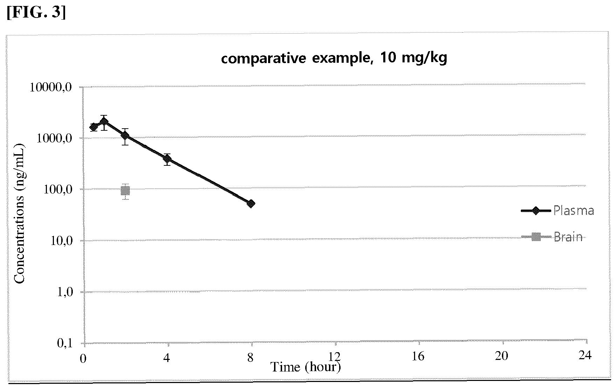

The values for average drug concentration in plasma and the brain v. time following oral administration of the Compound of Example 2 and Comparative Example compound are shown in Table 8, and graphs of these are shown in FIG. 2 and FIG. 3.

**Table 8: Brain/plasma concentration ratios of the compounds of Example 2 and the Comparative Example**

| [Table 8] | | | | | | |
|---|---|---|---|---|---|---|
| **Test article** | **Example 2** | | | **Comparative Example** | | |
| **Dose(mg/kg)** | 10 | | | 10 | | |
| Time (hr) | Plasma | Brain | B/P ratio | Plasma | Brain | B/P ratio |
| 0.5 | 333.7 | NT | - | 1642.4 | NT | - |
| 1 | 336.7 | NT | - | 2113.5 | NT | - |
| 2 | 398.2 | 682.8 | 1.71 | 1122.4 | 95.1 | 0.08 |
| 4 | 671.5 | NT | - | 389.2 | NT | - |
| 8 | 232.0 | NT | - | 50.8 | NT | - |
| 24 | 5.6 | 11.4 | 2.04 | 0.0 | 0.0 | NA |
| AUCₗₐₛₜ (ng·hr/mL or ng·hr/g) | 5396.0 | 8319.0 | 1.74 | 5359.1 | NA | NA |

| | | | | | | |
|---|---|---|---|---|---|---|
| NT: not tested, NA: not available | | | | | | |

### Analysis of the pharmacodynamic results for the compound of Example 2 and the Comparative Example compound

The area under the plasma concentration v. time curve (AUCₗₐₛₜ) for the compounds of Example 2 and the Comparative Example was measured at 5396 ng/mL v. 5359 ng/mL. Similar plasma exposure was exhibited.

However, in the case of the Comparative Example, at 2 hours after administration, drug concentration in plasma was measured at 1122 ng/mL and drug concentration in brain was measured at 95 ng/mL, representing a brain-plasma drug concentration ratio of 0.08. For the compound of Example 2, at 2 hours after administration drug concentration in plasma was 398 ng/mL and brain drug concentration was measured at 683 ng/mL. The brain-plasma drug concentration ratio was 1.71, exhibiting very high exposure in the brain. Further, whereas for the Comparative Example, no drug concentration was measured in the plasma and brain at 24 hours after administration, drug concentration in plasma was measured at 5.6 ng/mL and drug concentration in brain was measured at 11.4 ng/mL for Example 2, giving a brain-plasma drug concentration ratio of 2.04. In-brain drug exposure was still higher than the comparative example

Therefore, it can be known that the present invention has the characteristics of a compound which maintains fair levels of plasma exposure while having good brain exposure.

## Claims

1. A compound of Formula 1 below, an isomer thereof, a solvate thereof, a hydrate thereof, or a pharmaceutically acceptable salt thereof: 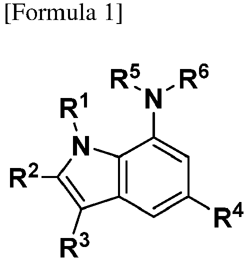 wherein,
R¹ is hydrogen or C₁₋₈ alkyl,
R² and R³ are each independently hydrogen, aryl with 5 to 10 atoms, heteroaryl with 5 to 10 atoms, C₁₋₈ alkyl, or C₁₋₈ alkoxy, and when R² and R³ are aryl or heteroaryl, R² and R³ are each independently unsubstituted or substituted with one or more substituents of halogen, C₁₋₈ alkyl, -P(=O)(C₁₋₈ alkyl)₂, and C₁₋₈ alkynyl-OH,
R⁴ is -C₁₋₈ alkylene-O-C₁₋₈ alkylene-O-R⁷, where
R⁷ is C₁₋₈ alkyl or -C₁₋₈ alkylene-O-C₁₋₈ alkyl,
R⁵ and R⁶ are each independently hydrogen, C₁₋₈ alkyl, C₁₋₈ alkoxy, C₃₋₁₀ cycloalkyl, -C₃₋₁₀ heterocycloalkyl, -(CH₂)ₘ-C₃₋₁₀ cycloalkyl, -(CH₂)ₘ-C₃₋₁₀ heterocycloalkyl, aryl with 5 to 10 atoms, benzyl, heteroaryl with 5 to 10 atoms or -(CH₂)ₘ-heteroaryl with 5 to 10 atoms, or are linked together with the N atom, to which they are bonded, to form C₃₋₁₀ heterocycloalkyl, heteroaryl with 5 to 10 atoms or C₁₋₈ alkylimine, where m is an integer of 1 to 4,
R⁵ and R⁶ are unsubstituted or substituted with one or more substituents R⁸ selected from the group consisting of C₁₋₈ alkyl, C₁₋₈ alkoxy, C₁₋₈ haloalkyl, halogen, -OH, oxo (=O), - C(=O)-C₁₋₈ alkyl, -C(=O)O-C₁₋₈ alkyl, -C(=O)NH-aryl with 5 to 10 atoms and -S(=O)₂-C₁₋₈ alkyl, and
R⁸ is unsubstituted or substituted with one or more substituents R⁹ selected from halogen and C₁₋₈ haloalkyl.

2. The compound according to Claim 1, an isomer thereof, a solvate thereof, a hydrate thereof, or a pharmaceutically acceptable salt thereof, wherein
the compound of Formula 1 is represented by Formula 2 below: 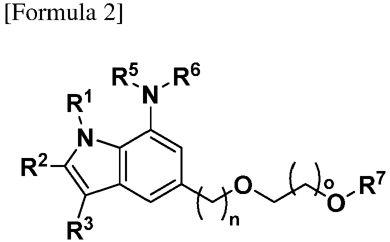 wherein, R¹ to R³ and R⁵ to R⁷ are each as defined in Claim 1, and n and o are each independently an integer of 1 to 4.

3. The compound according to Claim 1, an isomer thereof, a solvate thereof, a hydrate thereof, or a pharmaceutically acceptable salt thereof, wherein
R¹ is hydrogen or C₁₋₆ alkyl,
R² and R³ are each independently hydrogen, aryl with 5 to 8 atoms, heteroaryl with 5 to 8 atoms containing one or more heteroatoms of N, O, and S, C₁₋₆ alkyl, or C₁₋₆ alkoxy, and when R² and R³ are aryl or heteroaryl, R² and R³ are each independently unsubstituted or substituted with one or more substituents of halogen, C₁₋₆ alkyl, -P(=O)(C₁₋₆ alkyl)₂, and C₁₋₆ alkynyl-OH,
R⁴ is C₁₋₆ alkylene-O-C₁₋₆ alkylene-O-R⁷, where
R⁷ is C₁₋₆ alkyl or C₁₋₆ alkylene-O-C₁₋₆ alkyl,
R⁵ and R⁶ are each independently hydrogen, C₁₋₆ alkyl, C₁₋₆ alkoxy, C₃₋₇ cycloalkyl, C₃₋₇ heterocycloalkyl, -(CH₂)ₘ-C₃₋₇ cycloalkyl, -(CH₂)ₘ-C₃₋₇ heterocycloalkyl, aryl with 5 to 8 atoms, benzyl, heteroaryl with 5 to 8 atoms containing one or more heteroatoms of N, O, and S, or -(CH₂)ₘ-heteroaryl with 5 to 8 atoms, or are linked together with the N atom, to which they are bonded, to form C₃₋₇ heterocycloalkyl containing one or more heteroatoms of N, O, and S, heteroaryl with 5 to 10 atoms containing one or more heteroatoms of N, O, and S, or C₁₋₆ alkylimine, where m is an integer of 1 to 4,
R⁵ and R⁶ are unsubstituted or substituted with one or more substituents R⁸ selected from the group consisting of C₁₋₆ alkyl, C₁₋₆ alkoxy, C₁₋₆ haloalkyl, halogen, -OH, oxo (=O), - C(=O)-C₁₋₆ alkyl, -C(=O)O-C₁₋₆ alkyl, -C(=O)NH-aryl with 5 to 8 atoms and -S(=O)₂-C₁₋₆ alkyl, and
R⁸ is unsubstituted or substituted with one or more substituents R⁹ selected from halogen and C₁₋₆ haloalkyl.

4. The compound according to Claim 1, an isomer thereof, a solvate thereof, a hydrate thereof, or a pharmaceutically acceptable salt thereof, wherein
R² and R³ are each independently hydrogen, aryl with 5 to 8 atoms, or C₁₋₆ alkyl, and
when R² and R³ are aryl, R² and R³ are each independently unsubstituted or substituted with one or more substituents of halogen, C₁₋₆ alkyl, -P(=O)(C₁₋₆ alkyl)₂, and C₁₋₆ alkynyl-OH.

5. The compound according to Claim 1, an isomer thereof, a solvate thereof, a hydrate thereof, or a pharmaceutically acceptable salt thereof, wherein
when any one of R² and R³ is hydrogen, the other is aryl with 5 to 10 atoms, heteroaryl with 5 to 10 atoms, C₁₋₈ alkyl, or C₁₋₈ alkoxy.

6. The compound according to Claim 1, an isomer thereof, a solvate thereof, a hydrate thereof, or a pharmaceutically acceptable salt thereof, wherein
R⁵ and R⁶ are simultaneously C₃₋₇ cycloalkyl; or
any one of R⁵ and R⁶ is hydrogen, and the other is C₁₋₈ alkyl, C₁₋₈ alkoxy, C₃₋₁₀ cycloalkyl, C₃₋₁₀ heterocycloalkyl, -(CH₂)ₘ-C₃₋₁₀ cycloalkyl, -(CH₂)ₘ-C₃₋₁₀ heterocycloalkyl, aryl with 5 to 10 atoms, benzyl, heteroaryl with 5 to 10 atoms or -(CH₂)ₘ-heteroaryl with 5 to 10 atoms, and m is an integer of 1 to 4, where the heterocycloalkyl and heteroaryl contain one or more heteroatoms of N, O and S.

7. The compound according to Claim 1, an isomer thereof, a solvate thereof, a hydrate thereof, or a pharmaceutically acceptable salt thereof, wherein
R⁵ and R⁶ are linked together with the N atom, to which they are bonded, to form C₃₋₇ heterocycloalkyl, or C₂₋₅ alkylimine, and
C₂₋₅ alkylimine is substituted with C₁₋₈ haloalkyl.

8. The compound according to Claim 1, an isomer thereof, a solvate thereof, a hydrate thereof, or a pharmaceutically acceptable salt thereof, wherein
when one or more of R⁵ and R⁶ are C₁₋₈ alkyl, the substituent R⁸ is C₁₋₈ alkoxy or C₁₋₈ haloalkyl;
when one or more of R⁵ and R⁶ are C₃₋₁₀ heterocycloalkyl containing a heteroatom of N, the substituent R⁸ is selected from the group consisting of C₁₋₆ alkyl, C₁₋₆ alkoxy, C₁₋₆ haloalkyl, halogen, -OH, oxo (=O), -C(=O)-C₁₋₆ alkyl, -C(=O)O-C₁₋₆ alkyl, -C(=O)NH-aryl with 5 to 8 atoms and -S(=O)₂-C₁₋₆ alkyl; or
when one or more of R⁵ and R⁶ are C₃₋₁₀ heterocycloalkyl containing a heteroatom of S, the substituent R⁸ is oxo (=O).

9. The compound according to Claim 1, an isomer thereof, a solvate thereof, a hydrate thereof, or a pharmaceutically acceptable salt thereof, is **characterized in that**
the compound of Formula 1 is any one selected from the following compound group:
< 1 > 5-(2-methoxyethoxymethyl)-2-phenyl-N-tetrahydropyran-4-yl-1H-indol-7-amine; <2> N-cyclopentyl-5-(2-methoxyethoxymethyl)-2-phenyl-1H-indol-7-amine; <3> N,N-dicyclopentyl-5-(2-methoxyethoxymethyl)-2-phenyl-1H-indol-7-amine; <4> 5-(2-methoxyethoxymethyl)-2-phenyl-N-(tetrahydropyran-4-ylmethyl)-1H-indol-7-amine; <5> 4-[5-(2-methoxyethoxymethyl)-2-phenyl-1H-indol-7-yl]morpholine; <6> 5-(2-methoxyethoxymethyl)-2-phenyl-N-tetrahydrofuran-3-yl-1H-indol-7-amine; <7> 5-(2-methoxyethoxymethyl)-N-(oxetane-3-yl)-2-phenyl-1H-indol-7-amine; <8> N-cyclobutyl-5-(2-methoxyethoxymethyl)-2-phenyl-1H-indol-7-amine; <9> N,N-dicyclobutyl-5-(2-methoxyethoxymethyl)-2-phenyl-1H-indol-7-amine; <10> N-cyclobutyl-5-(2-methoxyethoxymethyl)-1-methyl-2-phenyl-indol-7-amine; <11> N-(2,2-dimethylpropyl)-5-(2-methoxyethoxymethyl)-2-phenyl-1H-indol-7-amine; <12> N-(cyclopentylmethyl)-5-(2-methoxyethoxymethyl)-2-phenyl-1H-indol-7-amine; <13> N-cyclohexyl-5-(2-methoxyethoxymethyl)-2-phenyl-1H-indol-7-amine; <14> N-(4,4-difluorocyclohexyl)-5-(2-methoxyethoxymethyl)-2-phenyl-1H-indol-7-amine; <15> N-isobutyl-5-(2-methoxyethoxymethyl)-2-phenyl-1H-indol-7-amine; <16> 5-(2-methoxyethoxymethyl)-2-phenyl-N-propyl-1H-indol-7-amine; <17> N-butyl-5-(2-methoxyethoxymethyl)-2-phenyl-1H-indol-7-amine; <18> N-(2-ethylbutyl)-5-(2-methoxyethoxymethyl)-2-phenyl-1H-indol-7-amine; <19> N-isopentyl-5-(2-methoxyethoxymethyl)-2-phenyl-1H-indol-7-amine; <20> 5-(2-methoxyethoxymethyl)-2-phenyl-N-sec-butyl-1H-indol-7-amine; <21> N-(cyclopropylmethyl)-5-(2-methoxyethoxymethyl)-2-phenyl-1H-indol-7-amine; <22> 5-(2-methoxyethoxymethyl)-N-(1-methylbutyl)-2-phenyl-1H-indol-7-amine; <23> N-(1-ethylpropyl)-5-(2-methoxyethoxymethyl)-2-phenyl-1H-indol-7-amine; <24> (E, Z)-1,1,1-trifluoro-N-[5-(2-methoxyethoxymethyl)-2-phenyl-1H-indol-7-yl]propane-2-imine; <25> 5-(2-methoxyethoxymethyl)-2-phenyl-N-(2,2,2,-trifluoro-1-methyl-ethyl)-1H-indol-7-amine; <26> N-(1,2-dimethylpropyl)-5-(2-methoxyethoxymethyl)-2-phenyl-1H-indol-7-amine; <27> N-isopropyl-5-(2-methoxyethoxymethyl)-2-phenyl-1H-indol-7-amine; <28> N-benzyl-5-(2-methoxyethoxymethyl)-2-phenyl-1H-indol-7-amine; <29> tert-butyl-4-[[5-(2-methoxyethoxymethyl)-2-phenyl-1H-indol-7-yl]amino]piperidine-1-carboxylate; <30> 1-[4-[[5-(2-methoxyethoxymethyl)-2-phenyl-1H-indol-7-yl]amino]piperidyl]-1-ethanon; <31> 5-(2-methoxyethoxymethyl)-N-(1-methylsulfonyl-4-piperidyl)-2-phenyl-1H-indol-7-amine; <32> N-(1,1-dioxothiane-4-yl)-5-(2-methoxyethoxymethyl)-2-phenyl-1H-indol-7-amine; <33> 5-(2-methoxyethoxymethyl)-N-(1-oxothiane-4-yl)-2-phenyl-1H-indol-7-amine; <34> 5-(2-methoxyethoxymethyl)-N-(1-methyl-4-piperidyl)-2-phenyl-1H-indol-7-amine; <35> 5-(2-ethoxyethoxymethyl)-2-phenyl-N-tetrahydropyran-4-yl-1H-indol-7-amine; <36> N-cyclopentyl-5-(2-ethoxyethoxymethyl)-2-phenyl-1H-indol-7-amine; <37> N-cyclopentyl-5-(2-isopropoxyethoxymethyl)-2-phenyl-1H-indol-7-amine; <38> 5-(2-isopropoxyethoxymethyl)-2-phenyl-N-tetrahydropyran-4-yl-1H-indol-7-amine; <39> N-cyclopentyl-5-[2-(2-methoxyethoxy)ethoxymethyl]-2-phenyl-1H-indol-7-amine; <40> 5-[2-(2-methoxyethoxy) ethoxymethyl]-2-phenyl-N-tetrahydropyran-4-yl-1H-indol-7-amine; <41> N-[3,5-bis(trifluoromethyl)phenyl]-4-[[5-(2-methoxyethoxymethyl)-2-phenyl-1H-indol-7-yl]amino]piperidine-1-carboxamide; <42> N-(3,5-dichlorophenyl)-4-[[5-(2-methoxyethoxymethyl)-2-phenyl-1H-indol-7-yl]amino]piperidine-1-carboxamide; <43> 5-(2-methoxyethoxymethyl)-2-phenyl-N-(2-thienylmethyl)-1H-indol-7-amine; <44> ethyl 4-[[5-(2-methoxyethoxymethyl)-2-phenyl-1H-indol-7-yl]amino]piperidine-1-carboxylate; <45> 5-(2-methoxyethoxymethyl)-2-phenyl-N-4-pyridylmethyl)-1H-indol-7-amine; <46> 5-(2-methoxyethoxymethyl)-N-(2-methoxyethyl)-2-phenyl-1H-indol-7-amine; <47> 5-(2-methoxyethoxymethyl)-N-(3-methoxypropyl)-2-phenyl-1H-indol-7-amine; <48> 2-(3-fluorophenyl)-5-(2-methoxyethoxymethyl)-N-tetrahydropyran-4-yl-1H-indol-7-amine; <49> N-cyclopentyl-5-(2-methoxyethoxymethyl)-3-methyl-1H-indol-7-amine; <50> 5-(2-methoxyethoxymethyl)-3-methyl-N-tetrahydropyran-4-yl-1H-indol-7-amine; <51> N-cyclopentyl-5-(2-methoxyethoxymethyl)-3-phenyl-1H-indol-7-amine; <52> 5-(2-methoxyethoxymethyl)-3-phenyl-N-tetrahydropyran-4-yl-1H-indol-7-amine; <53>5-((2-methoxyethoxy)methyl)-1-methyl-2-phenyl-N-(tetrahydro-2H-pyran-4-yl)-1H-indol-7-amine; <54>2-(4-bromophenyl)-N-cyclopentyl-5-((2-methoxyethoxy)methyl)-1H-indol-7-amine; <55>2-(4-bromophenyl)-5-((2-methoxyethoxy)methyl)-N-(tetrahydro-2H-pyran-4-yl)-1H-indol-7-amine; <56>(4-(7-(cyclopentylamino)-5-((2-methoxyethoxy)methyl)-1H-indol-2-yl)phenyl)dimethylphosphine oxide; <57>(4-(5-((2-methoxyethoxy)methyl)-7-((tetrahydro-2H-pyran-4-yl)amino-1H-indol-2-yl)phenyl)dimethylphosphine oxide; <58>4-(4-(7-(cyclopentylamino)-5-((2-methoxyethoxy)methyl)-1H-indol-2-yl)phenyl)-2-methylbut-3-yn-2-ol; <59>4-(4-(5-((2-methoxyethoxy)methyl)-7-((tetrahydro-2H-pyran-4-yl)amino)-1H-indol-2-yl)phenyl)-2-methylbut-3-yn-2-ol; <60>5-((2-methoxyethoxy)methyl)-N-(tetrahydro-2H-pyran-4-yl)-2-(p-tolyl)-1H-indol-7-amine; <61>N-cyclopentyl-5-((3-methoxypropoxy)methyl)-2-phenyl-1H-indol-7-amine; <62>5-((3-methoxypropoxy)methyl)-N-(oxetan-3-yl)-2-phenyl-1H-indol-7-amine; <63>5-((3-methoxypropoxy)methyl-2-phenyl-N-(tetrahydro-2H-pyran-4-yl)-1H-indol-7-amine; <64>5-((2-methoxyethoxy)methyl)-2-phenyl-N-(piperidin-4-yl)-1H-indol-7-amine; <65>5-(3-(2-methoxyethoxy)propyl)-2-phenyl-N-(tetrahydro-2H-pyran-4-yl)-1H-indol-7-amine; <66>N-cyclopentyl-5-(2-(2-methoxyethoxy)ethyl)-2-phenyl-1H-indol-7-amine; <67>5-(2-(2-methoxyethoxy)ethyl)-2-phenyl-N-(tetrahydro-2H-pyran-4-yl)-1H-indol-7-amine; <68>5-((2-methoxyethoxy)methyl)-2-phenyl-7-(piperidin-1-yl)-1H-indole; <69>4-((5-((2-methoxyethoxy)methyl)-2-phenyl-1H-indol-7-yl)amino)cyclohexan-1-ol; and <70>4-((5-((2-methoxyethoxy)methyl)-2-phenyl-1H-indol-7-yl)amino)-1-methylcyclohexan-1-ol.

10. A method for preparing a compound of Formula 1 comprising steps of:
reacting a compound of Formula 3 and HO-C₁₋₈ alkylene-O-R⁷ to prepare a compound of Formula 4;
reducing the compound of Formula 4; and
preparing the compound of Formula 1 from the reduced compound of Formula 4: 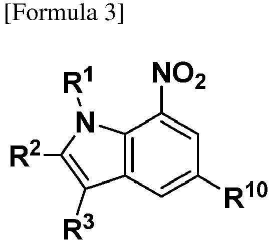 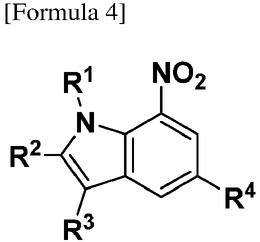 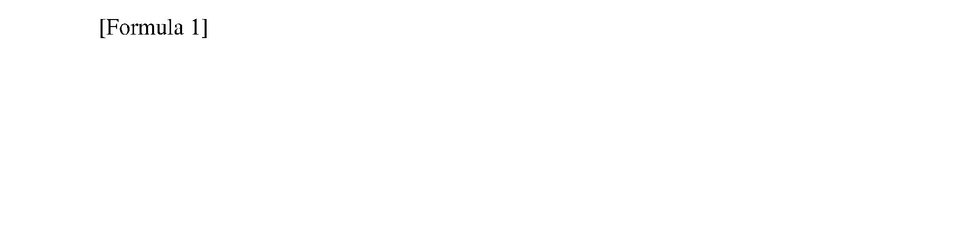 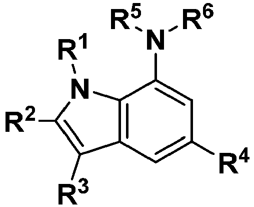
wherein, R¹⁰ is -C₁₋₈ alkylene-LG, where LG is a leaving group, and R¹ to R⁷ are each the same as defined in Claim 1.

11. A pharmaceutical composition for prevention or treatment of cell necrosis or ferroptosis-related diseases comprising the compound of Chemical Formula 1 of Claim 1, an isomer thereof, a hydrate thereof, a solvate thereof, or a pharmaceutically acceptable salt thereof, as an active ingredient; and a pharmaceutically acceptable carrier.

12. A pharmaceutical composition for prevention or treatment of cell necrosis or ferroptosis-related diseases selected from the following group, the composition comprising the compound of Chemical Formula 1 of Claim 1, an isomer thereof, a hydrate thereof, a solvate thereof, or a pharmaceutically acceptable salt thereof, as an active ingredient; and a pharmaceutically acceptable carrier:
Acute or chronic hepatic disease, dementia, Parkinson's disease, Huntington's disease, ischemic disease, diabetes mellitus, pancreatitis, bacterial or viral sepsis, necrotizing procolitis, cystic fibrosis, rheumatoid arthritis, degenerative arthritis, nephropathy, bacterial infection, viral infection, multiple sclerosis, leukemia, lymphoma, neonatal respiratory distress syndrome, asphyxia, tuberculosis, endometriosis, angiasthenia, psoriasis, chilblain, steroid treatment complications, gangrene, pressure sores, hemoglobinuria, bums, hyperthermia, Crohn's disease, celiac disease, compartment syndrome, spinal cord injury, glomerulonephritis, renal ischemia-reperfusion injury, muscular dystrophy, mycoplasmal disease, anthrax, Andersen's disease, congenital mitochondrial disease, phenylketonuria, placental infarction, syphilis, osteonecrosis; alcoholism, the exposure to cocaine, antibiotics, anti-cancer agent, NSAID, cyclosporine, chemical toxins, poison gas, agrochemicals, heavy metals, or injury due to the exposure to radioactivity/UV and associated necrosis thereof, acute/chronic renal disease, traumatic brain damage, amyotrophic lateral sclerosis, necrotizing colitis, viral infection, skin disease including psoriasis and allergic dermatitis, and organ preservation/organ transplant, chronic inflammatory pulmonary disease including acute lung injury syndrome / acute pulmonary disease, pneumonia, tuberculosis, asthma, pulmonary hypertension, chronic obstructive pulmonary disease, idiopathic pulmonary fibrosis and cystic fibrosis, demyelinating diseases including demyelination and amyotrophic lateral sclerosis (ALS), hypertension including pulmonary hypertension, stroke, prion disease, epilepsy, ataxia, migraine, reduced cognitive skill, seizure, tremors, psychiatric disorders , insulin resistance, hyperlipidemia, atherosclerosis, inflammatory bowel disease (IBD) including Crohn's Disease and ulcerative colitis, cancers and metastasis of cancer, visual impairment-associated disease including macular degeneration, retinitis pigmentosa, optic neuropathy, cataracts, glaucoma, anemia, cholestasis, hypoparathyroidism, pancytopenia, pancreatic disorder, lactic acidosis, lactacidaemia, hearing loss, short stature, intestinal obstruction, cardiac conduction defect, cardiomyopathy, myocardial infarction, myocardial ischemia reperfusion injury, heart failure, endometriosis, infertility, early menopause, muscular atrophy including limb girdle/Becker muscular dystrophy (GGMD/BMD) and Duchenne muscular dystrophy (DMD), aging and aging-related diseases, and mucositis.

13. The pharmaceutical composition for prevention or treatment of cell necrosis or ferroptosis-related diseases according to Claim 11, wherein
the disease is neurodegenerative disease, liver disease, kidney disease, stroke, myocardial infarction, ocular disease or lung disease.

14. The pharmaceutical composition for prevention or treatment of cell necrosis or ferroptosis-related diseases according to Claim 13, wherein
the neurodegenerative disease is one or more selected from the group consisting of Alzheimer's disease, Parkinson's disease, epilepsy, Huntington's disease, amyotrophic lateral sclerosis, Friedreich's ataxia, multiple sclerosis, CMT (Charcot-Marie-Tooth) disease, dementia with Lewy bodies, and traumatic brain injury.

15. A method for preventing or treating cell necrosis or ferroptosis-related diseases comprising a step of administering the compound of Formula 1 according to Claim 1, an isomer thereof, a hydrate thereof, a solvate thereof, or a pharmaceutically acceptable salt thereof, to a subject in need thereof in a pharmaceutically effective amount.

16. The method for preventing or treating cell necrosis or ferroptosis-related diseases according to Claim 15, wherein
the disease involves lipid peroxidation.

17. A method for suppressing ferroptosis comprising a step of administering the compound of Formula 1 according to Claim 1, an isomer thereof, a hydrate thereof, a solvate thereof, or a pharmaceutically acceptable salt thereof, to a subject in need thereof in a pharmaceutically effective amount.

18. A method for reducing reactive oxygen species (ROS) in cells comprising a step of contacting the cells with the compound of Formula 1 according to Claim 1, an isomer thereof, a hydrate thereof, a solvate thereof, or a pharmaceutically acceptable salt thereof.

19. A use of the compound of Formula 1 according to Claim 1, an isomer thereof, a solvate thereof, or a pharmaceutically acceptable salt thereof, for prevention or treatment of cell necrosis or ferroptosis-related diseases.
